# EUROPEAN PATENT APPLICATION

(11) **EP 4 471 138 A1**
(43) Date of publication of application: **04.12.2024**
(21) Application number: 23746440.9
(22) Date of filing: 29.01.2023
(51) Int. Cl.: C12N 9/52, C12N 15/57, A61K 38/48, A61P 37/00, A61P 13/12

(54) **IGA PROTEASE TRUNCATION, FUSION PROTEIN COMPRISING IGA PROTEASE TRUNCATION, AND USE THEREOF**

(30) Priority: 29.01.2022 CN 202210112254; 18.01.2023 CN 202310079241
(71) Applicant: Peking University First Hospital, Beijing 100034 (CN); Shanghai Alezyme Pharmaceuticals Ltd., Pudong New Area, Shanghai 201210 (CN)
(72) Inventor: LV, Jicheng, Beijing 100034 (CN); ZHANG, Hong, Beijing 100034 (CN); SHU, Chutian, Shanghai 201210 (CN)
(74) Representative: Simmons & Simmons LLP (Munich)
(86) International application number: PCT/CN2023/073698
(87) International publication number: WO 2023/143563

(57) **Abstract**

The present application relates to an IgA protease truncation, a fusion protein comprising the IgA protease truncation (e.g., a fusion protein comprising the IgA protease truncation and an Fc), and use thereof in treating an IgA deposition disease (e.g., IgA nephropathy).

## Description

### FIELD OF THE INVENTION

The present disclosure relates to the biopharmaceutical field. In particular, the present disclosure relates to a truncated form of IgA protease, a fusion protein comprising a truncated form of IgA protease, a pharmaceutical composition comprising the truncated form of IgA protease or the fusion protein, a nucleic acid encoding the truncated form of IgA protease or the fusion protein, a method for preparing the truncated form of IgA protease or the fusion protein, and use of the truncated form of IgA protease and the fusion protein in the manufacture of a medicament for treating diseases associated with IgA deposition.

### BACKGROUND

IgA nephropathy is currently one of the most common primary glomerular diseases in the world and places a heavy burden on patients and society. There is a lack of specific treatment for IgA nephropathy. Most clinical treatment is based on supportive therapy with RAS blockers to slow down the deterioration of renal function. Patients who fail to respond to supportive therapy are treated with a combination of hormonal immunosuppressive agents. However, the use of hormonal immunosuppressants is not effective in the long term and causes serious side effects.

There is an urgent need to develop effective therapeutic agents with low side effects.

### SUMMARY OF THE INVENTION

In one aspect, the present disclosure provides an isolated truncated form of IgA protease comprising a non-natural truncated fragment of a wild-type IgA protease obtained from or derived from *Clostridium ramosum* or having at least 70% sequence identity to the non-natural truncated fragment. In some embodiments, the non-natural truncated fragment has an amino acid substitution, deletion, insertion or modification compared to the wild-type IgA protease of *Clostridium ramosum,* such that the truncated form of IgA protease loses or reduces its self-cleaving function. In some embodiments, the amino acid substitution, deletion, insertion or modification occurs at a natural self-cleaving site of the wild-type IgA protease of *Clostridium ramosum,* within 5 sites upstream and/or within 5 sites downstream of the natural self-cleaving site. In some embodiments, the non-natural truncated fragment is a N-terminal or C-terminal truncated fragment of a wild-type IgA protease obtained from or derived from *Clostridium ramosum.* In some embodiments, the *Clostridium ramosum* is *Clostridium ramosum* strain AK183. In some embodiments, an amino acid sequence of the wild-type IgA protease of *Clostridium ramosum* is as set forth in SEQ ID NO: 1. In some embodiments, the natural self-cleaving site is between position 730 and position 840 (*e.g.*, between position 792 and position 797) of the amino acid sequence as set forth in SEQ ID NO: 1. In some embodiments, the natural self-cleaving site is at position 790, position 791, position 792, position 793, position 794, position 795, position 796, position 797, position 798, position 799 or position 800 of the amino acid sequence as set forth in SEQ ID NO: 1.

In some embodiments, the non-natural truncated fragment is a N-terminal or C-terminal truncated fragment of a wild-type IgA protease obtained from or derived from *Clostridium ramosum.* In some embodiments, the N-terminal truncated fragment comprises a polypeptide fragment of at least 760 continuous amino acids starting from position 31 of the N-terminus of a wild-type IgA protease obtained from or derived from *Clostridium ramosum* or having at least 70% sequence identity to the polypeptide fragment. In some embodiments, the truncated form of IgA protease comprises a polypeptide fragment of at least 760 (*e.g*., at least 761, at least 762, at least 763, at least 764, at least 765, at least 766, at least 767, at least 768, at least 769, at least 770, at least 771, at least 772, at least 773, at least 774, at least 775, at least 776, at least 777, at least 778, at least 779, at least 780, at least 781, at least 782, at least 783, at least 784, at least 785, at least 786, at least 787, at least 788, at least 789, at least 790, at least 791, at least 792, at least 793, at least 794, at least 795, at least 796, at least 797, at least 798, at least 799, at least 800, at least 801, at least 802, at least 803, at least 804, at least 805, at least 806, at least 807, at least 808, at least 809, at least 810, at least 900, at least 950, at least 1000, at least 1100, at least 1150 or at least 1200) continuous amino acids starting from position 31 of the amino acid sequence as set forth in SEQ ID NO: 1. In some embodiments, the truncated form of IgA protease provided herein comprises a polypeptide fragment selected from the group consisting of amino acids from position 31 to position 790 of the amino acid sequence as set forth in SEQ ID NO: 1, amino acids from position 31 to position 792 of the amino acid sequence as set forth in SEQ ID NO: 1, amino acids from position 31 to position 798 of the amino acid sequence as set forth in SEQ ID NO: 1, amino acids from position 31 to position 807 of the amino acid sequence as set forth in SEQ ID NO: 1, amino acids from position 31 to position 816 of the amino acid sequence as set forth in SEQ ID NO: 1, amino acids from position 31 to position 833 of the amino acid sequence as set forth in SEQ ID NO: 1, and a polypeptide fragment having at least 70% sequence identity thereto.

In some embodiments, the non-natural truncated fragment comprises a polypeptide fragment of at least 456 continuous amino acids starting from position 335 of the N-terminus of a wild-type IgA protease obtained from or derived from *Clostridium ramosum,* or having at least 90% or at least 95% sequence identity to the polypeptide fragment. In some embodiments, the truncated form of IgA protease provided herein comprises a polypeptide fragment of at least 456 (*e.g*., at least 457, at least 458, at least 459, at least 460, at least 461, at least 462, at least 463, at least 464, at least 465, at least 466, at least 467, at least 468, at least 469, at least 470, at least 471, at least 472, at least 473, at least 474, at least 475, at least 476, at least 477, at least 478, at least 479, at least 480, at least 481, at least 482, at least 483, at least 484, at least 485, at least 486, at least 487, at least 488, at least 489, at least 490, at least 491, at least 492, at least 493, at least 494, at least 495, at least 496, at least 497, at least 498, at least 499, at least 500, at least 550, at least 600, at least 650, at least 700, at least 750, at least 800, at least 850 or at least 900) continuous amino acids starting from position 335 of the amino acid sequence as set forth in SEQ ID NO: 1. In some embodiments, the truncated form of IgA protease provided herein comprises a polypeptide fragment selected from the group consisting of amino acids from position 335 to position 790 of the amino acid sequence as set forth in SEQ ID NO: 1, amino acids from position 335 to position 791 of the amino acid sequence as set forth in SEQ ID NO: 1, amino acids from position 335 to position 792 of the amino acid sequence as set forth in SEQ ID NO: 1, amino acids from position 285 to position 790 of the amino acid sequence as set forth in SEQ ID NO: 1, amino acids from position 285 to position 791 of the amino acid sequence as set forth in SEQ ID NO: 1, amino acids from position 285 to position 792 of the amino acid sequence as set forth in SEQ ID NO: 1, amino acids from position 330 to position 790 of the amino acid sequence as set forth in SEQ ID NO: 1, amino acids from position 330 to position 791 of the amino acid sequence as set forth in SEQ ID NO: 1, amino acids from position 330 to position 792 of the amino acid sequence as set forth in SEQ ID NO: 1, amino acids from position 285 to position 816 of the amino acid sequence as set forth in SEQ ID NO: 1, and a polypeptide sequence having at least 90% or at least 95% sequence identity thereto.

In some embodiments, the truncated form of IgA protease provided herein has an amino acid conservative substitution at one or more sites compared to the amino acid sequence of the polypeptide fragment. In some embodiments, an amino acid mutation occurs at one or more sites of the polypeptide fragment, wherein the one or more sites correspond to position 844, position 862, position 931, position 933, position 978, position 1002, and/or position 1004 of SEQ ID NO: 1. In some embodiments, one or more sites of the polypeptide fragment are mutated to glycine, wherein the one or more sites correspond to position 844, position 862, position 931, position 933, position 978, position 1002, and/or position 1004 of SEQ ID NO: 1. In some embodiments, the polypeptide fragment has an amino acid mutation at position 844, an amino acid mutation at position 862, amino acid mutations at positions 931 and 933, an amino acid mutation at position 978, or amino acid mutations at positions 1002 and 1004 corresponding to SEQ ID NO: 1. In some embodiments, the amino acid sequence of the polypeptide fragment is as set forth in SEQ ID NO: 53 (also referred to as "PA-GA Mut"), SEQ ID NO: 54 (also referred to as "PI-GI Mut"), SEQ ID NO: 55 (also referred to as "PAP-GAG Mut"), SEQ ID NO: 56 (also referred to as "PAT-GAT Mut") or SEQ ID NO: 57 (also referred to as "PIP-GIG Mut").

In some embodiments, the truncated form of IgA protease provided herein has an enzymatic activity of specifically cleaving human IgA. In some embodiments, the truncated form of IgA protease provided herein has an enzymatic activity of specifically cleaving human IgA heavy chain. In some embodiments, the truncated form of IgA protease provided herein has an enzymatic activity of specifically cleaving the intersection of human IgA heavy chain CH1 and hinge region. In some embodiments, the truncated form of IgA protease provided herein has an enzymatic activity of specifically cleaving human IgA1.

In another aspect, the present disclosure provides a fusion protein comprising a first polypeptide and a second polypeptide, wherein the first polypeptide comprises a full-length wild-type IgA protease obtained from or derived from *Clostridium ramosum,* a polypeptide formed by removing a signal peptide of a wild-type IgA protease obtained from or derived from *Clostridium ramosum,* or the truncated form of IgA protease provided herein; the second polypeptide comprises an amino acid sequence for extending half-life of the first polypeptide in a subject. In some embodiments, the first polypeptide comprises a sequence as set forth in SEQ ID NO: 1 or SEQ ID NO: 42. In some embodiments, the second polypeptide is located at N-terminus or C-terminus of the first polypeptide.

In some embodiments, the first polypeptide and the second polypeptide are linked via a linker. In some embodiments, the first polypeptide and the second polypeptide are directly linked to each other. In some embodiments, the linker is selected from the group consisting of a cleavable linker, a non-cleavable linker, a peptide linker, a flexible linker, a rigid linker, a helical linker and a non-helical linker. In some embodiments, the linker comprises a peptide linker. In some embodiments, the peptide linker comprises a linker comprising a glycine and a serine. In some embodiments, the linker comprising a glycine and a serine comprises one, two, three, four or more repeats of a sequence as set forth in SEQ ID NO: 21 (GGGS), SEQ ID NO: 22 (GGGGS), SEQ ID NO: 86 (GGGGGS) or SEQ ID NO: 87 (GGGGGGGS). In some embodiments, the linker comprises an amino acid sequence as set forth in SEQ ID NO: 23 (GGCGGCGGTGGATCC), SEQ ID NO: 58 (EEKKKEKEKEEQEERETK) or SEQ ID NO: 59 (HHHHHHHHHH).

In some embodiments, the second polypeptide is selected from an Fc domain and albumin. In some embodiments, the Fc domain comprises a hinge region. In some embodiments, the Fc domain is derived from human IgG Fc domain. In some embodiments, the Fc domain is derived from human IgG1 Fc domain, human IgG2 Fc domain, human IgG3 Fc domain or human IgG4 Fc domain. In some embodiments, the Fc domain comprises an amino acid sequence having at least 70%, at least 75%, at least 80%, at least 85%, at least 90% or at least 95% sequence identity to SEQ ID NO: 24, SEQ ID NO: 25, SEQ ID NO: 32 or SEQ ID NO: 77. In some embodiments, the Fc domain comprises an amino acid sequence as set forth in SEQ ID NO: 24, SEQ ID NO: 25, SEQ ID NO: 32 or SEQ ID NO: 77. In some embodiments, the Fc domain has an amino acid mutation at a site corresponding to position 7 of SEQ ID NO: 25. In some embodiments, amino acid (e.g., alanine) at a site of the Fc domain is mutated to valine, glycine, serine or leucine, wherein the site corresponds to position 7 of SEQ ID NO: 25. In some embodiments, the Fc domain comprises one or more mutations that extend half-life of the fusion protein. In some embodiments, the Fc domain is linked to C-terminus or N-terminus of the first polypeptide. In some embodiments, the albumin comprises one or more domains of human serum albumin. In some embodiments, the albumin comprises a D3 domain of human serum albumin.

In some embodiments, the fusion protein provided herein further comprises a label. In some embodiments, the label is selected from the group consisting of a fluorescent label, a luminescent label, a purification label and a chromogenic label. In some embodiments, the label is selected from the group consisting of a c-Myc tag, an HA tag, a VSV-G tag, a FLAG tag, a V5 tag and a HIS tag. In some embodiments, the label is a HIS tag comprising 6, 7, 8, 9 or 10 histidine. In some embodiments, the second polypeptide is located at C-terminus of the first polypeptide and the label is located at C-terminus of the second polypeptide.

In some embodiments, the fusion protein provided herein has a half-life of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days in blood circulation of a subject.

In another aspect, the present disclosure provides an isolated nucleic acid comprising a nucleotide sequence encoding the truncated form of IgA protease or comprising a nucleotide sequence encoding the fusion protein provided herein. In some embodiments, the nucleic acid provided herein comprises a nucleotide sequence selected from the group consisting of SEQ ID NO: 3, SEQ ID NO: 5, SEQ ID NO: 7, SEQ ID NO: 9, SEQ ID NO: 11, SEQ ID NO: 13, SEQ ID NO: 33, SEQ ID NO: 34, SEQ ID NO: 35, SEQ ID NO: 36, SEQ ID NO: 37, SEQ ID NO: 38 and a nucleotide sequence having at least 70% sequence identity thereto.

In another aspect, the present disclosure provides a vector comprising the nucleic acid described herein.

In another aspect, the present disclosure provides a cell comprising the nucleic acid or the vector described herein. In some embodiments, the cell is a prokaryotic cell or a eukaryotic cell. In some embodiments, the prokaryotic cell is an *E. coli* cell. In some embodiments, the eukaryotic cell is a mammalian cell. In some embodiments, the mammalian cell is a human cell or a Chinese hamster ovary (CHO) cell. In some embodiments, the mammalian cell is a human embryonic kidney cell 293 (HEK293 cell).

In another aspect, the present disclosure provides a pharmaceutical composition comprising the truncated form of IgA protease described herein, comprising the fusion protein described herein, comprising the nucleic acid described herein, comprising the vector described herein or comprising the cell described herein, and a pharmaceutically acceptable carrier.

In another aspect, the present disclosure provides a method of producing a fusion protein comprising a step of culturing the cell described herein.

In another aspect, the present disclosure provides a method of treating or preventing a disease associated with IgA deposition, comprising administering to a subject in need thereof the truncated form of IgA protease described herein, the fusion protein described herein, or the pharmaceutical composition described herein.

In another aspect, the present disclosure provides use of the truncated form of IgA protease described herein, the fusion protein described herein, or the pharmaceutical composition described herein in the manufacture of a medicament for treating or preventing a disease associated with IgA deposition.

In another aspect, the present disclosure provides the truncated form of IgA protease described herein, the fusion protein described herein, or the pharmaceutical composition described herein for treating or preventing a disease associated with IgA deposition.

In another aspect, the present disclosure provides a method of treating or preventing a disease associated with IgA deposition, comprising administering to a subject in need thereof an IgA protease or a truncated form thereof, a fusion protein comprising the IgA protease or a truncated form thereof, or a pharmaceutical composition comprising the IgA protease or a truncated form thereof, wherein an amino acid sequence of the IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof.

In another aspect, the present disclosure provides use of an IgA protease or a truncated form thereof, a fusion protein comprising the IgA protease or a truncated form thereof, or a pharmaceutical composition comprising the IgA protease or a truncated form thereof in the manufacture of a medicament for treating or preventing a disease associated with IgA deposition, wherein an amino acid sequence of the IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof.

In another aspect, the present disclosure provides an IgA protease or a truncated form thereof, a fusion protein comprising the IgA protease or a truncated form thereof, or a pharmaceutical composition comprising the IgA protease or a truncated form thereof for treating or preventing a disease associated with IgA deposition, wherein an amino acid sequence of the IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof.

In some embodiments, the disease associated with IgA deposition is selected from the group consisting of IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis. In some embodiments, the disease associated with IgA deposition is IgA nephropathy, IgA vasculitis or Kawasaki disease.

### BRIEF DESCFRIPTION OF THE DRAWINGS

**Figure 1** shows the results *of in vitro* enzymatic cleavage activity assay of four truncated forms of IgA protease (*i.e.*, AK183 (31-737), AK183 (31-768), AK183 (31-798) and AK183 (31-833)) against IgA1.
**Figure 2** shows the results *of in vitro* enzymatic cleavage activity assay of five truncated forms of IgA protease (*i.e.*, AK183 (31-773), AK183 (31-778), AK183 (31-782), AK183 (31-787) and AK183 (31-792)) against IgA1.
**Figures 3a****-c** show the results *of in vitro* enzymatic cleavage activity assay of four truncated forms of IgA protease (*i.e.*, AK183 (31-788), AK183 (31-789), AK183 (31-790) and AK183 (31-791)) against IgA1.
**Figure 4** shows a flow chart of the PET30a-AK183(31-790)-Fc plasmid construction.
**Figure 5** shows the expression result of the AK183(31-790)-Fc fusion protein.
**Figure 6a** shows the expression results of the AK183(31-792)-Fc fusion protein and **Figure 6b** shows the results of the *in vitro* enzymatic activity assay of the AK183(31-792)-Fc fusion protein against IgAl.
**Figure 7** shows the results *of in vitro* enzymatic cleavage activity assay of four fusion proteins (*i.e*., AK183(31-798)-Fc, AK183(31-807)-Fc, AK183(31-816)-Fc and AK183(31-833)-Fc) against IgA1.
**Figure 8** shows the results of the *in vivo* enzymatic cleavage activity assay of AK183(31-807)-Fc fusion protein against IgA1.
**Figure 9** shows the expression result of AK183(31-792)-Fc fusion protein in HEK293 cells.
**Figure 10** shows the results *of in vitro* enzymatic cleavage activity assay of seven truncated forms of IgA protease (*i.e*., AK183(285-792), AK183(330-792), AK183(380-792), AK183(430-792), AK183(480-792), AK183(530-792) and AK183(580-792)) against IgA1.
**Figure 11** shows the results *of in vitro* enzymatic cleavage activity assay of nine truncated forms of IgA protease (*i.e*., AK183(335-792), AK183(340-792), AK183(345-792), AK183(350-792), AK183(355-792), AK183(360-792), AK183(365-792), AK183(370-792) and AK183(375-792)) against IgA1.
**Figure 12** shows the results *of in vitro* enzymatic cleavage activity assay of four truncated forms of IgA protease (*i.e*., AK183 (336-792), AK183 (337-792), AK183 (338-792) and AK183 (339-792)) against IgA1.
**Figure 13** shows the revalidation results *of in vitro* enzymatic cleavage activity assay of ten truncated forms of IgA protease (*i.e.*, AK183(285-792), AK183(330-792), AK183(335-792), AK183(336-792), AK183(337-792), AK183(338-792), AK183(339-792), AK183(340-792), AK183(345-792) and AK183(350-792)) against IgA1.
**Figure 14** shows the expression results of two fusion proteins (*i.e.*, AK183(285-816)-Fc and Fc-AK183(285-816)).
**Figure 15** shows the results *of in vitro* enzymatic activity assay of two fusion proteins (*i.e*., AK183(285-816)-Fc and Fc-AK183(285-816)) against IgA1.
**Figure 16** shows the results of the enzymatic cleavage activity assay of Fc-AK183(285-816) fusion protein, AK183(285-816)-Fc fusion protein and AK183(285-816) truncated form of IgA protease against IgA1.
**Figure 17** shows the result of *in vivo* enzymatic activity assay of Fc-AK183(285-816) fusion protein against IgA1.
**Figure 18** shows the results of the enzymatic cleavage activity assay of AK183(285-816)-Fc fusion protein and Fc-AK183(31-1203) fusion protein against IgA1.
**Figure 19** shows the results of the enzymatic cleavage activity assay of AK183(31-816)-IgG1 Fc fusion protein, AK183(31-816)-IgG4 Fc fusion protein and AK183(31-816)-albumin fusion protein against IgA1.
**Figure 20** shows the results of the enzymatic cleavage activity assay of AK183(285-816)-Fc fusion proteins with different linkers (SEQ ID NO: 59, SEQ ID NO: 58, SEQ ID NO: 22, SEQ ID NO: 78, SEQ ID NO: 79 or SEQ ID NO: 80) against IgA1.
**Figure 21** shows the results of the enzymatic cleavage activity assay of five mutants of the truncated form of IgA protease as set forth in SEQ ID NO: 53, SEQ ID NO: 54, SEQ ID NO: 55, SEQ ID NO: 56 or SEQ ID NO: 57 against IgA1.
**Figure 22** shows the results of the enzymatic cleavage activity assay of four mutants against IgAl, wherein the four mutants are formed by four different mutations in the Fc region of the AK183(31-816)-Fc fusion protein, respectively.
**Figure 23a** and **Figure 23b** show the results of the enzymatic cleavage activity assay of 16 enzymes homologous to AK183 against IgA1.

### DETAILED DESCRIPTION OF THE INVENTION

Although the present disclosure will disclose various aspects and embodiments below, it will be apparent to one skilled in the art that various equivalents, changes, and modifications may be made without departing from the scope of the disclosure, and it is understood that such equivalent embodiments are to be included herein. The various aspects and embodiments disclosed herein are for illustrative purposes only and are not intended to limit the scope of the present application, and the actual protection scope of this application is subject to the claims. Unless otherwise indicated, all technical and scientific terms used herein have the same meanings as those generally understood by those of ordinary skill in the art to which the present application belongs. All references cited herein, including publications, patents and patent applications are incorporated herein by reference in their entirety.

### Definitions

As used herein, the term "*Clostridium ramosum*" or "*Ramibacterium ramosum*" refers to a human intestinal commensal bacterium that produces IgA protease.

As used herein, the term "protease" refers to an enzyme that has the ability to break down proteins and peptides. Proteases can break down proteins by hydrolyzing peptide bonds that link amino acids together in a peptide or polypeptide chain that forms the protein. Various methods are known in the art for testing the proteolytic activity of a particular protease. For example, the protein hydrolytic activity of a protease can be determined by a comparative assay of analyzing the ability of various proteases to hydrolyze suitable substrates. Exemplary substrates for protein hydrolytic activity analysis include, for example, dimethyl casein, bovine collagen, bovine elastin and the like. Colorimetric assays using these substrates are also known in the art (see, for example, WO99/34011 and US 6,376,450).

As used herein, the term "IgA protease" refers to an enzyme that is capable of specifically cleaving or breaking down an IgA immunoglobulin molecule (*e.g*., IgA1 or IgA2) in a subject (*e.g.*, human). For example, an IgA protease obtained from or derived from *Clostridium ramosum* is capable of specifically cleaving the peptide bond between proline (Pro) at position 221 and valine (Val) at position 222 of IgA1 and IgA2, thereby breaking down IgA1 and IgA2.

When reference is made to a polypeptide or protein, the term "wild-type" used herein refers to a naturally occurring polypeptide or protein that does not include an artificial substitution, insertion, deletion or modification at one or more amino acid sites. When reference is made to a nucleic acid, nucleotide or polynucleotide, the term "wild-type" used herein refers to a naturally occurring nucleic acid, nucleotide or polynucleotide that does not include an artificial substitution, insertion, deletion or modification at one or more nucleotide sites. However, polynucleotides encoding wild-type polypeptides are not limited to naturally occurring polynucleotides, but also include any polynucleotide encoding a wild-type polypeptide.

As used herein, the term "AK183" refers to strain AK183 of *Clostridium ramosum.* Strain AK183 of *Clostridium ramosum* produces a wild-type IgA protease with the amino acid sequence as set forth in SEQ ID NO: 1 (wherein amino acids at positions 1 to 30 are the signal peptide).

| |
|---|
| 10 20 30 40 50 MTKKLMTKKI TAIFLALYMA ISVLPMTIQA ASKPDIKVGD YVKMGVYNNA 60 70 80 90 100 SILWRCVSID NNGPLMLADK IVDTLAYDAK TNDNSNSKSH SRSYKRDDYG 110 120 130 140 150 SNYWKDSNMR SWLNSTAAEG KVDWLCGNPP KDGYVSGVGA YNEKAGFLNA 160 170 180 190 200 FSKSEIAAMK TVTQRSLVSH PEYNKGIVDG DANSDLLYYT DISEAVANYD 210 220 230 240 250 SSYFETTTEK VFLLDVKQAN AVWKNLKGYY VAYNNDGMAW PYWLRTPVTD 260 270 280 290 300 CNHDMRYISS SGQVGRYAPW YSDLGVRPAF YLDSEYFVTT SGSGSQSSPY 310 320 330 340 350 IGSAPNKQED DYTISEPAED ANPDWNVSTE QSIQLTLGPW YSNDGKYSNP 360 370 380 390 400 TIPVYTIQKT RSDTENMVVV VCGEGYTKSQ QGKFINDVKR LWQDAMKYEP 410 420 430 440 450 YRSYADRFNV YALCTASEST FDNGGSTFFD VIVDKYNSPV ISNNLHGSQW 460 470 480 490 500 KNHIFERCIG PEFIEKIHDA HIKKKCDPNT IPSGSEYEPY YYVHDYIAQF 510 520 530 540 550 AMVVNTKSDF GGAYNNREYG FHYFISPSDS YRASKTFAHE FGHGLLGLGD 560 570 580 590 600 EYSNGYLLDD KELKSLNLSS VEDPEKIKWR QLLGFRNTYT CRNAYGSKML 610 620 630 640 650 VSSYECIMRD TNYQFCEVCR LQGFKRMSQL VKDVDLYVAT PEVKEYTGAY 660 670 680 690 700 SKPSDFTDLE TSSYYNYTYN RNDRLLSGNS KSRFNTNMNG KKIELRTVIQ 710 720 730 740 750 NISDKNARQL KFKMWIKHSD GSVATDSSGN PLQTVQTFDI PVWNDKANFW 760 770 780 790 800 PLGALDHIKS DFNSGLKSCS LIYQIPSDAQ LKSGDTVAFQ VLDENGNVLA 810 820 830 840 850 DDNTETQRYT TVSIQYKFED GSEIPNTAGG TFTVPYGTKL DLTPAKTLYD 860 870 880 890 900 YEFIKVDGLN KPIVSDGTVV TYYYKNKNEE HTHNLTLVAA KAATCTTAGN 910 920 930 940 950 SAYYTCDGCD KWFADATGSV EITDKTSVKI PAPGHTAGTE WKSDDTNHWH 960 970 980 990 1000 ECTVAGCGVI IESTKSAHTA GEWIVDTPAT ATTAGTKHKE CTVCHRVLET 1010 1020 1030 1040 1050 QPIPSTGTEL KIIAGDNQIY NKASGSDVTI TCNGDFAKFT GIKVDGSVVD 1060 1070 1080 1090 1100 |
| SSNYTAVSGS TVLTLKASYL GTLTDGSHTI TFVYTDGEAN ANLTVRTAGS 1110          1120          1130          1140          1150 GHIHDYGTEW KSNADNHWHE CNCGDKKDEA AHSFKWVVDK EATATKKGSK 1160          1170          1180          1190          1200 HEECKICGYK RSAVEIPATG TSTAPTDTTK PNDTTKPGNT NGSEKSPQTG 1210          1220          1230 DNSNIFLWFA LLFVSAAGVT GITAYNKKKK EHAE (SEQ ID NO: 1) |

As used herein, the term "signal peptide" refers to a sequence of amino acid residues that can participate in the secretion or direct transport of a mature or precursor form of a protein. The signal peptide is usually located at the N-terminus of the precursor or mature protein sequence. Signal peptides can be endogenous or exogenous. A signal peptide is normally absent from the mature protein. A signal sequence is typically cleaved from the protein by a signal peptidase after the protein is transported. For example, after removing the signal peptide from the N-terminus, the amino acid sequence as set forth in SEQ ID NO: 1 forms the amino acid sequence as set forth in SEQ ID NO: 42.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA
NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD
YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI

SDKNARQLKFKMWIKHSDGSVATDSSGNPLQTVQTFDIPVWNDKANFWPLG
ALDHIKSDFNSGLKSCSLIYQIPSDAQLKSGDTVAFQVLDENGNVLADDNTET
QRYTTVSIQYKFEDGSEIPNTAGGTFTVPYGTKLDLTPAKTLYDYEFIKVDGL
NKPIVSDGTVVTYYYKNKNEEHTHNLTLVAAKAATCTTAGNSAYYTCDGCD
KWFADATGSVEITDKTSVKIPAPGHTAGTEWKSDDTNHWHECTVAGCGVIIE
STKSAHTAGEWIVDTPATATTAGTKHKECTVCHRVLETQPIPSTGTELKIIAGD
NQIYNKASGSDVTITCNGDFAKFTGIKVDGSVVDSSNYTAVSGSTVLTLKASY
LGTLTDGSHTITFVYTDGEANANLTVRTAGSGHIHDYGTEWKSNADNHWHE
CNCGDKKDEAAHSFKWVVDKEATATKKGSKHEECKICGYKRSAVEIPATGT
STAPTDTTKPNDTTKPGNTNGSEKSPQTGDNSNIFLWFALLFVSAAGVTGITA
YNKKKKEHAE (SEQ ID NO: 42)

As used herein, the term "subject" includes both human and non-human animals. Non-human animals include all vertebrate animals, such as mammals and non-mammals. A "subject" may also be a domestic animal, such as cattle, pigs, sheep, poultry and horses; or a rodent, such as rats, mice; or a primate, such as apes, monkeys, chimpanzees, gorillas, orangutans, baboons; or domesticated animals, such as dogs and cats. A "subject" may be male or female and may be elderly, adult, adolescent, child or infant. A human "subject" may be Caucasian, African, Asian, Semitic, or other races or a combination of these ethnic backgrounds.

As used herein, the terms "protein", "polypeptide" and "peptide" are used interchangeably and refer to a polymer of amino acids. The protein, polypeptide or peptide described herein may contain naturally occurring amino acids, or may contain non-naturally occurring amino acids, or analogues or mimics of amino acids. The protein, polypeptide or peptide described herein may be obtained by any method known in the art, for example, but not limited to, by natural isolation, recombinant expression, chemical synthesis, and the like.

The term "amino acid" used herein refers to an organic compound containing amino (-NH₂) and carboxyl (-COOH) functional groups and a side chain specific to each amino acid. The names of amino acid are also represented in this application by standard single-letter or three-letter codes, which are summarized as follows:

| **Name** | **Three-letter code** | **Single-letter code** |
|---|---|---|
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

A "conservative substitution" with reference to amino acid sequence refers to replacing an amino acid residue with a different amino acid residue having a side chain with similar physiochemical properties. For example, conservative substitutions can be made among amino acid residues with hydrophobic side chains (*e.g*., Met, Ala, Val, Leu, and Ile), among residues with neutral hydrophilic side chains (*e.g*., Cys, Ser, Thr, Asn and Gln), among residues with acidic side chains (*e.g*., Asp, Glu), among amino acids with basic side chains (*e.g*., His, Lys, and Arg), or among residues with aromatic side chains (*e.g.*, Trp, Tyr, and Phe). As known in the art, conservative substitution usually does not cause significant change in the protein conformational structure, and therefore could retain the biological activity of a protein.

As used herein, the term "homologous" refers to a nucleic acid sequence (or its complementary strand) or amino acid sequence having at least 60% (*e.g*., at least 65%, 70%, 75%, 80%, 85%, 88%, 90%, 91%, 92%, 93%, 94%, 95%, 96%, 97%, 98%, 99%) sequence identity to another sequence when optimally aligned.

As used herein, the term "percent (%) sequence identity" is defined as the percentage of amino acid (or nucleic acid) residues in a candidate sequence that are identical to the amino acid (or nucleic acid) residues in a reference sequence, after aligning the sequences and, if necessary, introducing gaps, to achieve the maximum number of identical amino acids (or nucleic acids). In other words, percent (%) sequence identity of an amino acid sequence (or nucleic acid sequence) can be calculated by dividing the number of amino acid residues (or bases) that are identical relative to the reference sequence to which it is being compared by the total number of the amino acid residues (or bases) in the candidate sequence or in the reference sequence, whichever is shorter. Conservative substitution of the amino acid residues may or may not be considered as identical residues. Alignment for purposes of determining percent amino acid (or nucleic acid) sequence identity can be achieved, for example, using publicly available tools such as BLASTN, BLASTp (available on the website of U.S. National Center for Biotechnology Information (NCBI), see also, Altschul S.F. et al., J. Mol. Biol., 215:403-410 (1990); Stephen F. et al., Nucleic Acids Res., 25:3389-3402 (1997)), ClustalW2 (available on the website of European Bioinformatics Institute, see also, Higgins D.G. et al., Methods in Enzymology, 266:383-402 (1996); Larkin M.A. et al., Bioinformatics (Oxford, England), 23(21): 2947-8 (2007)), and ALIGN or Megalign (DNASTAR) software. Those skilled in the art may use the default parameters provided by the tool or may customize the parameters as appropriate for the alignment, such as for example, by selecting a suitable algorithm.

An "isolated" substance has been artificially altered from its natural state. If an "isolated" composition or substance occurs in nature, it has been altered or removed from its original state, or both. For example, naturally occurring polynucleotides or polypeptides in a living animal are not "isolated", but may be considered "isolated" if they are sufficiently separate from the substance with which they coexist in their natural state and exist in an essentially pure state. An "isolated nucleic acid sequence" refers to the sequence of the isolated nucleic acid molecule. In some embodiments, an "isolated truncated form of IgA protease" refers to a truncated form of IgA protease with a purity of at least 60%, 70%, 75%, 80%, 81%, 82%, 83%, 84%, 85%, 86%, 87%, 88%, 89%, 90%, 91%, 92%, 93%, 94%, 95%, 96%, 97%, 98%. 96%, 97%, 98%, or 99%, wherein the purity is determined by electrophoretic methods (*e.g*., SDS-PAGE, isoelectric focusing, capillary electrophoresis), or chromatographic methods (*e.g*., ion exchange chromatography or reversed-phase HPLC).

The term "vector" as used herein refers to a vehicle into which a genetic element may be operably inserted so as to bring about the expression of that genetic element, such as to produce the protein encoded by the genetic element, RNA or DNA, or to replicate the genetic element. A vector may be used to transform, transduce, or transfect a host cell so as to bring about expression of the genetic element it carries within the host cell. Examples of vectors include plasmids, phagemids, cosmids, and artificial chromosomes such as yeast artificial chromosome (YAC), bacterial artificial chromosome (BAC), or P1-derived artificial chromosome (PAC), bacteriophages such as lambda phage or M13 phage, and animal viruses. A vector may contain a variety of elements for controlling expression, including a promoter sequence, a transcription initiation sequence, an enhancer sequence, a selectable element, and a reporter gene. In addition, the vector may further contain an origin of replication. A vector may also include materials to aid in its entry into the cell, including but not limited to a viral particle, a liposome, or a protein coating. A vector can be an expression vector or a cloning vector. The present disclosure provides vectors (*e.g*., expression vectors) comprising the nucleic acid sequence provided herein encoding the truncated form of IgA protease or fusion protein, at least one promoter (*e.g*., SV40, CMV, EF-1α) operably linked to the nucleic acid sequence, and at least one selection marker.

As used herein, a "treatment" or "therapy" for a disease, disorder or condition comprises preventing or alleviating a disease, disorder or condition, reducing the rate of occurrence or progression of a disease, disorder or condition, reducing the risk of developing a disease, disorder or condition, preventing or delaying the development of symptoms associated with a disease, disorder or condition, reducing or terminating symptoms associated with a disease, disorder or condition, generating a complete or partial reversal of a disease, disorder or condition, and curing a disease, disorder or condition, or a combination of the above.

The term "pharmaceutically acceptable" indicates that the designated carrier, medium, diluent, excipient and/or salt is generally chemically and/or physically compatible with the other ingredients that constitute the formulation and physiologically compatible with the recipient thereof.

The term "disease associated with IgA deposition" refers to a disease associated with an accumulation of IgA immunoglobulin (in an aggregated or non-aggregated form) in a tissue or organ of a subject. For example, a disease associated with IgA deposition includes but is not limited to, IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis.

The term "IgA nephropathy" refers to a kidney disease characterized by IgA deposition in the kidney.

### Truncated form of IgA protease

In one aspect, the present disclosure provides an isolated truncated form of IgA protease comprising a non-natural truncated fragment of a wild-type IgA protease obtained from or derived from *Clostridium ramosum* or having at least 70% sequence identity (*e.g*., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to the non-natural truncated fragment. In some embodiments, a truncated form of IgA protease having at least 70% sequence identity (*e.g*., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to the non-natural truncated fragment still retains the function or activity of the IgA protease (*e.g*., protein hydrolytic activity, enzymatic activity of specifically cleaving IgA, *etc*.)*.*

As used herein, the term "truncated form" or "truncated fragment" refers to a peptide formed by removing one or more amino acids from one or both ends of a wild-type polypeptide. Thus, a "truncated form" or "truncated fragment" described herein does not include the full length of the corresponding wild-type polypeptide, but may have one or more amino acid substitutions, deletions, insertions or modifications compared to the truncated form of the wild-type polypeptide. For example, a "truncated form of IgA protease" or a "truncated fragment of IgA protease" may comprise a peptide formed by removing one or more amino acids from one or both ends of a wild-type IgA protease, or may comprise a peptide with one or more amino acid substitutions, deletions, insertions or modifications compared to a truncated form of the wild-type IgA protease.

In some embodiments, the truncated form of the IgA protease described herein has one or more amino acid substitutions, deletions, insertions or modifications compared to its corresponding wild-type IgA protease. For example, in some embodiments, the truncated form of IgA protease described herein comprises a non-natural truncated fragment of a wild-type IgA protease obtained from or derived from *Clostridium ramosum,* wherein the non-natural truncated fragment has an amino acid substitution, deletion, insertion or modification compared to the wild-type IgA protease of *Clostridium ramosum,* such that the truncated form of IgA protease loses or reduces its self-cleaving function.

As used herein, the terms "obtained from" and "derived from" include not only a protein produced or producible by the organism in question, but also a protein encoded by a DNA sequence isolated from such organism and produced in a host organism containing such DNA sequence. Additionally, the terms also include a protein encoded by a DNA sequence of synthetic and/or cDNA origin and which has the identified characteristics of the protein in question. For example, a wild-type IgA protease obtained from or derived from *Clostridium ramosum* includes both an IgA protease that is naturally produced by *Clostridium ramosum,* as well as an IgA protease produced by other host cells (*e.g*., *E. coli*) transformed with a nucleic acid encoding the IgA protease by using genetic engineering techniques.

As used herein, the term "non-natural truncated fragment" refers to a fragment with an amino acid sequence that is different (*e.g.*, different amino acid length, different amino acid type, etc.) from the amino acid sequence of the truncated fragment formed by self-cleavage of the wild-type IgA protease of *Clostridium ramosum* in natural environment.

In some embodiments, the amino acid substitution, deletion, insertion or modification occurs at a natural self-cleaving site of the wild-type IgA protease of *Clostridium ramosum.* In some embodiments, the amino acid substitution, deletion, insertion or modification occurs within 5 sites upstream of the natural self-cleaving site (*e.g*., 1 site, 2 sites, 3 sites, 4 sites or 5 sites upstream of the natural self-cleaving site) of the wild-type IgA protease of *Clostridium ramosum.* In some embodiments, the amino acid substitution, deletion, insertion or modification occurs within 5 sites downstream of the natural self-cleaving site (*e.g*., 1 site, 2 sites, 3 sites, 4 sites or 5 sites downstream of the natural self-cleaving site) of the wild-type IgA protease of *Clostridium ramosum.* In some embodiments, the amino acid substitution, deletion, insertion or modification occurs within 5 sites (*e.g*., 1 site, 2 sites, 3 sites, 4 sites or 5 sites) upstream of the natural self-cleaving site and 5 sites (*e.g*., 1 site, 2 sites, 3 sites, 4 sites or 5 sites) downstream of the natural self-cleaving site of the wild-type IgA protease of *Clostridium ramosum.*

In some embodiments, the non-natural truncated fragment is aN-terminal truncated fragment or C-terminal truncated fragment of a wild-type IgA protease obtained from or derived from *Clostridium ramosum.*

As used herein, the term "N-terminal truncated fragment" refers to a truncated fragment comprising an amino acid sequence of the amino terminus of a wild-type IgA protease of *Clostridium ramosum.* The "amino terminus" may start at any site adjacent to the amino terminus of an amino acid sequence of a wild-type IgA protease of *Clostridium ramosum,* for example, at site 1 numbering from the amino terminus, or at some other site numbering from the amino terminus. For another example, if the full-length amino acid sequence of a wild-type IgA protease consists of 1000 amino acids, the amino-terminal start position of its N-terminal truncated fragment may be anywhere between site 1 and site 500 of its amino acid sequence counting from the amino terminus.

As used herein, the term "C-terminal truncated fragment" refers to a truncated fragment comprising an amino acid sequence of the carboxyl terminus of a wild-type IgA protease of *Clostridium ramosum.* The "carboxyl terminus" may terminate at any site adjacent to the carboxyl terminus of an amino acid sequence of a wild-type IgA protease of *Clostridium ramosum,* for example, at site 1 numbering from the carboxyl terminus, or at some other site numbering from the carboxyl terminus. For another example, if the full-length amino acid sequence of a wild-type IgA protease consists of 1000 amino acids, the carboxyl-terminal end position of its C-terminal truncated fragment may be anywhere between site 501 and site 1000 of its amino acid sequence counting from the amino terminus.

*Clostridium ramosum* is one of various species in the genus *Clostridium,* including a variety of strains such as AK183, VPI-0496A, NCTC 10474 and the like. In some embodiments, the *Clostridium ramosum* is *Clostridium ramosum* AK183 strain.

In some embodiments, the N-terminal truncated fragment comprises a polypeptide fragment of at least 760 continuous amino acids starting from position 31 of the N-terminus of a wild-type IgA protease obtained from or derived from *Clostridium ramosum,* or having at least 70% sequence identity (*e.g.*, having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to the polypeptide fragment. In some embodiments, a N-terminal truncated fragment of IgA protease having at least 70% sequence identity (*e.g*., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to the polypeptide fragment still retains the function or activity of the IgA protease (*e.g*., protein hydrolytic activity, enzymatic activity of specifically cleaving IgA, etc.).

In some embodiments, the non-natural truncated fragment of IgA protease described herein comprises a polypeptide fragment of at least 456 continuous amino acids starting from position 335 of the N-terminus of a wild-type IgA protease obtained from or derived from *Clostridium ramosum* or having at least 90% or at least 95% sequence identity (*e.g*., at least 91%, at least 92%, at least 93%, at least 94%, at least 95%, at least 96%, at least 97%, at least 98%, at least 99% sequence identity) to the polypeptide fragment. In some embodiments, a non-natural truncated fragment having at least 90% or at least 95% sequence identity (*e.g.*, at least 91%, at least 92%, at least 93%, at least 94%, at least 95%, at least 96%, at least 97%, at least 98%, at least 99% sequence identity) to the polypeptide fragment still retains the function or activity of the IgA protease (*e.g*., protein hydrolytic activity, enzymatic activity of specifically cleaving IgA, etc.)

In some embodiment, an amino acid sequence of the wild-type IgA protease of *Clostridium ramosum* is as set forth in SEQ ID NO: 1.

Unless otherwise stated, the amino acid positions of an IgA protease referred to herein correspond to the wild-type AK183 IgA protease (its amino acid sequence is as set forth in SEQ ID NO: 1). For example, position 790 of the AK183 IgA protease described herein corresponds to position 790 of SEQ ID NO: 1. Unless otherwise stated, the truncated form of AK183 IgA protease described herein is named according to the naming convention of AK183 (start position corresponding to SEQ ID NO: 1 - end position corresponding to SEQ ID NO: 1). For example, AK183 (31-790) refers to the truncated form of IgA protease formed by amino acids from position 31 to position 790 of SEQ ID NO: 1.

In some embodiments, the natural self-cleaving site of the IgA protease described herein is between position 730 and position 840 of the amino acid sequence as set forth in SEQ ID NO: 1. In some embodiments, the natural self-cleaving site of the IgA protease described herein is between position 710 and position 830, between position 720 and position 820, between position 730 and position 810, between position 740 and position 800, between position 750 and position 790, between position 791 and position 780 or between position 792 and position 797 of the amino acid sequence as set forth in SEQ ID NO: 1. In some embodiments, the natural self-cleaving site is at position 790, position 791, position 792, position 793, position 794, position 795, position 796, position 797, position 798, position 799 or position 800 of the amino acid sequence as set forth in SEQ ID NO: 1.

In some embodiments, the truncated form of IgA protease provided herein comprises a polypeptide fragment of at least 760 continuous amino acids starting from position 31 of the amino acid sequence as set forth in SEQ ID NO: 1. For example, in some embodiments, the truncated form of IgA protease provided herein comprises a polypeptide fragment of at least 761, at least 762, at least 763, at least 764, at least 765, at least 766, at least 767, at least 768, at least 769, at least 770, at least 771, at least 772, at least 773, at least 774, at least 775, at least 776, at least 777, at least 778, at least 779, at least 780, at least 781, at least 782, at least 783, at least 784, at least 785, at least 786, at least 787, at least 788, at least 789, at least 790, at least 791, at least 792, at least 793, at least 794, at least 795, at least 796, at least 797, at least 798, at least 799, at least 800, at least 801, at least 802, at least 803, at least 804, at least 805, at least 806, at least 807, at least 808, at least 809, at least 810, at least 850, at least 860, at least 870, at least 880, at least 890, at least 900, at least 910, at least 920, at least 930, at least 940, at least 950, at least 960, at least 970, at least 980, at least 990, at least 1000, at least 1050, at least 1100, at least 1150 or at least 1200 continuous amino acids starting from position 31 of the amino acid sequence as set forth in SEQ ID NO: 1.

In some embodiments, the truncated form of IgA protease provided herein comprises a polypeptide fragment of 760 continuous amino acids starting from position 31 of the amino acid sequence as set forth in SEQ ID NO: 1. In some embodiments, the truncated form of IgA protease provided herein comprises a polypeptide fragment of 761 continuous amino acids starting from position 31 of the amino acid sequence as set forth in SEQ ID NO: 1. In some embodiments, the truncated form of IgA protease provided herein comprises a polypeptide fragment of 762 continuous amino acids starting from position 31 of the amino acid sequence as set forth in SEQ ID NO: 1. In some embodiments, the truncated form of IgA protease provided herein comprises a polypeptide fragment of 768 continuous amino acids starting from position 31 of the amino acid sequence as set forth in SEQ ID NO: 1. In some embodiments, the truncated form of IgA protease provided herein comprises a polypeptide fragment of 777 continuous amino acids starting from position 31 of the amino acid sequence as set forth in SEQ ID NO: 1. In some embodiments, the truncated form of IgA protease provided herein comprises a polypeptide fragment of 786 continuous amino acids starting from position 31 of the amino acid sequence as set forth in SEQ ID NO: 1. In some embodiments, the truncated form of IgA protease provided herein comprises a polypeptide fragment of 803 continuous amino acids starting from position 31 of the amino acid sequence as set forth in SEQ ID NO: 1.

In some embodiments, the truncated form of IgA protease provided herein comprises a polypeptide fragment selected from the group consisting of amino acids from position 31 to position 790 of the amino acid sequence as set forth in SEQ ID NO: 1, amino acids from position 31 to position 792 of the amino acid sequence as set forth in SEQ ID NO: 1, amino acids from position 31 to position 798 of the amino acid sequence as set forth in SEQ ID NO: 1, amino acids from position 31 to position 807 of the amino acid sequence as set forth in SEQ ID NO: 1, amino acids from position 31 to position 816 of the amino acid sequence as set forth in SEQ ID NO: 1, amino acids from position 31 to position 833 of the amino acid sequence as set forth in SEQ ID NO: 1, and a polypeptide fragment having at least 70% sequence identity (*e.g*., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) thereto. In some embodiments, a truncated form of IgA protease has at least 70% sequence identity (*e.g.*, having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to the polypeptide fragment still retains the function or activity of the IgA protease (*e.g*., protein hydrolytic activity, enzymatic activity of specifically cleaving IgA, etc.).

In some embodiments, the truncated form of IgA protease provided herein comprises a polypeptide fragment of at least 456 continuous amino acids starting from position 335 of the amino acid sequence as set forth in SEQ ID NO: 1. For example, in some embodiments, the truncated form of IgA protease provided herein comprises a polypeptide fragment of at least 457, at least 458, at least 459, at least 460, at least 461, at least 462, at least 463, at least 464, at least 465, at least 466, at least 467, at least 468, at least 469, at least 470, at least 471, at least 472, at least 473, at least 474, at least 475, at least 476, at least 477, at least 478, at least 479, at least 480, at least 481, at least 482, at least 483, at least 484, at least 485, at least 486, at least 487, at least 488, at least 489, at least 490, at least 491, at least 492, at least 493, at least 494, at least 495, at least 496, at least 497, at least 498, at least 499, at least 500, at least 550, at least 600, at least 650, at least 700, at least 750, at least 800, at least 850 or at least 900 continuous amino acids starting from position 335 of the amino acid sequence as set forth in SEQ ID NO: 1.

In some embodiments, the truncated form of IgA protease provided herein comprises a polypeptide fragment selected from the group consisting of amino acids from position 335 to position 790 of the amino acid sequence as set forth in SEQ ID NO: 1, amino acids from position 335 to position 791 of the amino acid sequence as set forth in SEQ ID NO: 1, amino acids from position 335 to position 792 of the amino acid sequence as set forth in SEQ ID NO: 1, amino acids from position 285 to position 790 of the amino acid sequence as set forth in SEQ ID NO: 1, amino acids from position 285 to position 791 of the amino acid sequence as set forth in SEQ ID NO: 1, amino acids from position 285 to position 792 of the amino acid sequence as set forth in SEQ ID NO: 1, amino acids from position 330 to position 790 of the amino acid sequence as set forth in SEQ ID NO: 1, amino acids from position 330 to position 791 of the amino acid sequence as set forth in SEQ ID NO: 1, amino acids from position 330 to position 792 of the amino acid sequence as set forth in SEQ ID NO: 1, amino acids from position 285 to position 816 of the amino acid sequence as set forth in SEQ ID NO: 1, and a polypeptide sequence having at least 90% or at least 95% sequence identity (*e.g*., at least 91%, at least 92%, at least 93%, at least 94%, at least 95%, at least 96%, at least 97%, at least 98%, at least 99% sequence identity) thereto. In some embodiments, a truncated form of IgA protease having at least 90% or at least 95% sequence identity (*e.g*., at least 91%, at least 92%, at least 93%, at least 94%, at least 95%, at least 96%, at least 97%, at least 98%, at least 99% sequence identity) to the polypeptide fragment still retains the function or activity of the IgA protease (*e.g*., protein hydrolytic activity, enzymatic activity of specifically cleaving IgA, etc.).

In some embodiments, the present disclosure provides the truncated form of AK183(31-790) whose amino acid sequence is as set forth in SEQ ID NO: 14.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA
NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD
YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI
SDKNARQLKFKMWIKHSDGSVATDSSGNPLQTVQTFDIPVWNDKANFWPLG
ALDHIKSDFNSGLKSCSLIYQIPSDAQLKSGDTVAFQ (SEQ ID NO: 14)

In some embodiments, the present disclosure provides the truncated form of AK183(31-791) whose amino acid sequence is as set forth in SEQ ID NO: 15.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA

NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD
YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI
SDKNARQLKFKMWIKHSDGSVATDSSGNPLQTVQTFDIPVWNDKANFWPLG
ALDHIKSDFNSGLKSCSLIYQIPSDAQLKSGDTVAFQV (SEQ ID NO: 15)

In some embodiments, the present disclosure provides the truncated form of AK183(31-792) whose amino acid sequence is as set forth in SEQ ID NO: 16.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA
NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD
YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI
SDKNARQLKFKMWIKHSDGSVATDSSGNPLQTVQTFDIPVWNDKANFWPLG
ALDHIKSDFNSGLKSCSLIYQIPSDAQLKSGDTVAFQVL (SEQ ID NO: 16)

In some embodiments, the present disclosure provides the truncated form of AK183(31-798) whose amino acid sequence is as set forth in SEQ ID NO: 17.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA
NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD
YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI
SDKNARQLKFKMWIKHSDGSVATDSSGNPLQTVQTFDIPVWNDKANFWPLG
ALDHIKSDFNSGLKSCSLIYQIPSDAQLKSGDTVAFQVLDENGNV (SEQ ID
NO: 17)

In some embodiments, the present disclosure provides the truncated form of AK183(31-807) whose amino acid sequence is as set forth in SEQ ID NO: 18.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA
NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD

YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI
SDKNARQLKFKMWIKHSDGSVATDSSGNPLQTVQTFDIPVWNDKANFWPLG
ALDHIKSDFNSGLKSCSLIYQIPSDAQLKSGDTVAFQVLDENGNVLADDNTET
Q (SEQ ID NO: 18)

In some embodiments, the present disclosure provides the truncated form of AK183(31-816) whose amino acid sequence is as set forth in SEQ ID NO: 19.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA
NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD
YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI
SDKNARQLKFKMWIKHSDGSVATDSSGNPLQTVQTFDIPVWNDKANFWPLG
ALDHIKSDFNSGLKSCSLIYQIPSDAQLKSGDTVAFQVLDENGNVLADDNTET
QRYTTVSIQY    (SEQ ID NO: 19)

In some embodiments, the present disclosure provides the truncated form of AK183(31-833) whose amino acid sequence is as set forth in SEQ ID NO: 20.

ASKPDIKVGDYVKMGVYNNASILWRCVSIDNNGPLMLADKIVDTLAYDAKT
NDNSNSKSHSRSYKRDDYGSNYWKDSNMRSWLNSTAAEGKVDWLCGNPPK
DGYVSGVGAYNEKAGFLNAFSKSEIAAMKTVTQRSLVSHPEYNKGIVDGDA
NSDLLYYTDISEAVANYDSSYFETTTEKVFLLDVKQANAVWKNLKGYYVAY
NNDGMAWPYWLRTPVTDCNHDMRYISSSGQVGRYAPWYSDLGVRPAFYLD
SEYFVTTSGSGSQSSPYIGSAPNKQEDDYTISEPAEDANPDWNVSTEQSIQLTL
GPWYSNDGKYSNPTIPVYTIQKTRSDTENMVVVVCGEGYTKSQQGKFINDVK
RLWQDAMKYEPYRSYADRFNVYALCTASESTFDNGGSTFFDVIVDKYNSPVI
SNNLHGSQWKNHIFERCIGPEFIEKIHDAHIKKKCDPNTIPSGSEYEPYYYVHD
YIAQFAMVVNTKSDFGGAYNNREYGFHYFISPSDSYRASKTFAHEFGHGLLG
LGDEYSNGYLLDDKELKSLNLSSVEDPEKIKWRQLLGFRNTYTCRNAYGSK
MLVSSYECIMRDTNYQFCEVCRLQGFKRMSQLVKDVDLYVATPEVKEYTGA
YSKPSDFTDLETSSYYNYTYNRNDRLLSGNSKSRFNTNMNGKKIELRTVIQNI
SDKNARQLKFKMWIKHSDGSVATDSSGNPLQTVQTFDIPVWNDKANFWPLG
ALDHIKSDFNSGLKSCSLIYQIPSDAQLKSGDTVAFQVLDENGNVLADDNTET
QRYTTVSIQYKFEDGSEIPNTAGGTFT (SEQ ID NO: 20)

In some embodiments, the present disclosure provides the truncated form of AK183(285-790) whose amino acid sequence is as set forth in SEQ ID NO: 43. In some embodiments, the present disclosure provides the truncated form of AK183(285-791) whose amino acid sequence is as set forth in SEQ ID NO: 44. In some embodiments, the present disclosure provides the truncated form of AK183(285-792) whose amino acid sequence is as set forth in SEQ ID NO: 45. In some embodiments, the present disclosure provides the truncated form of AK183(285-816) whose amino acid sequence is as set forth in SEQ ID NO: 46. In some embodiments, the present disclosure provides the truncated form of AK183(330-790) whose amino acid sequence is as set forth in SEQ ID NO: 47. In some embodiments, the present disclosure provides the truncated form of AK183(330-791) whose amino acid sequence is as set forth in SEQ ID NO: 48. In some embodiments, the present disclosure provides the truncated form of AK183(330-792) whose amino acid sequence is as set forth in SEQ ID NO: 49. In some embodiments, the present disclosure provides the truncated form of AK183(335-790) whose amino acid sequence is as set forth in SEQ ID NO: 50. In some embodiments, the present disclosure provides the truncated form of AK183(335-791) whose amino acid sequence is as set forth in SEQ ID NO: 51. In some embodiments, the present disclosure provides the truncated form of AK183(335-792) whose amino acid sequence is as set forth in SEQ ID NO: 52.

The sequences of SEQ ID NOs: 43 ~ 52 are shown below.

| **SEQ ID NO** | **Sequence Description** | **Amino Acid Sequence** |
|---|---|---|
| 43 | AK183(285-790) | EYFVTTSGSGSQSSPYIGSAPNKQEDDYTISEP AEDANPDWNVSTEQSIQLTLGPWYSNDGKYS NPTIPVYTIQKTRSDTENMVVVVCGEGYTKSQ QGKFINDVKRLWQDAMKYEPYRSYADRFNV YALCTASESTFDNGGSTFFDVIVDKYNSPVISN NLHGSQWKNHIFERCIGPEFIEKIHDAHIKKKC DPNTIPSGSEYEPYYYVHDYIAQFAMVVNTKS DFGGAYNNREYGFHYFISPSDSYRASKTFAHE FGHGLLGLGDEYSNGYLLDDKELKSLNLSSV EDPEKIKWRQLLGFRNTYTCRNAYGSKMLVS SYECIMRDTNYQFCEVCRLQGFKRMSQLVKD VDLYVATPEVKEYTGAYSKPSDFTDLETSSYY NYTYNRNDRLLSGNSKSRFNTNMNGKKIELR TVIQNISDKNARQLKFKMWIKHSDGSVATDSS GNPLQTVQTFDIPVWNDKANFWPLGALDHIK SDFNSGLKSCSLIYQIPSDAQLKSGDTVAFQ |
| 44 | AK183(285-791) | EYFVTTSGSGSQSSPYIGSAPNKQEDDYTISEP AEDANPDWNVSTEQSIQLTLGPWYSNDGKYS NPTIPVYTIQKTRSDTENMVVVVCGEGYTKSQ |
| | | QGKFINDVKRLWQDAMKYEPYRSYADRFNV YALCTASESTFDNGGSTFFDVIVDKYNSPVISN NLHGSQWKNHIFERCIGPEFIEKIHDAHIKKKC DPNTIPSGSEYEPYYYVHDYIAQFAMVVNTKS DFGGAYNNREYGFHYFISPSDSYRASKTFAHE FGHGLLGLGDEYSNGYLLDDKELKSLNLSSV EDPEKIKWRQLLGFRNTYTCRNAYGSKMLVS SYECIMRDTNYQFCEVCRLQGFKRMSQLVKD VDLYVATPEVKEYTGAYSKPSDFTDLETSSYY NYTYNRNDRLLSGNSKSRFNTNMNGKKIELR TVIQNISDKNARQLKFKMWIKHSDGSVATDSS GNPLQTVQTFDIPVWNDKANFWPLGALDHIK SDFNSGLKSCSLIYQIPSDAQLKSGDTVAFQV |
| 45 | AK183(285-792) | EYFVTTSGSGSQSSPYIGSAPNKQEDDYTISEP AEDANPDWNVSTEQSIQLTLGPWYSNDGKYS NPTIPVYTIQKTRSDTENMVVVVCGEGYTKSQ QGKFINDVKRLWQDAMKYEPYRSYADRFNV YALCTASESTFDNGGSTFFDVIVDKYNSPVISN NLHGSQWKNHIFERCIGPEFIEKIHDAHIKKKC DPNTIPSGSEYEPYYYVHDYIAQFAMVVNTKS DFGGAYNNREYGFHYFISPSDSYRASKTFAHE FGHGLLGLGDEYSNGYLLDDKELKSLNLSSV EDPEKIKWRQLLGFRNTYTCRNAYGSKMLVS SYECIMRDTNYQFCEVCRLQGFKRMSQLVKD VDLYVATPEVKEYTGAYSKPSDFTDLETSSYY NYTYNRNDRLLSGNSKSRFNTNMNGKKIELR TVIQNISDKNARQLKFKMWIKHSDGSVATDSS GNPLQTVQTFDIPVWNDKANFWPLGALDHIK SDFNSGLKSCSLIYQIPSDAQLKSGDTVAFQVL |
| 46 | AK183(285-816) | EYFVTTSGSGSQSSPYIGSAPNKQEDDYTISEP AEDANPDWNVSTEQSIQLTLGPWYSNDGKYS NPTIPVYTIQKTRSDTENMVVVVCGEGYTKSQ |
| | | QGKFINDVKRLWQDAMKYEPYRSYADRFNV YALCTASESTFDNGGSTFFDVIVDKYNSPVISN NLHGSQWKNHIFERCIGPEFIEKIHDAHIKKKC DPNTIPSGSEYEPYYYVHDYIAQFAMVVNTKS DFGGAYNNREYGFHYFISPSDSYRASKTFAHE FGHGLLGLGDEYSNGYLLDDKELKSLNLSSV EDPEKIKWRQLLGFRNTYTCRNAYGSKMLVS SYECIMRDTNYQFCEVCRLQGFKRMSQLVKD VDLYVATPEVKEYTGAYSKPSDFTDLETSSYY NYTYNRNDRLLSGNSKSRFNTNMNGKKIELR TVIQNISDKNARQLKFKMWIKHSDGSVATDSS GNPLQTVQTFDIPVWNDKANFWPLGALDHIK SDFNSGLKSCSLIYQIPSDAQLKSGDTVAFQVL DENGNVLADDNTETQRYTTVSIQY |
| 47 | AK183(330-790) | EQSIQLTLGPWYSNDGKYSNPTIPVYTIQKTRS DTENMVVVVCGEGYTKSQQGKFINDVKRLW QDAMKYEPYRSYADRFNVYALCTASESTFDN GGSTFFDVIVDKYNSPVISNNLHGSQWKNHIF ERCIGPEFIEKIHDAHIKKKCDPNTIPSGSEYEP YYYVHDYIAQFAMVVNTKSDFGGAYNNREY GFHYFISPSDSYRASKTFAHEFGHGLLGLGDE YSNGYLLDDKELKSLNLSSVEDPEKIKWRQLL GFRNTYTCRNAYGSKMLVSSYECIMRDTNYQ FCEVCRLQGFKRMSQLVKDVDLYVATPEVKE YTGAYSKPSDFTDLETSSYYNYTYNRNDRLLS GNSKSRFNTNMNGKKIELRTVIQNISDKNARQ LKFKMWIKHSDGSVATDSSGNPLQTVQTFDIP VWNDKANFWPLGALDHIKSDFNSGLKSCSLI YQIPSDAQLKSGDTVAFQ |
| 48 | AK183(330-791) | EQSIQLTLGPWYSNDGKYSNPTIPVYTIQKTRS DTENMVVVVCGEGYTKSQQGKFINDVKRLW QDAMKYEPYRSYADRFNVYALCTASESTFDN |
| | | GGSTFFDVIVDKYNSPVISNNLHGSQWKNHIF ERCIGPEFIEKIHDAHIKKKCDPNTIPSGSEYEP YYYVHDYIAQFAMVVNTKSDFGGAYNNREY GFHYFISPSDSYRASKTFAHEFGHGLLGLGDE YSNGYLLDDKELKSLNLSSVEDPEKIKWRQLL GFRNTYTCRNAYGSKMLVSSYECIMRDTNYQ FCEVCRLQGFKRMSQLVKDVDLYVATPEVKE YTGAYSKPSDFTDLETSSYYNYTYNRNDRLLS GNSKSRFNTNMNGKKIELRTVIQNISDKNARQ LKFKMWIKHSDGSVATDSSGNPLQTVQTFDIP VWNDKANFWPLGALDHIKSDFNSGLKSCSLI YQIPSDAQLKSGDTVAFQV |
| 49 | AK183(330-792) | EQSIQLTLGPWYSNDGKYSNPTIPVYTIQKTRS DTENMVVVVCGEGYTKSQQGKFINDVKRLW QDAMKYEPYRSYADRFNVYALCTASESTFDN GGSTFFDVIVDKYNSPVISNNLHGSQWKNHIF ERCIGPEFIEKIHDAHIKKKCDPNTIPSGSEYEP YYYVHDYIAQFAMVVNTKSDFGGAYNNREY GFHYFISPSDSYRASKTFAHEFGHGLLGLGDE YSNGYLLDDKELKSLNLSSVEDPEKIKWRQLL GFRNTYTCRNAYGSKMLVSSYECIMRDTNYQ FCEVCRLQGFKRMSQLVKDVDLYVATPEVKE YTGAYSKPSDFTDLETSSYYNYTYNRNDRLLS GNSKSRFNTNMNGKKIELRTVIQNISDKNARQ LKFKMWIKHSDGSVATDSSGNPLQTVQTFDIP VWNDKANFWPLGALDHIKSDFNSGLKSCSLI YQIPSDAQLKSGDTVAFQVL |
| 50 | AK183(335-790) | LTLGPWYSNDGKYSNPTIPVYTIQKTRSDTEN MVVVVCGEGYTKSQQGKFINDVKRLWQDA MKYEPYRSYADRFNVYALCTASESTFDNGGS TFFDVIVDKYNSPVISNNLHGSQWKNHIFERCI GPEFIEKIHDAHIKKKCDPNTIPSGSEYEPYYY |
| | | VHDYIAQFAMVVNTKSDFGGAYNNREYGFH YFISPSDSYRASKTFAHEFGHGLLGLGDEYSN GYLLDDKELKSLNLSSVEDPEKIKWRQLLGFR NTYTCRNAYGSKMLVSSYECIMRDTNYQFCE VCRLQGFKRMSQLVKDVDLYVATPEVKEYT GAYSKPSDFTDLETSSYYNYTYNRNDRLLSG NSKSRFNTNMNGKKIELRTVIQNISDKNARQL KFKMWIKHSDGSVATDSSGNPLQTVQTFDIPV WNDKANFWPLGALDHIKSDFNSGLKSCSLIY QIPSDAQLKSGDTVAFQ |
| 51 | AK183(335-791) | LTLGPWYSNDGKYSNPTIPVYTIQKTRSDTEN MVVVVCGEGYTKSQQGKFINDVKRLWQDA MKYEPYRSYADRFNVYALCTASESTFDNGGS TFFDVIVDKYNSPVISNNLHGSQWKNHIFERCI GPEFIEKIHDAHIKKKCDPNTIPSGSEYEPYYY VHDYIAQFAMVVNTKSDFGGAYNNREYGFH YFISPSDSYRASKTFAHEFGHGLLGLGDEYSN GYLLDDKELKSLNLSSVEDPEKIKWRQLLGFR NTYTCRNAYGSKMLVSSYECIMRDTNYQFCE VCRLQGFKRMSQLVKDVDLYVATPEVKEYT GAYSKPSDFTDLETSSYYNYTYNRNDRLLSG NSKSRFNTNMNGKKIELRTVIQNISDKNARQL KFKMWIKHSDGSVATDSSGNPLQTVQTFDIPV WNDKANFWPLGALDHIKSDFNSGLKSCSLIY QIPSDAQLKSGDTVAFQV |
| 52 | AK183(335-792) | LTLGPWYSNDGKYSNPTIPVYTIQKTRSDTEN MVVVVCGEGYTKSQQGKFINDVKRLWQDA MKYEPYRSYADRFNVYALCTASESTFDNGGS TFFDVIVDKYNSPVISNNLHGSQWKNHIFERCI GPEFIEKIHDAHIKKKCDPNTIPSGSEYEPYYY VHDYIAQFAMVVNTKSDFGGAYNNREYGFH YFISPSDSYRASKTFAHEFGHGLLGLGDEYSN |
| | | GYLLDDKELKSLNLSSVEDPEKIKWRQLLGFR NTYTCRNAYGSKMLVSSYECIMRDTNYQFCE VCRLQGFKRMSQLVKDVDLYVATPEVKEYT GAYSKPSDFTDLETSSYYNYTYNRNDRLLSG NSKSRFNTNMNGKKIELRTVIQNISDKNARQL KFKMWIKHSDGSVATDSSGNPLQTVQTFDIPV WNDKANFWPLGALDHIKSDFNSGLKSCSLIY QIPSDAQLKSGDTVAFQVL |

In some embodiments, the truncated form of the IgA protease provided herein has an amino acid conservative substitution at one or more sites (*e.g*., at 1, 2, 3, 4, 5 or more sites) compared to the amino acid sequence of the polypeptide fragment mentioned above. An amino acid conservative substitution refers to a substitution between amino acids with similar properties, for example, between polar amino acids (*e.g*., between glutamine and asparagine), between hydrophobic amino acids (*e.g*., between leucine, isoleucine, methionine and valine) and between amino acids with the same charge (*e.g.*, between arginine, lysine and histidine, or substitutions between glutamic acid and aspartic acid), etc. In some embodiments, the truncated form of IgA protease described herein has an amino acid conservative substitution at 1, 2, 3, 4, 5, 6, 7, 8, 9, 10, 11 15, 20 or more sites compared to the amino acid sequence as set forth in SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51 or SEQ ID NO: 52.

In some embodiments, an amino acid mutation occurs at one or more sites of the polypeptide fragment, wherein the one or more sites correspond to position 844, position 862, position 931, position 933, position 978, position 1002, position 1004 of SEQ ID NO: 1. In some embodiments, the polypeptide fragment has an amino acid mutation at position 844 corresponding to SEQ ID NO: 1. In some embodiments, the polypeptide fragment has an amino acid mutation at position 862 corresponding to SEQ ID NO: 1. In some embodiments, the polypeptide fragment has amino acid mutations at position 931 and position 933 corresponding to SEQ ID NO: 1. In some embodiments, the polypeptide fragment has an amino acid mutation at position 978 corresponding to SEQ ID NO: 1. In some embodiments, the polypeptide fragment has amino acid mutations at position 1002 and position 1004 corresponding to SEQ ID NO: 1.

In some embodiments, one or more sites of the polypeptide fragment are mutated to glycine, wherein the one or more sites correspond to position 844, position 862, position 931, position 933, position 978, position 1002, position 1004 of SEQ ID NO: 1. In some embodiments, the proline (P) at one or more sites of the polypeptide fragment is mutated to glycine (G), wherein the one or more sites correspond to position 844, position 862, position 931, position 933, position 978, position 1002, position 1004 of SEQ ID NO: 1. In some embodiments, the proline of the polypeptide fragment is mutated to glycine at position 844 corresponding to SEQ ID NO: 1. In some embodiments, the proline of the polypeptide fragment is mutated to glycine at position 862 corresponding to SEQ ID NO: 1. In some embodiments, the proline of the polypeptide fragment is mutated to glycine at position 931 and position 933 corresponding to SEQ ID NO: 1. In some embodiments, the proline of the polypeptide fragment is mutated to glycine at position 978 corresponding to SEQ ID NO: 1. In some embodiments, the proline of the polypeptide fragment is mutated to glycine at position 1002 and position 1004 corresponding to SEQ ID NO: 1.

In some embodiments, the amino acid sequence of the polypeptide fragment is as set forth in SEQ ID NO: 53 (also referred to as "PA-GA Mut" ), SEQ ID NO: 54 (also referred to as "PI-GI Mut" ), SEQ ID NO: 55 (also referred to as "PAP-GAG Mut" ), SEQ ID NO: 56 (also referred to as "PAT-GAT Mut" ) or SEQ ID NO: 57 (also referred to as "PIP-GIG Mut" ).

The sequences of SEQ ID NOs: 53 ~ 57 are shown below.

| **SEQ ID NO** | **Sequence Description** | **Amino Acid Sequence** |
|---|---|---|
| 53 | PA-GA Mut | ASKPDIKVGDYVKMGVYNNASILWRCVSIDN NGPLMLADKIVDTLAYDAKTNDNSNSKSHSR SYKRDDYGSNYWKDSNMRSWLNSTAAEGK VDWLCGNPPKDGYVSGVGAYNEKAGFLNAF SKSEIAAMKTVTQRSLVSHPEYNKGIVDGDA NSDLLYYTDISEAVANYDSSYFETTTEKVFLL DVKQANAVWKNLKGYYVAYNNDGMAWPY WLRTPVTDCNHDMRYISSSGQVGRYAPWYS DLGVRPAFYLDSEYFVTTSGSGSQSSPYIGSAP NKQEDDYTISEPAEDANPDWNVSTEQSIQLTL GPWYSNDGKYSNPTIPVYTIQKTRSDTENMV VVVCGEGYTKSQQGKFINDVKRLWQDAMKY EPYRSYADRFNVYALCTASESTFDNGGSTFFD VIVDKYNSPVISNNLHGSQWKNHIFERCIGPEF IEKIHDAHIKKKCDPNTIPSGSEYEPYYYVHDY IAQFAMVVNTKSDFGGAYNNREYGFHYFISPS DSYRASKTFAHEFGHGLLGLGDEYSNGYLLD DKELKSLNLSSVEDPEKIKWRQLLGFRNTYTC RNAYGSKMLVSSYECIMRDTNYQFCEVCRLQ GFKRMSQLVKDVDLYVATPEVKEYTGAYSK PSDFTDLETSSYYNYTYNRNDRLLSGNSKSRF NTNMNGKKIELRTVIQNISDKNARQLKFKMW IKHSDGSVATDSSGNPLQTVQTFDIPVWNDKA NFWPLGALDHIKSDFNSGLKSCSLIYQIPSDAQ LKSGDTVAFQVLDENGNVLADDNTETQRYTT VSIQYKFEDGSEIPNTAGGTFTVPYGTKLDLT GAKTLYDYEFIKVDGLNKPIVSDGTVVTYYY KNKNEEHTHNLTLVAAKAATCTTAGNSAYY TCDGCDKWFADATGSVEITDKTSVKIPAPGHT AGTEWKSDDTNHWHECTVAGCGVIIESTKSA HTAGEWIVDTPATATTAGTKHKECTVCHRVL |
| | | ETQPIPSTGTELKIIAGDNQIYNKASGSDVTITC NGDFAKFTGIKVDGSVVDSSNYTAVSGSTVL TLKASYLGTLTDGSHTITFVYTDGEANANLTV RTAGSGHIHDYGTEWKSNADNHWHECNCGD KKDEAAHSFKWVVDKEATATKKGSKHEECK ICGYKRSAVEIPATGTSTAPTDTTKPNDTTKPG NTNGSEKSPQTGDNS |
| 54 | PI-GI Mut | ASKPDIKVGDYVKMGVYNNASILWRCVSIDN NGPLMLADKIVDTLAYDAKTNDNSNSKSHSR SYKRDDYGSNYWKDSNMRSWLNSTAAEGK VDWLCGNPPKDGYVSGVGAYNEKAGFLNAF SKSEIAAMKTVTQRSLVSHPEYNKGIVDGDA NSDLLYYTDISEAVANYDSSYFETTTEKVFLL DVKQANAVWKNLKGYYVAYNNDGMAWPY WLRTPVTDCNHDMRYISSSGQVGRYAPWYS DLGVRPAFYLDSEYFVTTSGSGSQSSPYIGSAP NKQEDDYTISEPAEDANPDWNVSTEQSIQLTL GPWYSNDGKYSNPTIPVYTIQKTRSDTENMV VVVCGEGYTKSQQGKFINDVKRLWQDAMKY EPYRSYADRFNVYALCTASESTFDNGGSTFFD VIVDKYNSPVISNNLHGSQWKNHIFERCIGPEF IEKIHDAHIKKKCDPNTIPSGSEYEPYYYVHDY IAQFAMVVNTKSDFGGAYNNREYGFHYFISPS DSYRASKTFAHEFGHGLLGLGDEYSNGYLLD DKELKSLNLSSVEDPEKIKWRQLLGFRNTYTC RNAYGSKMLVSSYECIMRDTNYQFCEVCRLQ GFKRMSQLVKDVDLYVATPEVKEYTGAYSK PSDFTDLETSSYYNYTYNRNDRLLSGNSKSRF NTNMNGKKIELRTVIQNISDKNARQLKFKMW IKHSDGSVATDSSGNPLQTVQTFDIPVWNDKA NFWPLGALDHIKSDFNSGLKSCSLIYQIPSDAQ LKSGDTVAFQVLDENGNVLADDNTETQRYTT |
| | | VSIQYKFEDGSEIPNTAGGTFTVPYGTKLDLTP AKTLYDYEFIKVDGLNKGIVSDGTVVTYYYK NKNEEHTHNLTLVAAKAATCTTAGNSAYYT CDGCDKWFADATGSVEITDKTSVKIPAPGHT AGTEWKSDDTNHWHECTVAGCGVIIESTKSA HTAGEWIVDTPATATTAGTKHKECTVCHRVL ETQPIPSTGTELKIIAGDNQIYNKASGSDVTITC NGDFAKFTGIKVDGSVVDSSNYTAVSGSTVL TLKASYLGTLTDGSHTITFVYTDGEANANLTV RTAGSGHIHDYGTEWKSNADNHWHECNCGD KKDEAAHSFKWVVDKEATATKKGSKHEECK ICGYKRSAVEIPATGTSTAPTDTTKPNDTTKPG NTNGSEKSPQTGDNS |
| 55 | PAP-GAG Mut | ASKPDIKVGDYVKMGVYNNASILWRCVSIDN NGPLMLADKIVDTLAYDAKTNDNSNSKSHSR SYKRDDYGSNYWKDSNMRSWLNSTAAEGK VDWLCGNPPKDGYVSGVGAYNEKAGFLNAF SKSEIAAMKTVTQRSLVSHPEYNKGIVDGDA NSDLLYYTDISEAVANYDSSYFETTTEKVFLL DVKQANAVWKNLKGYYVAYNNDGMAWPY WLRTPVTDCNHDMRYISSSGQVGRYAPWYS DLGVRPAFYLDSEYFVTTSGSGSQSSPYIGSAP NKQEDDYTISEPAEDANPDWNVSTEQSIQLTL GPWYSNDGKYSNPTIPVYTIQKTRSDTENMV VVVCGEGYTKSQQGKFINDVKRLWQDAMKY EPYRSYADRFNVYALCTASESTFDNGGSTFFD VIVDKYNSPVISNNLHGSQWKNHIFERCIGPEF IEKIHDAHIKKKCDPNTIPSGSEYEPYYYVHDY IAQFAMVVNTKSDFGGAYNNREYGFHYFISPS DSYRASKTFAHEFGHGLLGLGDEYSNGYLLD DKELKSLNLSSVEDPEKIKWRQLLGFRNTYTC RNAYGSKMLVSSYECIMRDTNYQFCEVCRLQ |
| | | GFKRMSQLVKDVDLYVATPEVKEYTGAYSK PSDFTDLETSSYYNYTYNRNDRLLSGNSKSRF NTNMNGKKIELRTVIQNISDKNARQLKFKMW IKHSDGSVATDSSGNPLQTVQTFDIPVWNDKA NFWPLGALDHIKSDFNSGLKSCSLIYQIPSDAQ LKSGDTVAFQVLDENGNVLADDNTETQRYTT VSIQYKFEDGSEIPNTAGGTFTVPYGTKLDLTP AKTLYDYEFIKVDGLNKPIVSDGTVVTYYYK NKNEEHTHNLTLVAAKAATCTTAGNSAYYT CDGCDKWFADATGSVEITDKTSVKIGAGGHT AGTEWKSDDTNHWHECTVAGCGVIIESTKSA HTAGEWIVDTPATATTAGTKHKECTVCHRVL ETQPIPSTGTELKIIAGDNQIYNKASGSDVTITC NGDFAKFTGIKVDGSVVDSSNYTAVSGSTVL TLKASYLGTLTDGSHTITFVYTDGEANANLTV RTAGSGHIHDYGTEWKSNADNHWHECNCGD KKDEAAHSFKWVVDKEATATKKGSKHEECK ICGYKRSAVEIPATGTSTAPTDTTKPNDTTKPG NTNGSEKSPQTGDNS |
| 56 | PAT-GAT Mut | ASKPDIKVGDYVKMGVYNNASILWRCVSIDN NGPLMLADKIVDTLAYDAKTNDNSNSKSHSR SYKRDDYGSNYWKDSNMRSWLNSTAAEGK VDWLCGNPPKDGYVSGVGAYNEKAGFLNAF SKSEIAAMKTVTQRSLVSHPEYNKGIVDGDA NSDLLYYTDISEAVANYDSSYFETTTEKVFLL DVKQANAVWKNLKGYYVAYNNDGMAWPY WLRTPVTDCNHDMRYISSSGQVGRYAPWYS DLGVRPAFYLDSEYFVTTSGSGSQSSPYIGSAP NKQEDDYTISEPAEDANPDWNVSTEQSIQLTL GPWYSNDGKYSNPTIPVYTIQKTRSDTENMV VVVCGEGYTKSQQGKFINDVKRLWQDAMKY EPYRSYADRFNVYALCTASESTFDNGGSTFFD |
| | | VIVDKYNSPVISNNLHGSQWKNHIFERCIGPEF IEKIHDAHIKKKCDPNTIPSGSEYEPYYYVHDY IAQFAMVVNTKSDFGGAYNNREYGFHYFISPS DSYRASKTFAHEFGHGLLGLGDEYSNGYLLD DKELKSLNLSSVEDPEKIKWRQLLGFRNTYTC RNAYGSKMLVSSYECIMRDTNYQFCEVCRLQ GFKRMSQLVKDVDLYVATPEVKEYTGAYSK PSDFTDLETSSYYNYTYNRNDRLLSGNSKSRF NTNMNGKKIELRTVIQNISDKNARQLKFKMW IKHSDGSVATDSSGNPLQTVQTFDIPVWNDKA NFWPLGALDHIKSDFNSGLKSCSLIYQIPSDAQ LKSGDTVAFQVLDENGNVLADDNTETQRYTT VSIQYKFEDGSEIPNTAGGTFTVPYGTKLDLTP AKTLYDYEFIKVDGLNKPIVSDGTVVTYYYK NKNEEHTHNLTLVAAKAATCTTAGNSAYYT CDGCDKWFADATGSVEITDKTSVKIPAPGHT AGTEWKSDDTNHWHECTVAGCGVIIESTKSA HTAGEWIVDTGATATTAGTKHKECTVCHRV LETQPIPSTGTELKIIAGDNQIYNKASGSDVTIT CNGDFAKFTGIKVDGSVVDSSNYTAVSGSTV LTLKASYLGTLTDGSHTITFVYTDGEANANLT VRTAGSGHIHDYGTEWKSNADNHWHECNCG DKKDEAAHSFKWVVDKEATATKKGSKHEEC KICGYKRSAVEIPATGTSTAPTDTTKPNDTTKP GNTNGSEKSPQTGDNS |
| 57 | PIP-GIG Mut | ASKPDIKVGDYVKMGVYNNASILWRCVSIDN NGPLMLADKIVDTLAYDAKTNDNSNSKSHSR SYKRDDYGSNYWKDSNMRSWLNSTAAEGK VDWLCGNPPKDGYVSGVGAYNEKAGFLNAF SKSEIAAMKTVTQRSLVSHPEYNKGIVDGDA NSDLLYYTDISEAVANYDSSYFETTTEKVFLL DVKQANAVWKNLKGYYVAYNNDGMAWPY |
| | | WLRTPVTDCNHDMRYISSSGQVGRYAPWYS DLGVRPAFYLDSEYFVTTSGSGSQSSPYIGSAP NKQEDDYTISEPAEDANPDWNVSTEQSIQLTL GPWYSNDGKYSNPTIPVYTIQKTRSDTENMV VVVCGEGYTKSQQGKFINDVKRLWQDAMKY EPYRSYADRFNVYALCTASESTFDNGGSTFFD VIVDKYNSPVISNNLHGSQWKNHIFERCIGPEF IEKIHDAHIKKKCDPNTIPSGSEYEPYYYVHDY IAQFAMVVNTKSDFGGAYNNREYGFHYFISPS DSYRASKTFAHEFGHGLLGLGDEYSNGYLLD DKELKSLNLSSVEDPEKIKWRQLLGFRNTYTC RNAYGSKMLVSSYECIMRDTNYQFCEVCRLQ GFKRMSQLVKDVDLYVATPEVKEYTGAYSK PSDFTDLETSSYYNYTYNRNDRLLSGNSKSRF NTNMNGKKIELRTVIQNISDKNARQLKFKMW IKHSDGSVATDSSGNPLQTVQTFDIPVWNDKA NFWPLGALDHIKSDFNSGLKSCSLIYQIPSDAQ LKSGDTVAFQVLDENGNVLADDNTETQRYTT VSIQYKFEDGSEIPNTAGGTFTVPYGTKLDLTP AKTLYDYEFIKVDGLNKPIVSDGTVVTYYYK NKNEEHTHNLTLVAAKAATCTTAGNSAYYT CDGCDKWFADATGSVEITDKTSVKIPAPGHT AGTEWKSDDTNHWHECTVAGCGVIIESTKSA HTAGEWIVDTPATATTAGTKHKECTVCHRVL ETQGIGSTGTELKIIAGDNQIYNKASGSDVTIT CNGDFAKFTGIKVDGSVVDSSNYTAVSGSTV LTLKASYLGTLTDGSHTITFVYTDGEANANLT VRTAGSGHIHDYGTEWKSNADNHWHECNCG DKKDEAAHSFKWVVDKEATATKKGSKHEEC KICGYKRSAVEIPATGTSTAPTDTTKPNDTTKP GNTNGSEKSPQTGDNS |

Provided that activity is not compromised, the truncated form of IgA protease provided herein may also comprise non-natural amino acids. Non-natural amino acids comprise, for example, β-fluorosubstituted alanine, 1-methylhistidine, γ-methylene glutamic acid, α-methylleucine, 4,5-dehydrolysine, hydroxyproline, 3-fluorosubstituted phenylalanine, 3-amino-tyrosine, 4-methyltryptophan and the like.

The truncated form of IgA protease provided herein can also be modified using methods well known in the art. Examples include, but are not limited to, PEGylation, glycosylation, amino-terminal modification, fatty acylation, carboxy-terminal modification, phosphorylation, methylation and the like. A person skilled in the art shall understand that after modification using methods well known in the art, the truncated form of IgA protease provided herein still retains substantially similar functions to IgA protease or the truncated form of IgA protease.

In some embodiments, the truncated form of IgA protease provided herein has an enzymatic activity of specifically cleaving human IgA. In some embodiments, the truncated form of IgA protease provided herein has an enzymatic activity of specifically cleaving human IgA heavy chain. In some embodiments, the truncated form of IgA protease provided herein has an enzymatic activity of specifically cleaving the intersection of human IgA heavy chain CH1 and hinge region. In some embodiments, the truncated form of IgA protease provided herein has an enzymatic activity of specifically cleaving human IgA1.

In some embodiments, the truncated form of IgA protease provided herein has an amino acid conservative substitution at one or more sites compared to the amino acid sequence of the polypeptide fragment mentioned above, but still has the enzymatic activity of cleaving human IgA (*e.g.*, IgA1). In some embodiments, the truncated form of IgA protease provided herein has at least 70% sequence identity (*e.g*., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to the polypeptide fragment mentioned above, and still has the enzymatic activity of cleaving human IgA (*e.g*., IgA1).

### Fusion Protein

In another aspect, the present disclosure provides a fusion protein comprising a first polypeptide and a second polypeptide, wherein the first polypeptide comprises a full-length wild-type IgA protease obtained from or derived from *Clostridium ramosum,* a polypeptide formed by removing a signal peptide of a wild-type IgA protease obtained from or derived from *Clostridium ramosum,* or the truncated form of IgA protease provided herein; the second polypeptide comprises an amino acid sequence for extending half-life of the first polypeptide in a subject. In some embodiments, the first polypeptide comprises a sequence as set forth in SEQ ID NO: 1 or SEQ ID NO: 42. In some embodiments, the second polypeptide is located at N-terminus of the first polypeptide. In some embodiments, the second polypeptide is located at C-terminus of the first polypeptide.

In some embodiments, the first polypeptide and the second polypeptide are linked via a linker. In some embodiments, the first polypeptide and the second polypeptide are directly linked to each other (*i.e*., linked without a linker). As used herein, the term "linker" refers to an artificial amino acid sequence having 1, 2, 3, 4 or 5 amino acid residues, or between 5 and 15, 20, 30, 50 or more amino acid residues in length, linked by a peptide bond and used to link one or more polypeptides. The linker may or may not have a secondary structure. Linker sequences are known in the art, for example, see Holliger et al., Proc. Natl. Acad. Sci. USA 90:6444-6448 (1993); Poljak et al., Structure 2:1121-1123 (1994).

In some embodiments, the linker is selected from the group consisting of a cleavable linker, a non-cleavable linker, a peptide linker, a flexible linker, a rigid linker, a helical linker and a non-helical linker. Any suitable linker known in the art can be used. In some embodiments, the linker comprises a peptide linker. For example, useful linkers in the present disclosure may be rich in glycine and serine residues. Examples include linkers having single or repeated sequences comprising threonine/serine and glycine, such as GGGS (SEQ ID NO: 21) or GGGGS (SEQ ID NO: 22), GGGGGS (SEQ ID NO: 86) or GGGGGGGS (SEQ ID NO: 87) or tandem repeats thereof (*e.g*., 2, 3, 4, 5, 6, 7 8, 9, 10 or more repeats). In some embodiments, the linker used in the present disclosure comprises GGCGGCGGTGGATCC (SEQ ID NO: 23). Optionally, the linker may be a long peptide chain comprising one or more sequential or tandem repeats of an amino acid sequence as set forth in GGCGGCGGTGGATCC (SEQ ID NO: 23). In some embodiments, the linker comprises 1, 2, 3, 4, 5, 6, 7, 8, 9, 10 or more sequential or tandem repeats of SEQ ID NO: 23. In some embodiments, the linker comprises or consists of an amino acid sequence selected from the following group: an amino acid sequence having at least 80%, at least 85%, at least 90%, at least 91%, at least 92%, at least 93%, at least 94%, at least 95%, at least 96%, at least 97%, at least 98%, at least 99% sequence identity to any one of SEQ ID NO: 21, 22, 23.

In some embodiments, the linker used in the present disclosure comprises an amino acid sequence as set forth in SEQ ID NO: 58 (EEKKKEKEKEEQEERETK). Optionally, the linker may be a long peptide chain comprising one or more sequential or tandem repeats of an amino acid sequence as set forth in SEQ ID NO: 58. In some embodiments, the linker comprises 1, 2, 3, 4, 5, 6, 7, 8, 9, 10 or more sequential or tandem repeats of SEQ ID NO: 58. In some embodiments, the linker comprises a sequence having at least 80%, at least 85%, at least 90%, at least 91%, at least 92%, at least 93%, at least 94%, at least 95%, at least 96%, at least 97%, at least 98%, at least 99% sequence identity to SEQ ID NO: 58.

In some embodiments, the linker used in the present disclosure comprises an amino acid sequence as set forth in SEQ ID NO: 59 (HHHHHHHHHH). In some embodiments, the linker comprises a sequence having at least 80%, at least 85%, at least 90%, at least 91%, at least 92%, at least 93%, at least 94%, at least 95%, at least 96%, at least 97%, at least 98%, at least 99% sequence identity to SEQ ID NO: 59.

In some embodiments, the second polypeptide is selected from an Fc domain and albumin. In some embodiments, the Fc domain comprises a hinge region. In some embodiments, the Fc domain comprises a lower hinge region. In some embodiments, the Fc domain comprises a core hinge region and a lower hinge region. In some embodiments, the Fc domain comprises an upper hinge region, a core hinge region and a lower hinge region. In some embodiments, the Fc domain does not comprise a hinge region. In some embodiments, the Fc domain is derived from human IgG Fc domain. In some embodiments, the Fc domain is derived from human IgG1 Fc domain, human IgG2 Fc domain, human IgG3 Fc domain or human IgG4 Fc domain.

In some embodiments, the Fc domain comprises an amino acid sequence as set forth in SEQ ID NO: 24. In some embodiments, the Fc domain consists of an amino acid sequence as set forth in SEQ ID NO: 24. In some embodiments, the amino acid sequence of the Fc domain has at least 70%, at least 75%, at least 80%, at least 85%, at least 90% or at least 95% sequence identity to an amino acid sequence as set forth in SEQ ID NO: 24.

EPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFS
CSVMHEALHNHYTQKSLSLSPGK (SEQ ID NO: 24)

In some embodiments, the nucleic acid sequence encoding the Fc domain comprises a nucleotide sequence as set forth in SEQ ID NO: 39. In some embodiments, the nucleic acid sequence encoding the Fc domain consists of a nucleotide sequence as set forth in SEQ ID NO: 39. In some embodiments, the nucleic acid sequence encoding the Fc domain has at least 70%, at least 75%, at least 80%, at least 85%, at least 90% or at least 95% sequence identity to a nucleotide sequence as set forth in SEQ ID NO: 39.

GAGCCCAAATCTTGTGACAAAACTCACACATGCCCACCGTGCCCAGCACC
TGAACTCCTGGGGGGACCGTCAGTCTTCCTCTTCCCCCCAAAACCCAAGG
ACACCCTCATGATCTCCCGGACCCCTGAGGTCACATGCGTGGTGGTGGAC
GTGAGCCACGAAGACCCTGAGGTCAAGTTCAACTGGTACGTGGACGGCGT

GGAGGTGCATAATGCCAAGACAAAGCCGCGGGAGGAGCAGTACAACAGC
ACGTACCGGGTGGTCAGCGTCCTCACCGTCCTGCACCAGGACTGGCTGAA
TGGCAAGGAGTACAAGTGCAAGGTCTCCAACAAAGCCCTCCCAGCCCCCA
TCGAGAAAACCATCTCCAAAGCCAAAGGGCAGCCCCGAGAACCACAGGT
GTACACCCTGCCCCCATCCCGGGATGAGCTGACCAAGAACCAGGTCAGCC
TGACCTGCCTGGTCAAAGGCTTCTATCCCAGCGACATCGCCGTGGAGTGG
GAGAGCAATGGGCAGCCGGAGAACAACTACAAGACCACGCCTCCCGTGC
TGGACTCCGACGGCTCCTTCTTCCTCTACAGCAAGCTCACCGTGGACAAG
AGCAGGTGGCAGCAGGGGAACGTCTTCTCATGCTCCGTGATGCATGAGGC
TCTGCACAACCACTACACGCAGAAGAGCCTCTCCCTGTCTCCGGGTAAA
(SEQ ID NO: 39)

In some embodiments, the Fc domain comprises an amino acid sequence as set forth in SEQ ID NO: 25. In some embodiments, the Fc domain consists of an amino acid sequence as set forth in SEQ ID NO: 25. In some embodiments, the amino acid sequence of the Fc domain has at least 70%, at least 75%, at least 80%, at least 85%, at least 90% or at least 95% sequence identity to an amino acid sequence as set forth in SEQ ID NO: 25.

TCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEW
ESNGQPENNYKTTPPVLDSDGSFFLYSKLTVDKSRWQQGNVFSCSVMHEALH
NHYTQKSLSLSPGK    (SEQ ID NO: 25)

In some embodiments, the nucleic acid sequence encoding the Fc domain comprises a nucleotide sequence as set forth in SEQ ID NO: 40. In some embodiments, the nucleic acid sequence encoding the Fc domain consists of a nucleotide sequence as set forth in SEQ ID NO: 40. In some embodiments, the nucleic acid sequence encoding the Fc domain has at least 70%, at least 75%, at least 80%, at least 85%, at least 90% or at least 95% sequence identity to a nucleotide sequence as set forth in SEQ ID NO: 40.

ACATGCCCACCGTGCCCAGCACCTGAACTCCTGGGGGGACCGTCAGTCTT
CCTCTTCCCCCCAAAACCCAAGGACACCCTCATGATCTCCCGGACCCCTGA
GGTCACATGCGTGGTGGTGGACGTGAGCCACGAAGACCCTGAGGTCAAGT
TCAACTGGTACGTGGACGGCGTGGAGGTGCATAATGCCAAGACAAAGCC
GCGGGAGGAGCAGTACAACAGCACGTACCGGGTGGTCAGCGTCCTCACC
GTCCTGCACCAGGACTGGCTGAATGGCAAGGAGTACAAGTGCAAGGTCTC
CAACAAAGCCCTCCCAGCCCCCATCGAGAAAACCATCTCCAAAGCCAAAG
GGCAGCCCCGAGAACCACAGGTGTACACCCTGCCCCCATCCCGGGATGAG
CTGACCAAGAACCAGGTCAGCCTGACCTGCCTGGTCAAAGGCTTCTATCC
CAGCGACATCGCCGTGGAGTGGGAGAGCAATGGGCAGCCGGAGAACAAC
TACAAGACCACGCCTCCCGTGCTGGACTCCGACGGCTCCTTCTTCCTCTAC
AGCAAGCTCACCGTGGACAAGAGCAGGTGGCAGCAGGGGAACGTCTTCT
CATGCTCCGTGATGCATGAGGCTCTGCACAACCACTACACGCAGAAGAGC
CTCTCCCTGTCTCCGGGTAAA    (SEQ ID NO: 40)

In some embodiments, the Fc domain comprises an amino acid sequence as set forth in SEQ ID NO: 32. In some embodiments, the Fc domain consists of an amino acid sequence as set forth in SEQ ID NO: 32. In some embodiments, the amino acid sequence of the Fc domain has at least 70%, at least 75%, at least 80%, at least 85%, at least 90% or at least 95% sequence identity to an amino acid sequence as set forth in SEQ ID NO: 32.

ELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQK
SLSLSPGK (SEQ ID NO: 32)

In some embodiments, the Fc domain comprises an amino acid sequence as set forth in SEQ ID NO: 77. In some embodiments, the Fc domain consists of an amino acid sequence as set forth in SEQ ID NO: 77. In some embodiments, the amino acid sequence of the Fc domain has at least 70%, at least 75%, at least 80%, at least 85%, at least 90% or at least 95% sequence identity to an amino acid sequence as set forth in SEQ ID NO: 77.

ESKYGPPCPSCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPS
DIAVEWESNGQPENNYKTTPPVLDSDGSFFLYSRLTVDKSRWQEGNVFSCSV
MHEALHNHYTQKSLSLSLGK    (SEQ ID NO: 77)

In some embodiments, the Fc domain has one or more amino acid mutation. In some embodiments, the Fc domain has an amino acid mutation at a site corresponding to position 7 of SEQ ID NO: 25. In some embodiments, amino acid (e.g., alanine) at a site of the Fc domain is mutated to valine, wherein the site corresponds to position 7 of SEQ ID NO: 25. In some embodiments, amino acid (e.g., alanine) at a site of the Fc domain is mutated to glycine, wherein the site corresponds to position 7 of SEQ ID NO: 25. In some embodiments, amino acid (e.g., alanine) at a site of the Fc domain is mutated to serine, wherein the site corresponds to position 7 of SEQ ID NO: 25. In some embodiments, amino acid (*e.g*., alanine) at a site of the Fc domain is mutated to leucine, wherein the site corresponds to position 7 of SEQ ID NO: 25.

In some embodiments, the Fc domain comprises one or more mutations that extend half-life of the fusion protein. In some embodiments, the Fc domain is linked to C-terminus of the first polypeptide. In some embodiments, the Fc domain is linked to N-terminus of the first polypeptide.

In some embodiments, the second polypeptide is albumin. In some embodiments, the amino acid sequence of albumin is as set forth in SEQ ID NO: 60. In some embodiments, the albumin comprises one or more domains of human serum albumin. In some embodiments, the albumin comprises a D3 domain of human serum albumin.












    (SEQ ID NO: 60)

In some embodiments, the fusion protein provided herein further comprises a label. In some embodiments, the label is selected from the group consisting of a fluorescent label, a luminescent label, a purification label and a chromogenic label. In some embodiments, the label is selected from the group consisting of a c-Myc tag, an HA tag, a VSV-G tag, a FLAG tag, a V5 tag and a HIS tag. In some embodiments, the label is a HIS tag. In some embodiments, the label is a HIS tag comprising 6, 7, 8, 9 or 10 histidine. In some embodiments, the second polypeptide is located at C-terminus of the first polypeptide and the label is located at C-terminus of the second polypeptide.

In some embodiments, the fusion protein provided herein comprises an amino acid sequence as set forth in SEQ ID NO: 26, SEQ ID NO: 27, SEQ ID NO: 28, SEQ ID NO: 29, SEQ ID NO: 30, SEQ ID NO: 31, SEQ ID NO: 81, SEQ ID NO: 82, SEQ ID NO: 83, SEQ ID NO: 84 or SEQ ID NO: 85. In some embodiments, the fusion protein provided herein comprises an amino acid sequence selected from the group consisting of SEQ ID NO: 26, SEQ ID NO: 27, SEQ ID NO: 28, SEQ ID NO: 29, SEQ ID NO: 30, SEQ ID NO: 31, SEQ ID NO: 81, SEQ ID NO: 82, SEQ ID NO: 83, SEQ ID NO: 84, or SEQ ID NO: 85, or an amino acid sequence having at least 70%, at least 75%, at least 80%, at least 85%, at least 90%, at least 95% sequence identity thereto. In some embodiments, the fusion protein having at least 70%, at least 75%, at least 80%, at least 85%, at least 90%, at least 95% sequence identity to SEQ ID NO: 26, SEQ ID NO: 27, SEQ ID NO: 28, SEQ ID NO: 29, SEQ ID NO: 30, SEQ ID NO: 31, SEQ ID NO: 81, SEQ ID NO: 82, SEQ ID NO: 83, SEQ ID NO: 84, or SEQ ID NO: 85 still retains the function or activity of the IgA protease (*e.g*., protein hydrolytic activity, enzymatic activity of specifically cleaving IgA, etc.).

| **SEQ ID NO** | **Amino Acid Sequence** |
|---|---|
| 26 | ASKPDIKVGDYVKMGVYNNASILWRCVSIDNNGPLMLADKIV DTLAYDAKTNDNSNSKSHSRSYKRDDYGSNYWKDSNMRSWL NSTAAEGKVDWLCGNPPKDGYVSGVGAYNEKAGFLNAFSKSE IAAMKTVTQRSLVSHPEYNKGIVDGDANSDLLYYTDISEAVAN YDSSYFETTTEKVFLLDVKQANAVWKNLKGYYVAYNNDGMA WPYWLRTPVTDCNHDMRYISSSGQVGRYAPWYSDLGVRPAFY LDSEYFVTTSGSGSQSSPYIGSAPNKQEDDYTISEPAEDANPDW NVSTEQSIQLTLGPWYSNDGKYSNPTIPVYTIQKTRSDTENMVV VVCGEGYTKSQQGKFINDVKRLWQDAMKYEPYRSYADRFNV YALCTASESTFDNGGSTFFDVIVDKYNSPVISNNLHGSQWKNHI FERCIGPEFIEKIHDAHIKKKCDPNTIPSGSEYEPYYYVHDYIAQF AMVVNTKSDFGGAYNNREYGFHYFISPSDSYRASKTFAHEFGH GLLGLGDEYSNGYLLDDKELKSLNLSSVEDPEKIKWRQLLGFR NTYTCRNAYGSKMLVSSYECIMRDTNYQFCEVCRLQGFKRMS QLVKDVDLYVATPEVKEYTGAYSKPSDFTDLETSSYYNYTYNR NDRLLSGNSKSRFNTNMNGKKIELRTVIQNISDKNARQLKFKM WIKHSDGSVATDSSGNPLQTVQTFDIPVWNDKANFWPLGALD HIKSDFNSGLKSCSLIYQIPSDAQLKSGDTVAFQVLGGGGSEPKS CDKTHTCPPCPAPELLGGPSVFLFPPKPKDTLMISRTPEVTCVVV DVSHEDPEVKFNWYVDGVEVHNAKTKPREEQYNSTYRVVSVL TVLHQDWLNGKEYKCKVSNKALPAPIEKTISKAKGQPREPQVY TLPPSRDELTKNQVSLTCLVKGFYPSDIAVEWESNGQPENNYK |
| | TTPPVLDSDGSFFLYSKLTVDKSRWQQGNVFSCSVMHEALHNH YTQKSLSLSPGK |
| 27 | MYRMQLLSCIALSLALVTNSGTASKPDIKVGDYVKMGVYNNA SILWRCVSIDNNGPLMLADKIVDTLAYDAKTNDNSNSKSHSRS YKRDDYGSNYWKDSNMRSWLNSTAAEGKVDWLCGNPPKDG YVSGVGAYNEKAGFLNAFSKSEIAAMKTVTQRSLVSHPEYNK GIVDGDANSDLLYYTDISEAVANYDSSYFETTTEKVFLLDVKQ ANAVWKNLKGYYVAYNNDGMAWPYWLRTPVTDCNHDMRYI SSSGQVGRYAPWYSDLGVRPAFYLDSEYFVTTSGSGSQSSPYIG SAPNKQEDDYTISEPAEDANPDWNVSTEQSIQLTLGPWYSNDG KYSNPTIPVYTIQKTRSDTENMVVVVCGEGYTKSQQGKFINDV KRLWQDAMKYEPYRSYADRFNVYALCTASESTFDNGGSTFFD VIVDKYNSPVISNNLHGSQWKNHIFERCIGPEFIEKIHDAHIKKK CDPNTIPSGSEYEPYYYVHDYIAQFAMVVNTKSDFGGAYNNRE YGFHYFISPSDSYRASKTFAHEFGHGLLGLGDEYSNGYLLDDK ELKSLNLSSVEDPEKIKRQLLGFRNTYTCRNAYGSKMLVSSYEC IMRDTNYQFCEVCRLQGFKRMSQLVKDVDLYVATPEVKEYTG AYSKPSDFTDLETSSYYNYTYNRNDRLLSGNSKSRFNTNMNGK KIELRTVIQNISDKNARQLKFKMWIKHSDGSVATDSSGNPLQTV QTFDIPVWNDKANFWPLGALDHIKSDFNSGLKSCSLIYQIPSDA QLKSGDTVAFQVLGGGGSEPKSCDKTHTCPPCPAPELLGGPSVF LFPPKPKDTLMISRTPEVTCVVVDVSHEDPEVKFNWYVDGVEV HNAKTKPREEQYNSTYRVVSVLTVLHQDWLNGKEYKCKVSN KALPAPIEKTISKAKGQPREPQVYTLPPSRDELTKNQVSLTCLV KGFYPSDIAVEWESNGQPENNYKTTPPVLDSDGSFFLYSKLTVD KSRWQQGNVFSCSVMHEALHNHYTQKSLSLSPGK |
| 28 | ASKPDIKVGDYVKMGVYNNASILWRCVSIDNNGPLMLADKIV DTLAYDAKTNDNSNSKSHSRSYKRDDYGSNYWKDSNMRSWL NSTAAEGKVDWLCGNPPKDGYVSGVGAYNEKAGFLNAFSKSE IAAMKTVTQRSLVSHPEYNKGIVDGDANSDLLYYTDISEAVAN YDSSYFETTTEKVFLLDVKQANAVWKNLKGYYVAYNNDGMA WPYWLRTPVTDCNHDMRYISSSGQVGRYAPWYSDLGVRPAFY |
| | LDSEYFVTTSGSGSQSSPYIGSAPNKQEDDYTISEPAEDANPDW NVSTEQSIQLTLGPWYSNDGKYSNPTIPVYTIQKTRSDTENMVV VVCGEGYTKSQQGKFINDVKRLWQDAMKYEPYRSYADRFNV YALCTASESTFDNGGSTFFDVIVDKYNSPVISNNLHGSQWKNHI FERCIGPEFIEKIHDAHIKKKCDPNTIPSGSEYEPYYYVHDYIAQF AMVVNTKSDFGGAYNNREYGFHYFISPSDSYRASKTFAHEFGH GLLGLGDEYSNGYLLDDKELKSLNLSSVEDPEKIKWRQLLGFR NTYTCRNAYGSKMLVSSYECIMRDTNYQFCEVCRLQGFKRMS QLVKDVDLYVATPEVKEYTGAYSKPSDFTDLETSSYYNYTYNR NDRLLSGNSKSRFNTNMNGKKIELRTVIQNISDKNARQLKFKM WIKHSDGSVATDSSGNPLQTVQTFDIPVWNDKANFWPLGALD HIKSDFNSGLKSCSLIYQIPSDAQLKSGDTVAFQVLDENGNVGG GGSHHHHHHHHHHTCPPCPAPELLGGPSVFLFPPKPKDTLMISR TPEVTCVVVDVSHEDPEVKFNWYVDGVEVHNAKTKPREEQYN STYRVVSVLTVLHQDWLNGKEYKCKVSNKALPAPIEKTISKAK GQPREPQVYTLPPSRDELTKNQVSLTCLVKGFYPSDIAVEWESN GQPENNYKTTPPVLDSDGSFFLYSKLTVDKSRWQQGNVFSCSV MHEALHNHYTQKSLSLSPGK |
| 29 | ASKPDIKVGDYVKMGVYNNASILWRCVSIDNNGPLMLADKIV DTLAYDAKTNDNSNSKSHSRSYKRDDYGSNYWKDSNMRSWL NSTAAEGKVDWLCGNPPKDGYVSGVGAYNEKAGFLNAFSKSE IAAMKTVTQRSLVSHPEYNKGIVDGDANSDLLYYTDISEAVAN YDSSYFETTTEKVFLLDVKQANAVWKNLKGYYVAYNNDGMA WPYWLRTPVTDCNHDMRYISSSGQVGRYAPWYSDLGVRPAFY LDSEYFVTTSGSGSQSSPYIGSAPNKQEDDYTISEPAEDANPDW NVSTEQSIQLTLGPWYSNDGKYSNPTIPVYTIQKTRSDTENMVV VVCGEGYTKSQQGKFINDVKRLWQDAMKYEPYRSYADRFNV YALCTASESTFDNGGSTFFDVIVDKYNSPVISNNLHGSQWKNHI FERCIGPEFIEKIHDAHIKKKCDPNTIPSGSEYEPYYYVHDYIAQF AMVVNTKSDFGGAYNNREYGFHYFISPSDSYRASKTFAHEFGH GLLGLGDEYSNGYLLDDKELKSLNLSSVEDPEKIKWRQLLGFR NTYTCRNAYGSKMLVSSYECIMRDTNYQFCEVCRLQGFKRMS QLVKDVDLYVATPEVKEYTGAYSKPSDFTDLETSSYYNYTYNR |
| | NDRLLSGNSKSRFNTNMNGKKIELRTVIQNISDKNARQLKFKM WIKHSDGSVATDSSGNPLQTVQTFDIPVWNDKANFWPLGALD HIKSDFNSGLKSCSLIYQIPSDAQLKSGDTVAFQVLDENGNVLA DDNTETQGGGGSHHHHHHHHHHTCPPCPAPELLGGPSVFLFPP KPKDTLMISRTPEVTCVVVDVSHEDPEVKFNWYVDGVEVHNA KTKPREEQYNSTYRVVSVLTVLHQDWLNGKEYKCKVSNKALP APIEKTISKAKGQPREPQVYTLPPSRDELTKNQVSLTCLVKGFY PSDIAVEWESNGQPENNYKTTPPVLDSDGSFFLYSKLTVDKSR WQQGNVFSCSVMHEALHNHYTQKSLSLSPGK |
| 30 | ASKPDIKVGDYVKMGVYNNASILWRCVSIDNNGPLMLADKIV DTLAYDAKTNDNSNSKSHSRSYKRDDYGSNYWKDSNMRSWL NSTAAEGKVDWLCGNPPKDGYVSGVGAYNEKAGFLNAFSKSE IAAMKTVTQRSLVSHPEYNKGIVDGDANSDLLYYTDISEAVAN YDSSYFETTTEKVFLLDVKQANAVWKNLKGYYVAYNNDGMA WPYWLRTPVTDCNHDMRYISSSGQVGRYAPWYSDLGVRPAFY LDSEYFVTTSGSGSQSSPYIGSAPNKQEDDYTISEPAEDANPDW NVSTEQSIQLTLGPWYSNDGKYSNPTIPVYTIQKTRSDTENMVV VVCGEGYTKSQQGKFINDVKRLWQDAMKYEPYRSYADRFNV YALCTASESTFDNGGSTFFDVIVDKYNSPVISNNLHGSQWKNHI FERCIGPEFIEKIHDAHIKKKCDPNTIPSGSEYEPYYYVHDYIAQF AMVVNTKSDFGGAYNNREYGFHYFISPSDSYRASKTFAHEFGH GLLGLGDEYSNGYLLDDKELKSLNLSSVEDPEKIKWRQLLGFR NTYTCRNAYGSKMLVSSYECIMRDTNYQFCEVCRLQGFKRMS QLVKDVDLYVATPEVKEYTGAYSKPSDFTDLETSSYYNYTYNR NDRLLSGNSKSRFNTNMNGKKIELRTVIQNISDKNARQLKFKM WIKHSDGSVATDSSGNPLQTVQTFDIPVWNDKANFWPLGALD HIKSDFNSGLKSCSLIYQIPSDAQLKSGDTVAFQVLDENGNVLA DDNTETQRYTTVSIQYGGGGSHHHHHHHHHTCPPCPAPELLG GPSVFLFPPKPKDTLMISRTPEVTCVVVDVSHEDPEVKFNWYV DGVEVHNAKTKPREEQYNSTYRVVSVLTVLHQDWLNGKEYK CKVSNKALPAPIEKTISKAKGQPREPQVYTLPPSRDELTKNQVS LTCLVKGFYPSDIAVEWESNGQPENNYKTTPPVLDSDGSFFLYS |
| | KLTVDKSRWQQGNVFSCSVMHEALHNHYTQKSLSLSPGK |
| 31 | ASKPDIKVGDYVKMGVYNNASILWRCVSIDNNGPLMLADKIV DTLAYDAKTNDNSNSKSHSRSYKRDDYGSNYWKDSNMRSWL NSTAAEGKVDWLCGNPPKDGYVSGVGAYNEKAGFLNAFSKSE IAAMKTVTQRSLVSHPEYNKGIVDGDANSDLLYYTDISEAVAN YDSSYFETTTEKVFLLDVKQANAVWKNLKGYYVAYNNDGMA WPYWLRTPVTDCNHDMRYISSSGQVGRYAPWYSDLGVRPAFY LDSEYFVTTSGSGSQSSPYIGSAPNKQEDDYTISEPAEDANPDW NVSTEQSIQLTLGPWYSNDGKYSNPTIPVYTIQKTRSDTENMVV VVCGEGYTKSQQGKFINDVKRLWQDAMKYEPYRSYADRFNV YALCTASESTFDNGGSTFFDVIVDKYNSPVISNNLHGSQWKNHI FERCIGPEFIEKIHDAHIKKKCDPNTIPSGSEYEPYYYVHDYIAQF AMVVNTKSDFGGAYNNREYGFHYFISPSDSYRASKTFAHEFGH GLLGLGDEYSNGYLLDDKELKSLNLSSVEDPEKIKWRQLLGFR NTYTCRNAYGSKMLVSSYECIMRDTNYQFCEVCRLQGFKRMS QLVKDVDLYVATPEVKEYTGAYSKPSDFTDLETSSYYNYTYNR NDRLLSGNSKSRFNTNMNGKKIELRTVIQNISDKNARQLKFKM WIKHSDGSVATDSSGNPLQTVQTFDIPVWNDKANFWPLGALD HIKSDFNSGLKSCSLIYQIPSDAQLKSGDTVAFQVLDENGNVLA DDNTETQRYTTVSIQYKFEDGSEIPNTAGGTFTGGGGSHHHHH HHHHHTCPPCPAPELLGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREPQ VYTLPPSRDELTKNQVSLTCLVKGFYPSDIAVEWESNGQPENN YKTTPPVLDSDGSFFLYSKLTVDKSRWQQGNVFSCSVMHEALH NHYTQKSLSLSPGK |
| 81 | EYFVTTSGSGSQSSPYIGSAPNKQEDDYTISEPAEDANPDWNVS TEQSIQLTLGPWYSNDGKYSNPTIPVYTIQKTRSDTENMVVVVC GEGYTKSQQGKFINDVKRLWQDAMKYEPYRSYADRFNVYAL CTASESTFDNGGSTFFDVIVDKYNSPVISNNLHGSQWKNHIFER CIGPEFIEKIHDAHIKKKCDPNTIPSGSEYEPYYYVHDYIAQFAM VVNTKSDFGGAYNNREYGFHYFISPSDSYRASKTFAHEFGHGL |
| | LGLGDEYSNGYLLDDKELKSLNLSSVEDPEKIKWRQLLGFRNT YTCRNAYGSKMLVSSYECIMRDTNYQFCEVCRLQGFKRMSQL VKDVDLYVATPEVKEYTGAYSKPSDFTDLETSSYYNYTYNRN DRLLSGNSKSRFNTNMNGKKIELRTVIQNISDKNARQLKFKMW IKHSDGSVATDSSGNPLQTVQTFDIPVWNDKANFWPLGALDHI KSDFNSGLKSCSLIYQIPSDAQLKSGDTVAFQVLDENGNVLAD DNTETQRYTTVSIQYGGGGGSTCPPCPAPELLGGPSVFLFPPKP KDTLMISRTPEVTCVVVDVSHEDPEVKFNWYVDGVEVHNAKT KPREEQYNSTYRVVSVLTVLHQDWLNGKEYKCKVSNKALPAP IEKTISKAKGQPREPQVYTLPPSRDELTKNQVSLTCLVKGFYPS DIAVEWESNGQPENNYKTTPPVLDSDGSFFLYSKLTVDKSRWQ QGNVFSCSVMHEALHNHYTQKSLSLSPGK |
| 82 | TCPPCPAPELLGGPSVFLFPPKPKDTLMISRTPEVTCVVVDVSHE DPEVKFNWYVDGVEVHNAKTKPREEQYNSTYRVVSVLTVLHQ DWLNGKEYKCKVSNKALPAPIEKTISKAKGQPREPQVYTLPPS RDELTKNQVSLTCLVKGFYPSDIAVEWESNGQPENNYKTTPPV LDSDGSFFLYSKLTVDKSRWQQGNVFSCSVMHEALHNHYTQK SLSLSPGKGGGGGSEYFVTTSGSGSQSSPYIGSAPNKQEDDYTIS EPAEDANPDWNVSTEQSIQLTLGPWYSNDGKYSNPTIPVYTIQK TRSDTENMVVVVCGEGYTKSQQGKFINDVKRLWQDAMKYEP YRSYADRFNVYALCTASESTFDNGGSTFFDVIVDKYNSPVISNN LHGSQWKNHIFERCIGPEFIEKIHDAHIKKKCDPNTIPSGSEYEP YYYVHDYIAQFAMVVNTKSDFGGAYNNREYGFHYFISPSDSYR ASKTFAHEFGHGLLGLGDEYSNGYLLDDKELKSLNLSSVEDPE KIKWRQLLGFRNTYTCRNAYGSKMLVSSYECIMRDTNYQFCE VCRLQGFKRMSQLVKDVDLYVATPEVKEYTGAYSKPSDFTDL ETSSYYNYTYNRNDRLLSGNSKSRFNTNMNGKKIELRTVIQNIS DKNARQLKFKMWIKHSDGSVATDSSGNPLQTVQTFDIPVWND KANFWPLGALDHIKSDFNSGLKSCSLIYQIPSDAQLKSGDTVAF QVLDENGNVLADDNTETQRYTTVSIQY |
| 83 | EPKSCDKTHTCPPCPAPELLGGPSVFLFPPKPKDTLMISRTPEVT CVVVDVSHEDPEVKFNWYVDGVEVHNAKTKPREEQYNSTYR |
| | VVSVLTVLHQDWLNGKEYKCKVSNKALPAPIEKTISKAKGQPR EPQVYTLPPSRDELTKNQVSLTCLVKGFYPSDIAVEWESNGQPE NNYKTTPPVLDSDGSFFLYSKLTVDKSRWQQGNVFSCSVMHE ALHNHYTQKSLSLSPGKGGGGGGGSASKPDIKVGDYVKMGVY NNASILWRCVSIDNNGPLMLADKIVDTLAYDAKTNDNSNSKSH SRSYKRDDYGSNYWKDSNMRSWLNSTAAEGKVDWLCGNPPK DGYVSGVGAYNEKAGFLNAFSKSEIAAMKTVTQRSLVSHPEY NKGIVDGDANSDLLYYTDISEAVANYDSSYFETTTEKVFLLDV KQANAVWKNLKGYYVAYNNDGMAWPYWLRTPVTDCNHDM RYISSSGQVGRYAPWYSDLGVRPAFYLDSEYFVTTSGSGSQSSP YIGSAPNKQEDDYTISEPAEDANPDWNVSTEQSIQLTLGPWYSN DGKYSNPTIPVYTIQKTRSDTENMVVVVCGEGYTKSQQGKFIN DVKRLWQDAMKYEPYRSYADRFNVYALCTASESTFDNGGSTF FDVIVDKYNSPVISNNLHGSQWKNHIFERCIGPEFIEKIHDAHIK KKCDPNTIPSGSEYEPYYYVHDYIAQFAMVVNTKSDFGGAYNN REYGFHYFISPSDSYRASKTFAHEFGHGLLGLGDEYSNGYLLDD KELKSLNLSSVEDPEKIKWRQLLGFRNTYTCRNAYGSKMLVSS YECIMRDTNYQFCEVCRLQGFKRMSQLVKDVDLYVATPEVKE YTGAYSKPSDFTDLETSSYYNYTYNRNDRLLSGNSKSRFNTNM NGKKIELRTVIQNISDKNARQLKFKMWIKHSDGSVATDSSGNP LQTVQTFDIPVWNDKANFWPLGALDHIKSDFNSGLKSCSLIYQI PSDAQLKSGDTVAFQVLDENGNVLADDNTETQRYTTVSIQYKF EDGSEIPNTAGGTFTVPYGTKLDLTPAKTLYDYEFIKVDGLNKP IVSDGTVVTYYYKNKNEEHTHNLTLVAAKAATCTTAGNSAYY TCDGCDKWFADATGSVEITDKTSVKIPAPGHTAGTEWKSDDTN HWHECTVAGCGVIIESTKSAHTAGEWIVDTPATATTAGTKHKE CTVCHRVLETQPIPSTGTELKIIAGDNQIYNKASGSDVTITCNGD FAKFTGIKVDGSVVDSSNYTAVSGSTVLTLKASYLGTLTDGSH TITFVYTDGEANANLTVRTAGSGHIHDYGTEWKSNADNHWHE CNCGDKKDEAAHSFKWVVDKEATATKKGSKHEECKICGYKRS AVEIPATGTSTAPTDTTKPNDTTKPGNTNGSEKSPQTGDNS |
| 84 | ASKPDIKVGDYVKMGVYNNASILWRCVSIDNNGPLMLADKIV DTLAYDAKTNDNSNSKSHSRSYKRDDYGSNYWKDSNMRSWL |
| | NSTAAEGKVDWLCGNPPKDGYVSGVGAYNEKAGFLNAFSKSE IAAMKTVTQRSLVSHPEYNKGIVDGDANSDLLYYTDISEAVAN YDSSYFETTTEKVFLLDVKQANAVWKNLKGYYVAYNNDGMA WPYWLRTPVTDCNHDMRYISSSGQVGRYAPWYSDLGVRPAFY LDSEYFVTTSGSGSQSSPYIGSAPNKQEDDYTISEPAEDANPDW NVSTEQSIQLTLGPWYSNDGKYSNPTIPVYTIQKTRSDTENMVV VVCGEGYTKSQQGKFINDVKRLWQDAMKYEPYRSYADRFNV YALCTASESTFDNGGSTFFDVIVDKYNSPVISNNLHGSQWKNHI FERCIGPEFIEKIHDAHIKKKCDPNTIPSGSEYEPYYYVHDYIAQF AMVVNTKSDFGGAYNNREYGFHYFISPSDSYRASKTFAHEFGH GLLGLGDEYSNGYLLDDKELKSLNLSSVEDPEKIKWRQLLGFR NTYTCRNAYGSKMLVSSYECIMRDTNYQFCEVCRLQGFKRMS QLVKDVDLYVATPEVKEYTGAYSKPSDFTDLETSSYYNYTYNR NDRLLSGNSKSRFNTNMNGKKIELRTVIQNISDKNARQLKFKM WIKHSDGSVATDSSGNPLQTVQTFDIPVWNDKANFWPLGALD HIKSDFNSGLKSCSLIYQIPSDAQLKSGDTVAFQVLDENGNVLA DDNTETQRYTTVSIQYGGGGGSESKYGPPCPSCPAPEFLGGPSV FLFPPKPKDTLMISRTPEVTCVVVDVSQEDPEVQFNWYVDGVE VHNAKTKPREEQFNSTYRVVSVLTVLHQDWLNGKEYKCKVSN KGLPSSIEKTISKAKGQPREPQVYTLPPSQEEMTKNQVSLTCLV KGFYPSDIAVEWESNGQPENNYKTTPPVLDSDGSFFLYSRLTVD KSRWQEGNVFSCSVMHEALHNHYTQKSLSLSLGK |
| 85 | ASKPDIKVGDYVKMGVYNNASILWRCVSIDNNGPLMLADKIV DTLAYDAKTNDNSNSKSHSRSYKRDDYGSNYWKDSNMRSWL NSTAAEGKVDWLCGNPPKDGYVSGVGAYNEKAGFLNAFSKSE IAAMKTVTQRSLVSHPEYNKGIVDGDANSDLLYYTDISEAVAN YDSSYFETTTEKVFLLDVKQANAVWKNLKGYYVAYNNDGMA WPYWLRTPVTDCNHDMRYISSSGQVGRYAPWYSDLGVRPAFY LDSEYFVTTSGSGSQSSPYIGSAPNKQEDDYTISEPAEDANPDW NVSTEQSIQLTLGPWYSNDGKYSNPTIPVYTIQKTRSDTENMVV VVCGEGYTKSQQGKFINDVKRLWQDAMKYEPYRSYADRFNV YALCTASESTFDNGGSTFFDVIVDKYNSPVISNNLHGSQWKNHI FERCIGPEFIEKIHDAHIKKKCDPNTIPSGSEYEPYYYVHDYIAQF |
| | AMVVNTKSDFGGAYNNREYGFHYFISPSDSYRASKTFAHEFGH GLLGLGDEYSNGYLLDDKELKSLNLSSVEDPEKIKWRQLLGFR NTYTCRNAYGSKMLVSSYECIMRDTNYQFCEVCRLQGFKRMS QLVKDVDLYVATPEVKEYTGAYSKPSDFTDLETSSYYNYTYNR NDRLLSGNSKSRFNTNMNGKKIELRTVIQNISDKNARQLKFKM WIKHSDGSVATDSSGNPLQTVQTFDIPVWNDKANFWPLGALD HIKSDFNSGLKSCSLIYQIPSDAQLKSGDTVAFQVLDENGNVLA DDNTETQRYTTVSIQYGGGGGSDAHKSEVAHRFKDLGEENFK ALVLIAFAQYLQQCPFEDHVKLVNEVTEFAKTCVADESAENCD KSLHTLFGDKLCTVATLRETYGEMADCCAKQEPERNECFLQH KDDNPNLPRLVRPEVDVMCTAFHDNEETFLKKYLYEIARRHPY FYAPELLFFAKRYKAAFTECCQAADKAACLLPKLDELRDEGKA SSAKQRLKCASLQKFGERAFKAWAVARLSQRFPKAEFAEVSKL VTDLTKVHTECCHGDLLECADDRADLAKYICENQDSISSKLKE CCEKPLLEKSHCIAEVENDEMPADLPSLAADFVESKDVCKNYA EAKDVFLGMFLYEYARRHPDYSVVLLLRLAKTYETTLEKCCA AADPHECYAKVFDEFKPLVEEPQNLIKQNCELFEQLGEYKFQN ALLVRYTKKVPQVSTPTLVEVSRNLGKVGSKCCKHPEAKRMP CAEDYLSVVLNQLCVLHEKTPVSDRVTKCCTESLVNRRPCFSA LEVDETYVPKEFNAETFTFHADICTLSEKERQIKKQTALVELVK HKPKATKEQLKAVMDDFAAFVEKCCKADDKETCFAEEGKKL VAASQAALGL |

In some embodiments, the fusion protein provided herein comprises an amino acid sequence as set forth in SEQ ID NO: 2, SEQ ID NO: 4, SEQ ID NO: 6, SEQ ID NO: 8, SEQ ID NO: 10, or SEQ ID NO: 12. In some embodiments, the fusion protein provided herein comprises an amino acid sequence selected from the group consisting of: SEQ ID NO: 2, SEQ ID NO: 4, SEQ ID NO: 6, SEQ ID NO: 8, SEQ ID NO: 10, SEQ ID NO: 12, or an amino acid sequence having at least 70%, at least 75%, at least 80%, at least 85%, at least 90%, at least 95% sequence identity thereto.

| **SEQ ID NO** | **Amino Acid Sequence** |
|---|---|
| 2 | HMASKPDIKVGDYVKMGVYNNASILWRCVSIDNNGPLMLADK IVDTLAYDAKTNDNSNSKSHSRSYKRDDYGSNYWKDSNMRS WLNSTAAEGKVDWLCGNPPKDGYVSGVGAYNEKAGFLNAFS KSEIAAMKTVTQRSLVSHPEYNKGIVDGDANSDLLYYTDISEA VANYDSSYFETTTEKVFLLDVKQANAVWKNLKGYYVAYNND GMAWPYWLRTPVTDCNHDMRYISSSGQVGRYAPWYSDLGVR PAFYLDSEYFVTTSGSGSQSSPYIGSAPNKQEDDYTISEPAEDAN PDWNVSTEQSIQLTLGPWYSNDGKYSNPTIPVYTIQKTRSDTEN MVVVVCGEGYTKSQQGKFINDVKRLWQDAMKYEPYRSYADR FNVYALCTASESTFDNGGSTFFDVIVDKYNSPVISNNLHGSQW KNHIFERCIGPEFIEKIHDAHIKKKCDPNTIPSGSEYEPYYYVHD YIAQFAMVVNTKSDFGGAYNNREYGFHYFISPSDSYRASKTFA HEFGHGLLGLGDEYSNGYLLDDKELKSLNLSSVEDPEKIKWRQ LLGFRNTYTCRNAYGSKMLVSSYECIMRDTNYQFCEVCRLQGF KRMSQLVKDVDLYVATPEVKEYTGAYSKPSDFTDLETSSYYN YTYNRNDRLLSGNSKSRFNTNMNGKKIELRTVIQNISDKNARQ LKFKMWIKHSDGSVATDSSGNPLQTVQTFDIPVWNDKANFWP LGALDHIKSDFNSGLKSCSLIYQIPSDAQLKSGDTVAFQVLGGG GSEPKSCDKTHTCPPCPAPELLGGPSVFLFPPKPKDTLMISRTPE VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGKHHHHHHAA |
| 4 | KLMYRMQLLSCIALSLALVTNSGTASKPDIKVGDYVKMGVYN NASILWRCVSIDNNGPLMLADKIVDTLAYDAKTNDNSNSKSHS RSYKRDDYGSNYWKDSNMRSWLNSTAAEGKVDWLCGNPPKD GYVSGVGAYNEKAGFLNAFSKSEIAAMKTVTQRSLVSHPEYN KGIVDGDANSDLLYYTDISEAVANYDSSYFETTTEKVFLLDVK QANAVWKNLKGYYVAYNNDGMAWPYWLRTPVTDCNHDMR |
| | YISSSGQVGRYAPWYSDLGVRPAFYLDSEYFVTTSGSGSQSSPY IGSAPNKQEDDYTISEPAEDANPDWNVSTEQSIQLTLGPWYSND GKYSNPTIPVYTIQKTRSDTENMVVVVCGEGYTKSQQGKFIND VKRLWQDAMKYEPYRSYADRFNVYALCTASESTFDNGGSTFF DVIVDKYNSPVISNNLHGSQWKNHIFERCIGPEFIEKIHDAHIKK KCDPNTIPSGSEYEPYYYVHDYIAQFAMVVNTKSDFGGAYNNR EYGFHYFISPSDSYRASKTFAHEFGHGLLGLGDEYSNGYLLDD KELKSLNLSSVEDPEKIKRQLLGFRNTYTCRNAYGSKMLVSSY ECIMRDTNYQFCEVCRLQGFKRMSQLVKDVDLYVATPEVKEY TGAYSKPSDFTDLETSSYYNYTYNRNDRLLSGNSKSRFNTNMN GKKIELRTVIQNISDKNARQLKFKMWIKHSDGSVATDSSGNPL QTVQTFDIPVWNDKANFWPLGALDHIKSDFNSGLKSCSLIYQIP SDAQLKSGDTVAFQVLGGGGSEPKSCDKTHTCPPCPAPELLGG PSVFLFPPKPKDTLMISRTPEVTCVVVDVSHEDPEVKFNWYVD GVEVHNAKTKPREEQYNSTYRVVSVLTVLHQDWLNGKEYKC KVSNKALPAPIEKTISKAKGQPREPQVYTLPPSRDELTKNQVSL TCLVKGFYPSDIAVEWESNGQPENNYKTTPPVLDSDGSFFLYSK LTVDKSRWQQGNVFSCSVMHEALHNHYTQKSLSLSPGKHHHH HHHHAA |
| 6 | HMASKPDIKVGDYVKMGVYNNASILWRCVSIDNNGPLMLADK IVDTLAYDAKTNDNSNSKSHSRSYKRDDYGSNYWKDSNMRS WLNSTAAEGKVDWLCGNPPKDGYVSGVGAYNEKAGFLNAFS KSEIAAMKTVTQRSLVSHPEYNKGIVDGDANSDLLYYTDISEA VANYDSSYFETTTEKVFLLDVKQANAVWKNLKGYYVAYNND GMAWPYWLRTPVTDCNHDMRYISSSGQVGRYAPWYSDLGVR PAFYLDSEYFVTTSGSGSQSSPYIGSAPNKQEDDYTISEPAEDAN PDWNVSTEQSIQLTLGPWYSNDGKYSNPTIPVYTIQKTRSDTEN MVVVVCGEGYTKSQQGKFINDVKRLWQDAMKYEPYRSYADR FNVYALCTASESTFDNGGSTFFDVIVDKYNSPVISNNLHGSQW KNHIFERCIGPEFIEKIHDAHIKKKCDPNTIPSGSEYEPYYYVHD YIAQFAMVVNTKSDFGGAYNNREYGFHYFISPSDSYRASKTFA HEFGHGLLGLGDEYSNGYLLDDKELKSLNLSSVEDPEKIKWRQ LLGFRNTYTCRNAYGSKMLVSSYECIMRDTNYQFCEVCRLQGF |
| | KRMSQLVKDVDLYVATPEVKEYTGAYSKPSDFTDLETSSYYN YTYNRNDRLLSGNSKSRFNTNMNGKKIELRTVIQNISDKNARQ LKFKMWIKHSDGSVATDSSGNPLQTVQTFDIPVWNDKANFWP LGALDHIKSDFNSGLKSCSLIYQIPSDAQLKSGDTVAFQVLDEN GNVGGGGSHHHHHHHHHTCPPCPAPELLGGPSVFLFPPKPKD TLMISRTPEVTCVVVDVSHEDPEVKFNWYVDGVEVHNAKTKP REEQYNSTYRVVSVLTVLHQDWLNGKEYKCKVSNKALPAPIE KTISKAKGQPREPQVYTLPPSRDELTKNQVSLTCLVKGFYPSDI AVEWESNGQPENNYKTTPPVLDSDGSFFLYSKLTVDKSRWQQ GNVFSCSVMHEALHNHYTQKSLSLSPGKAA |
| 8 | HMASKPDIKVGDYVKMGVYNNASILWRCVSIDNNGPLMLADK IVDTLAYDAKTNDNSNSKSHSRSYKRDDYGSNYWKDSNMRS WLNSTAAEGKVDWLCGNPPKDGYVSGVGAYNEKAGFLNAFS KSEIAAMKTVTQRSLVSHPEYNKGIVDGDANSDLLYYTDISEA VANYDSSYFETTTEKVFLLDVKQANAVWKNLKGYYVAYNND GMAWPYWLRTPVTDCNHDMRYISSSGQVGRYAPWYSDLGVR PAFYLDSEYFVTTSGSGSQSSPYIGSAPNKQEDDYTISEPAEDAN PDWNVSTEQSIQLTLGPWYSNDGKYSNPTIPVYTIQKTRSDTEN MVVVVCGEGYTKSQQGKFINDVKRLWQDAMKYEPYRSYADR FNVYALCTASESTFDNGGSTFFDVIVDKYNSPVISNNLHGSQW KNHIFERCIGPEFIEKIHDAHIKKKCDPNTIPSGSEYEPYYYVHD YIAQFAMVVNTKSDFGGAYNNREYGFHYFISPSDSYRASKTFA HEFGHGLLGLGDEYSNGYLLDDKELKSLNLSSVEDPEKIKWRQ LLGFRNTYTCRNAYGSKMLVSSYECIMRDTNYQFCEVCRLQGF KRMSQLVKDVDLYVATPEVKEYTGAYSKPSDFTDLETSSYYN YTYNRNDRLLSGNSKSRFNTNMNGKKIELRTVIQNISDKNARQ LKFKMWIKHSDGSVATDSSGNPLQTVQTFDIPVWNDKANFWP LGALDHIKSDFNSGLKSCSLIYQIPSDAQLKSGDTVAFQVLDEN GNVLADDNTETQGGGGSHHHHHHHHHHTCPPCPAPELLGGPS VFLFPPKPKDTLMISRTPEVTCVVVDVSHEDPEVKFNWYVDGV EVHNAKTKPREEQYNSTYRVVSVLTVLHQDWLNGKEYKCKVS NKALPAPIEKTISKAKGQPREPQVYTLPPSRDELTKNQVSLTCL VKGFYPSDIAVEWESNGQPENNYKTTPPVLDSDGSFFLYSKLTV |
| | DKSRWQQGNVFSCSVMHEALHNHYTQKSLSLSPGKAA |
| 10 | HMASKPDIKVGDYVKMGVYNNASILWRCVSIDNNGPLMLADK IVDTLAYDAKTNDNSNSKSHSRSYKRDDYGSNYWKDSNMRS WLNSTAAEGKVDWLCGNPPKDGYVSGVGAYNEKAGFLNAFS KSEIAAMKTVTQRSLVSHPEYNKGIVDGDANSDLLYYTDISEA VANYDSSYFETTTEKVFLLDVKQANAVWKNLKGYYVAYNND GMAWPYWLRTPVTDCNHDMRYISSSGQVGRYAPWYSDLGVR PAFYLDSEYFVTTSGSGSQSSPYIGSAPNKQEDDYTISEPAEDAN PDWNVSTEQSIQLTLGPWYSNDGKYSNPTIPVYTIQKTRSDTEN MVVVVCGEGYTKSQQGKFINDVKRLWQDAMKYEPYRSYADR FNVYALCTASESTFDNGGSTFFDVIVDKYNSPVISNNLHGSQW KNHIFERCIGPEFIEKIHDAHIKKKCDPNTIPSGSEYEPYYYVHD YIAQFAMVVNTKSDFGGAYNNREYGFHYFISPSDSYRASKTFA HEFGHGLLGLGDEYSNGYLLDDKELKSLNLSSVEDPEKIKWRQ LLGFRNTYTCRNAYGSKMLVSSYECIMRDTNYQFCEVCRLQGF KRMSQLVKDVDLYVATPEVKEYTGAYSKPSDFTDLETSSYYN YTYNRNDRLLSGNSKSRFNTNMNGKKIELRTVIQNISDKNARQ LKFKMWIKHSDGSVATDSSGNPLQTVQTFDIPVWNDKANFWP LGALDHIKSDFNSGLKSCSLIYQIPSDAQLKSGDTVAFQVLDEN GNVLADDNTETQRYTTVSIQYGGGGSHHHHHHHHHTCPPCP APELLGGPSVFLFPPKPKDTLMISRTPEVTCVVVDVSHEDPEVK FNWYVDGVEVHNAKTKPREEQYNSTYRVVSVLTVLHQDWLN GKEYKCKVSNKALPAPIEKTISKAKGQPREPQVYTLPPSRDELT KNQVSLTCLVKGFYPSDIAVEWESNGQPENNYKTTPPVLDSDG SFFLYSKLTVDKSRWQQGNVFSCSVMHEALHNHYTQKSLSLSP GKAA |
| 12 | HMASKPDIKVGDYVKMGVYNNASILWRCVSIDNNGPLMLADK IVDTLAYDAKTNDNSNSKSHSRSYKRDDYGSNYWKDSNMRS WLNSTAAEGKVDWLCGNPPKDGYVSGVGAYNEKAGFLNAFS KSEIAAMKTVTQRSLVSHPEYNKGIVDGDANSDLLYYTDISEA VANYDSSYFETTTEKVFLLDVKQANAVWKNLKGYYVAYNND GMAWPYWLRTPVTDCNHDMRYISSSGQVGRYAPWYSDLGVR |
| | PAFYLDSEYFVTTSGSGSQSSPYIGSAPNKQEDDYTISEPAEDAN PDWNVSTEQSIQLTLGPWYSNDGKYSNPTIPVYTIQKTRSDTEN MVVVVCGEGYTKSQQGKFINDVKRLWQDAMKYEPYRSYADR FNVYALCTASESTFDNGGSTFFDVIVDKYNSPVISNNLHGSQW KNHIFERCIGPEFIEKIHDAHIKKKCDPNTIPSGSEYEPYYYVHD YIAQFAMVVNTKSDFGGAYNNREYGFHYFISPSDSYRASKTFA HEFGHGLLGLGDEYSNGYLLDDKELKSLNLSSVEDPEKIKWRQ LLGFRNTYTCRNAYGSKMLVSSYECIMRDTNYQFCEVCRLQGF KRMSQLVKDVDLYVATPEVKEYTGAYSKPSDFTDLETSSYYN YTYNRNDRLLSGNSKSRFNTNMNGKKIELRTVIQNISDKNARQ LKFKMWIKHSDGSVATDSSGNPLQTVQTFDIPVWNDKANFWP LGALDHIKSDFNSGLKSCSLIYQIPSDAQLKSGDTVAFQVLDEN GNVLADDNTETQRYTTVSIQYKFEDGSEIPNTAGGTFTGGGGS HHHHHHHHHTCPPCPAPELLGGPSVFLFPPKPKDTLMISRTPE VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGKAA |

In some embodiments, the fusion protein has a half-life of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, or at least 14 days in blood circulation of a subject.

### Nucleic Acid

In another aspect, the present disclosure provides an isolated nucleic acid comprising a nucleotide sequence encoding the truncated form of IgA protease described herein or comprising a nucleotide sequence encoding the fusion protein described herein.

As used herein, the term "nucleic acid" or "nucleotide" refers to deoxyribonucleic acid (DNA) or ribonucleic acid (RNA) in single-stranded or doublestranded form and polymers thereof. Unless otherwise indicated, a particular polynucleotide sequence also implicitly encompasses conservatively modified variants thereof (*e.g*., degenerate codon substitutions), alleles, orthologs, SNPs, and complementary sequences as well as the sequence explicitly indicated. Specifically, degenerate codon substitutions may be achieved by generating sequences in which the third position of one or more (or all) selected codons is substituted with mixed-base and/or deoxyinosine residues (see Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

Using the convention procedures, the DNA encoding the truncated form of IgA protease or the DNA encoding the fusion protein described herein can be easily isolated and sequenced (*e.g.*, by using oligonucleotide probes capable of binding specifically to the gene encoding the truncated form of IgA protease or fusion protein). The encoding DNA may also be obtained by synthetic methods.

In some embodiments, the nucleic acid provided herein comprises a nucleic acid sequence as set forth in SEQ ID NO: 33, SEQ ID NO: 34, SEQ ID NO: 35, SEQ ID NO: 36, SEQ ID NO: 37, or SEQ ID NO: 38. In some embodiments, the nucleic acid provided herein comprises a nucleotide sequence selected from the group consisting of SEQ ID NO: 33, SEQ ID NO: 34, SEQ ID NO: 35, SEQ ID NO: 36, SEQ ID NO: 37, SEQ ID NO: 38, or a nucleotide sequence having at least 90%, at least 91%, at least 92%, at least 93%, at least 94%, at least 95%, at least 96%, at least 97%, at least 98%, at least 99% sequence identity thereto.

| **SEQ ID NO** | **Nucleotide Sequence** |
|---|---|
| 33 | GCGAGCAAACCGGACATCAAAGTGGGCGACTACGTGAAAAT GGGTGTGTATAATAACGCAAGCATCCTGTGGCGCTGTGTGA GCATCGACAACAATGGCCCGCTGATGCTGGCCGATAAAATT |
| | GTTGACACGCTGGCGTATGATGCTAAAACCAACGACAATTC GAACAGCAAATCTCATAGTCGTTCCTACAAACGCGATGACT ACGGCAGCAACTATTGGAAAGATAGTAATATGCGCTCCTGG CTGAACTCAACCGCGGCCGAGGGTAAAGTGGATTGGCTGTG CGGCAATCCGCCGAAAGACGGTTACGTCAGCGGCGTGGGTG CATATAATGAAAAAGCTGGTTTTCTGAACGCGTTCTCAAAAT CGGAAATTGCAGCTATGAAAACGGTGACCCAGCGTAGCCTG GTTTCTCATCCGGAATATAATAAAGGCATTGTTGATGGTGAC GCGAACTCGGATCTGCTGTATTACACCGACATCAGCGAAGC AGTGGCTAACTACGATAGCTCTTATTTTGAAACCACGACCGA AAAAGTTTTCCTGCTGGATGTCAAACAGGCGAACGCCGTCT GGAAAAATCTGAAAGGCTATTACGTGGCTTACAACAATGAT GGTATGGCATGGCCGTATTGGCTGCGTACCCCGGTGACGGA TTGTAATCATGACATGCGCTATATTAGTTCCTCAGGCCAGGT TGGTCGTTACGCTCCGTGGTATTCTGATCTGGGCGTCCGTCC GGCGTTTTACCTGGACAGTGAATATTTCGTGACGACCAGCGG CTCTGGTAGTCAGTCGAGCCCGTACATTGGTTCCGCGCCGAA CAAACAAGAAGATGACTATACCATCTCAGAACCGGCGGAAG ATGCCAACCCGGACTGGAATGTTTCGACGGAACAGAGCATT CAACTGACCCTGGGCCCGTGGTACTCGAATGATGGTAAATA TAGCAACCCGACCATTCCGGTGTATACCATCCAGAAAACGC GCTCGGATACCGAAAACATGGTGGTTGTCGTGTGCGGCGAA GGTTATACCAAATCACAGCAAGGCAAATTTATCAATGATGTT AAACGTCTGTGGCAGGACGCTATGAAATATGAACCGTACCG TAGCTATGCGGATCGCTTTAATGTGTATGCACTGTGTACGGC TTCCGAATCAACCTTCGATAACGGCGGTTCTACCTTTTTCGA TGTGATCGTTGACAAATACAACTCTCCGGTTATCAGTAACAA TCTGCATGGCAGTCAGTGGAAAAATCACATTTTTGAACGCTG CATCGGTCCGGAATTCATTGAAAAAATCCATGATGCCCACAT TAAGAAAAAATGTGACCCGAACACCATCCCGTCGGGTAGCG AATACGAACCGTATTACTATGTGCATGATTATATTGCACAGT TTGCTATGGTTGTCAATACCAAATCCGACTTCGGCGGTGCAT ATAACAATCGCGAATACGGCTTTCACTATTTCATCTCTCCGA |
| | GTGATTCCTACCGTGCCTCTAAAACCTTTGCACATGAATTCG GCCACGGTCTGCTGGGCCTGGGTGATGAATACTCGAATGGTT ATCTGCTGGATGACAAAGAACTGAAAAGCCTGAACCTGTCT AGTGTGGAAGATCCGGAAAAAATTAAATGGCGTCAGCTGCT GGGCTTTCGCAATACGTACACCTGCCGTAACGCGTATGGTTC TAAAATGCTGGTTTCCTCATACGAATGTATCATGCGCGATAC CAACTATCAATTTTGCGAAGTCTGTCGCCTGCAGGGCTTCAA ACGTATGAGCCAACTGGTTAAAGATGTCGACCTGTATGTGG CCACGCCGGAAGTTAAAGAATACACCGGTGCATATAGTAAA CCGTCCGATTTTACGGACCTGGAAACCTCGAGCTACTACAAC TACACCTACAACCGTAACGATCGCCTGCTGAGTGGCAACTC AAAATCGCGTTTCAATACGAACATGAATGGCAAGAAAATTG AACTGCGCACCGTTATTCAGAACATCAGCGATAAAAACGCC CGTCAACTGAAATTCAAAATGTGGATCAAACATTCAGATGG CTCGGTGGCAACCGACTCTAGTGGTAACCCGCTGCAGACCG TCCAAACGTTTGATATTCCGGTGTGGAACGACAAAGCCAATT TCTGGCCGCTGGGCGCACTGGATCACATCAAATCCGACTTTA ATTCAGGTCTGAAAAGCTGCTCTCTGATTTATCAGATCCCGT CTGATGCTCAACTGAAAAGTGGCGACACCGTGGCGTTCCAG GTTCTG |
| 34 | GCGAGCAAACCGGACATCAAAGTGGGCGACTACGTGAAAAT GGGTGTGTATAATAACGCAAGCATCCTGTGGCGCTGTGTGA GCATCGACAACAATGGCCCGCTGATGCTGGCCGATAAAATT GTTGACACGCTGGCGTATGATGCTAAAACCAACGACAATTC GAACAGCAAATCTCATAGTCGTTCCTACAAACGCGATGACT ACGGCAGCAACTATTGGAAAGATAGTAATATGCGCTCCTGG CTGAACTCAACCGCGGCCGAGGGTAAAGTGGATTGGCTGTG CGGCAATCCGCCGAAAGACGGTTACGTCAGCGGCGTGGGTG CATATAATGAAAAAGCTGGTTTTCTGAACGCGTTCTCAAAAT CGGAAATTGCAGCTATGAAAACGGTGACCCAGCGTAGCCTG GTTTCTCATCCGGAATATAATAAAGGCATTGTTGATGGTGAC GCGAACTCGGATCTGCTGTATTACACCGACATCAGCGAAGC AGTGGCTAACTACGATAGCTCTTATTTTGAAACCACGACCGA |
| | AAAAGTTTTCCTGCTGGATGTCAAACAGGCGAACGCCGTCT GGAAAAATCTGAAAGGCTATTACGTGGCTTACAACAATGAT GGTATGGCATGGCCGTATTGGCTGCGTACCCCGGTGACGGA TTGTAATCATGACATGCGCTATATTAGTTCCTCAGGCCAGGT TGGTCGTTACGCTCCGTGGTATTCTGATCTGGGCGTCCGTCC GGCGTTTTACCTGGACAGTGAATATTTCGTGACGACCAGCGG CTCTGGTAGTCAGTCGAGCCCGTACATTGGTTCCGCGCCGAA CAAACAAGAAGATGACTATACCATCTCAGAACCGGCGGAAG ATGCCAACCCGGACTGGAATGTTTCGACGGAACAGAGCATT CAACTGACCCTGGGCCCGTGGTACTCGAATGATGGTAAATA TAGCAACCCGACCATTCCGGTGTATACCATCCAGAAAACGC GCTCGGATACCGAAAACATGGTGGTTGTCGTGTGCGGCGAA GGTTATACCAAATCACAGCAAGGCAAATTTATCAATGATGTT AAACGTCTGTGGCAGGACGCTATGAAATATGAACCGTACCG TAGCTATGCGGATCGCTTTAATGTGTATGCACTGTGTACGGC TTCCGAATCAACCTTCGATAACGGCGGTTCTACCTTTTTCGA TGTGATCGTTGACAAATACAACTCTCCGGTTATCAGTAACAA TCTGCATGGCAGTCAGTGGAAAAATCACATTTTTGAACGCTG CATCGGTCCGGAATTCATTGAAAAAATCCATGATGCCCACAT TAAGAAAAAATGTGACCCGAACACCATCCCGTCGGGTAGCG AATACGAACCGTATTACTATGTGCATGATTATATTGCACAGT TTGCTATGGTTGTCAATACCAAATCCGACTTCGGCGGTGCAT ATAACAATCGCGAATACGGCTTTCACTATTTCATCTCTCCGA GTGATTCCTACCGTGCCTCTAAAACCTTTGCACATGAATTCG GCCACGGTCTGCTGGGCCTGGGTGATGAATACTCGAATGGTT ATCTGCTGGATGACAAAGAACTGAAAAGCCTGAACCTGTCT AGTGTGGAAGATCCGGAAAAAATTAAATGGCGTCAGCTGCT GGGCTTTCGCAATACGTACACCTGCCGTAACGCGTATGGTTC TAAAATGCTGGTTTCCTCATACGAATGTATCATGCGCGATAC CAACTATCAATTTTGCGAAGTCTGTCGCCTGCAGGGCTTCAA ACGTATGAGCCAACTGGTTAAAGATGTCGACCTGTATGTGG CCACGCCGGAAGTTAAAGAATACACCGGTGCATATAGTAAA CCGTCCGATTTTACGGACCTGGAAACCTCGAGCTACTACAAC |
| | TACACCTACAACCGTAACGATCGCCTGCTGAGTGGCAACTC AAAATCGCGTTTCAATACGAACATGAATGGCAAGAAAATTG AACTGCGCACCGTTATTCAGAACATCAGCGATAAAAACGCC CGTCAACTGAAATTCAAAATGTGGATCAAACATTCAGATGG CTCGGTGGCAACCGACTCTAGTGGTAACCCGCTGCAGACCG TCCAAACGTTTGATATTCCGGTGTGGAACGACAAAGCCAATT TCTGGCCGCTGGGCGCACTGGATCACATCAAATCCGACTTTA ATTCAGGTCTGAAAAGCTGCTCTCTGATTTATCAGATCCCGT CTGATGCTCAACTGAAAAGTGGCGACACCGTGGCGTTCCAG GTTCTG |
| 35 | GCGAGCAAACCGGACATCAAAGTGGGCGACTACGTGAAAAT GGGTGTGTATAATAACGCAAGCATCCTGTGGCGCTGTGTGA GCATCGACAACAATGGCCCGCTGATGCTGGCCGATAAAATT GTTGACACGCTGGCGTATGATGCTAAAACCAACGACAATTC GAACAGCAAATCTCATAGTCGTTCCTACAAACGCGATGACT ACGGCAGCAACTATTGGAAAGATAGTAATATGCGCTCCTGG CTGAACTCAACCGCGGCCGAGGGTAAAGTGGATTGGCTGTG CGGCAATCCGCCGAAAGACGGTTACGTCAGCGGCGTGGGTG CATATAATGAAAAAGCTGGTTTTCTGAACGCGTTCTCAAAAT CGGAAATTGCAGCTATGAAAACGGTGACCCAGCGTAGCCTG GTTTCTCATCCGGAATATAATAAAGGCATTGTTGATGGTGAC GCGAACTCGGATCTGCTGTATTACACCGACATCAGCGAAGC AGTGGCTAACTACGATAGCTCTTATTTTGAAACCACGACCGA AAAAGTTTTCCTGCTGGATGTCAAACAGGCGAACGCCGTCT GGAAAAATCTGAAAGGCTATTACGTGGCTTACAACAATGAT GGTATGGCATGGCCGTATTGGCTGCGTACCCCGGTGACGGA TTGTAATCATGACATGCGCTATATTAGTTCCTCAGGCCAGGT TGGTCGTTACGCTCCGTGGTATTCTGATCTGGGCGTCCGTCC GGCGTTTTACCTGGACAGTGAATATTTCGTGACGACCAGCGG CTCTGGTAGTCAGTCGAGCCCGTACATTGGTTCCGCGCCGAA CAAACAAGAAGATGACTATACCATCTCAGAACCGGCGGAAG ATGCCAACCCGGACTGGAATGTTTCGACGGAACAGAGCATT CAACTGACCCTGGGCCCGTGGTACTCGAATGATGGTAAATA |
| | TAGCAACCCGACCATTCCGGTGTATACCATCCAGAAAACGC GCTCGGATACCGAAAACATGGTGGTTGTCGTGTGCGGCGAA GGTTATACCAAATCACAGCAAGGCAAATTTATCAATGATGTT AAACGTCTGTGGCAGGACGCTATGAAATATGAACCGTACCG TAGCTATGCGGATCGCTTTAATGTGTATGCACTGTGTACGGC TTCCGAATCAACCTTCGATAACGGCGGTTCTACCTTTTTCGA TGTGATCGTTGACAAATACAACTCTCCGGTTATCAGTAACAA TCTGCATGGCAGTCAGTGGAAAAATCACATTTTTGAACGCTG CATCGGTCCGGAATTCATTGAAAAAATCCATGATGCCCACAT TAAGAAAAAATGTGACCCGAACACCATCCCGTCGGGTAGCG AATACGAACCGTATTACTATGTGCATGATTATATTGCACAGT TTGCTATGGTTGTCAATACCAAATCCGACTTCGGCGGTGCAT ATAACAATCGCGAATACGGCTTTCACTATTTCATCTCTCCGA GTGATTCCTACCGTGCCTCTAAAACCTTTGCACATGAATTCG GCCACGGTCTGCTGGGCCTGGGTGATGAATACTCGAATGGTT ATCTGCTGGATGACAAAGAACTGAAAAGCCTGAACCTGTCT AGTGTGGAAGATCCGGAAAAAATTAAATGGCGTCAGCTGCT GGGCTTTCGCAATACGTACACCTGCCGTAACGCGTATGGTTC TAAAATGCTGGTTTCCTCATACGAATGTATCATGCGCGATAC CAACTATCAATTTTGCGAAGTCTGTCGCCTGCAGGGCTTCAA ACGTATGAGCCAACTGGTTAAAGATGTCGACCTGTATGTGG CCACGCCGGAAGTTAAAGAATACACCGGTGCATATAGTAAA CCGTCCGATTTTACGGACCTGGAAACCTCGAGCTACTACAAC TACACCTACAACCGTAACGATCGCCTGCTGAGTGGCAACTC AAAATCGCGTTTCAATACGAACATGAATGGCAAGAAAATTG AACTGCGCACCGTTATTCAGAACATCAGCGATAAAAACGCC CGTCAACTGAAATTCAAAATGTGGATCAAACATTCAGATGG CTCGGTGGCAACCGACTCTAGTGGTAACCCGCTGCAGACCG TCCAAACGTTTGATATTCCGGTGTGGAACGACAAAGCCAATT TCTGGCCGCTGGGCGCACTGGATCACATCAAATCCGACTTTA ATTCAGGTCTGAAAAGCTGCTCTCTGATTTATCAGATCCCGT CTGATGCTCAACTGAAAAGTGGCGACACCGTGGCGTTCCAG GTTCTGGATGAAAACGGTAATGTG |
| 36 | GCGAGCAAACCGGACATCAAAGTGGGCGACTACGTGAAAAT GGGTGTGTATAATAACGCAAGCATCCTGTGGCGCTGTGTGA GCATCGACAACAATGGCCCGCTGATGCTGGCCGATAAAATT GTTGACACGCTGGCGTATGATGCTAAAACCAACGACAATTC GAACAGCAAATCTCATAGTCGTTCCTACAAACGCGATGACT ACGGCAGCAACTATTGGAAAGATAGTAATATGCGCTCCTGG CTGAACTCAACCGCGGCCGAGGGTAAAGTGGATTGGCTGTG CGGCAATCCGCCGAAAGACGGTTACGTCAGCGGCGTGGGTG CATATAATGAAAAAGCTGGTTTTCTGAACGCGTTCTCAAAAT CGGAAATTGCAGCTATGAAAACGGTGACCCAGCGTAGCCTG GTTTCTCATCCGGAATATAATAAAGGCATTGTTGATGGTGAC GCGAACTCGGATCTGCTGTATTACACCGACATCAGCGAAGC AGTGGCTAACTACGATAGCTCTTATTTTGAAACCACGACCGA AAAAGTTTTCCTGCTGGATGTCAAACAGGCGAACGCCGTCT GGAAAAATCTGAAAGGCTATTACGTGGCTTACAACAATGAT GGTATGGCATGGCCGTATTGGCTGCGTACCCCGGTGACGGA TTGTAATCATGACATGCGCTATATTAGTTCCTCAGGCCAGGT TGGTCGTTACGCTCCGTGGTATTCTGATCTGGGCGTCCGTCC GGCGTTTTACCTGGACAGTGAATATTTCGTGACGACCAGCGG CTCTGGTAGTCAGTCGAGCCCGTACATTGGTTCCGCGCCGAA CAAACAAGAAGATGACTATACCATCTCAGAACCGGCGGAAG ATGCCAACCCGGACTGGAATGTTTCGACGGAACAGAGCATT CAACTGACCCTGGGCCCGTGGTACTCGAATGATGGTAAATA TAGCAACCCGACCATTCCGGTGTATACCATCCAGAAAACGC GCTCGGATACCGAAAACATGGTGGTTGTCGTGTGCGGCGAA GGTTATACCAAATCACAGCAAGGCAAATTTATCAATGATGTT AAACGTCTGTGGCAGGACGCTATGAAATATGAACCGTACCG TAGCTATGCGGATCGCTTTAATGTGTATGCACTGTGTACGGC TTCCGAATCAACCTTCGATAACGGCGGTTCTACCTTTTTCGA TGTGATCGTTGACAAATACAACTCTCCGGTTATCAGTAACAA TCTGCATGGCAGTCAGTGGAAAAATCACATTTTTGAACGCTG CATCGGTCCGGAATTCATTGAAAAAATCCATGATGCCCACAT TAAGAAAAAATGTGACCCGAACACCATCCCGTCGGGTAGCG |
| | AATACGAACCGTATTACTATGTGCATGATTATATTGCACAGT TTGCTATGGTTGTCAATACCAAATCCGACTTCGGCGGTGCAT ATAACAATCGCGAATACGGCTTTCACTATTTCATCTCTCCGA GTGATTCCTACCGTGCCTCTAAAACCTTTGCACATGAATTCG GCCACGGTCTGCTGGGCCTGGGTGATGAATACTCGAATGGTT ATCTGCTGGATGACAAAGAACTGAAAAGCCTGAACCTGTCT AGTGTGGAAGATCCGGAAAAAATTAAATGGCGTCAGCTGCT GGGCTTTCGCAATACGTACACCTGCCGTAACGCGTATGGTTC TAAAATGCTGGTTTCCTCATACGAATGTATCATGCGCGATAC CAACTATCAATTTTGCGAAGTCTGTCGCCTGCAGGGCTTCAA ACGTATGAGCCAACTGGTTAAAGATGTCGACCTGTATGTGG CCACGCCGGAAGTTAAAGAATACACCGGTGCATATAGTAAA CCGTCCGATTTTACGGACCTGGAAACCTCGAGCTACTACAAC TACACCTACAACCGTAACGATCGCCTGCTGAGTGGCAACTC AAAATCGCGTTTCAATACGAACATGAATGGCAAGAAAATTG AACTGCGCACCGTTATTCAGAACATCAGCGATAAAAACGCC CGTCAACTGAAATTCAAAATGTGGATCAAACATTCAGATGG CTCGGTGGCAACCGACTCTAGTGGTAACCCGCTGCAGACCG TCCAAACGTTTGATATTCCGGTGTGGAACGACAAAGCCAATT TCTGGCCGCTGGGCGCACTGGATCACATCAAATCCGACTTTA ATTCAGGTCTGAAAAGCTGCTCTCTGATTTATCAGATCCCGT CTGATGCTCAACTGAAAAGTGGCGACACCGTGGCGTTCCAG GTTCTGGATGAAAACGGTAATGTGCTGGCGGATGACAACAC GGAAACCCAG |
| 37 | GCGAGCAAACCGGACATCAAAGTGGGCGACTACGTGAAAAT GGGTGTGTATAATAACGCAAGCATCCTGTGGCGCTGTGTGA GCATCGACAACAATGGCCCGCTGATGCTGGCCGATAAAATT GTTGACACGCTGGCGTATGATGCTAAAACCAACGACAATTC GAACAGCAAATCTCATAGTCGTTCCTACAAACGCGATGACT ACGGCAGCAACTATTGGAAAGATAGTAATATGCGCTCCTGG CTGAACTCAACCGCGGCCGAGGGTAAAGTGGATTGGCTGTG CGGCAATCCGCCGAAAGACGGTTACGTCAGCGGCGTGGGTG |
| | CATATAATGAAAAAGCTGGTTTTCTGAACGCGTTCTCAAAAT CGGAAATTGCAGCTATGAAAACGGTGACCCAGCGTAGCCTG GTTTCTCATCCGGAATATAATAAAGGCATTGTTGATGGTGAC GCGAACTCGGATCTGCTGTATTACACCGACATCAGCGAAGC AGTGGCTAACTACGATAGCTCTTATTTTGAAACCACGACCGA AAAAGTTTTCCTGCTGGATGTCAAACAGGCGAACGCCGTCT GGAAAAATCTGAAAGGCTATTACGTGGCTTACAACAATGAT GGTATGGCATGGCCGTATTGGCTGCGTACCCCGGTGACGGA TTGTAATCATGACATGCGCTATATTAGTTCCTCAGGCCAGGT TGGTCGTTACGCTCCGTGGTATTCTGATCTGGGCGTCCGTCC GGCGTTTTACCTGGACAGTGAATATTTCGTGACGACCAGCGG CTCTGGTAGTCAGTCGAGCCCGTACATTGGTTCCGCGCCGAA CAAACAAGAAGATGACTATACCATCTCAGAACCGGCGGAAG ATGCCAACCCGGACTGGAATGTTTCGACGGAACAGAGCATT CAACTGACCCTGGGCCCGTGGTACTCGAATGATGGTAAATA TAGCAACCCGACCATTCCGGTGTATACCATCCAGAAAACGC GCTCGGATACCGAAAACATGGTGGTTGTCGTGTGCGGCGAA GGTTATACCAAATCACAGCAAGGCAAATTTATCAATGATGTT AAACGTCTGTGGCAGGACGCTATGAAATATGAACCGTACCG TAGCTATGCGGATCGCTTTAATGTGTATGCACTGTGTACGGC TTCCGAATCAACCTTCGATAACGGCGGTTCTACCTTTTTCGA TGTGATCGTTGACAAATACAACTCTCCGGTTATCAGTAACAA TCTGCATGGCAGTCAGTGGAAAAATCACATTTTTGAACGCTG CATCGGTCCGGAATTCATTGAAAAAATCCATGATGCCCACAT TAAGAAAAAATGTGACCCGAACACCATCCCGTCGGGTAGCG AATACGAACCGTATTACTATGTGCATGATTATATTGCACAGT TTGCTATGGTTGTCAATACCAAATCCGACTTCGGCGGTGCAT ATAACAATCGCGAATACGGCTTTCACTATTTCATCTCTCCGA GTGATTCCTACCGTGCCTCTAAAACCTTTGCACATGAATTCG |
| | GCCACGGTCTGCTGGGCCTGGGTGATGAATACTCGAATGGTT ATCTGCTGGATGACAAAGAACTGAAAAGCCTGAACCTGTCT AGTGTGGAAGATCCGGAAAAAATTAAATGGCGTCAGCTGCT GGGCTTTCGCAATACGTACACCTGCCGTAACGCGTATGGTTC TAAAATGCTGGTTTCCTCATACGAATGTATCATGCGCGATAC CAACTATCAATTTTGCGAAGTCTGTCGCCTGCAGGGCTTCAA ACGTATGAGCCAACTGGTTAAAGATGTCGACCTGTATGTGG CCACGCCGGAAGTTAAAGAATACACCGGTGCATATAGTAAA CCGTCCGATTTTACGGACCTGGAAACCTCGAGCTACTACAAC TACACCTACAACCGTAACGATCGCCTGCTGAGTGGCAACTC AAAATCGCGTTTCAATACGAACATGAATGGCAAGAAAATTG AACTGCGCACCGTTATTCAGAACATCAGCGATAAAAACGCC CGTCAACTGAAATTCAAAATGTGGATCAAACATTCAGATGG CTCGGTGGCAACCGACTCTAGTGGTAACCCGCTGCAGACCG TCCAAACGTTTGATATTCCGGTGTGGAACGACAAAGCCAATT TCTGGCCGCTGGGCGCACTGGATCACATCAAATCCGACTTTA ATTCAGGTCTGAAAAGCTGCTCTCTGATTTATCAGATCCCGT CTGATGCTCAACTGAAAAGTGGCGACACCGTGGCGTTCCAG GTTCTGGATGAAAACGGTAATGTGCTGGCGGATGACAACAC GGAAACCCAGCGCTACACGACCGTTTCTATCCAATAC |
| 38 | GCGAGCAAACCGGACATCAAAGTGGGCGACTACGTGAAAAT GGGTGTGTATAATAACGCAAGCATCCTGTGGCGCTGTGTGA GCATCGACAACAATGGCCCGCTGATGCTGGCCGATAAAATT GTTGACACGCTGGCGTATGATGCTAAAACCAACGACAATTC GAACAGCAAATCTCATAGTCGTTCCTACAAACGCGATGACT ACGGCAGCAACTATTGGAAAGATAGTAATATGCGCTCCTGG CTGAACTCAACCGCGGCCGAGGGTAAAGTGGATTGGCTGTG CGGCAATCCGCCGAAAGACGGTTACGTCAGCGGCGTGGGTG CATATAATGAAAAAGCTGGTTTTCTGAACGCGTTCTCAAAAT CGGAAATTGCAGCTATGAAAACGGTGACCCAGCGTAGCCTG |
| | GTTTCTCATCCGGAATATAATAAAGGCATTGTTGATGGTGAC GCGAACTCGGATCTGCTGTATTACACCGACATCAGCGAAGC AGTGGCTAACTACGATAGCTCTTATTTTGAAACCACGACCGA AAAAGTTTTCCTGCTGGATGTCAAACAGGCGAACGCCGTCT GGAAAAATCTGAAAGGCTATTACGTGGCTTACAACAATGAT GGTATGGCATGGCCGTATTGGCTGCGTACCCCGGTGACGGA TTGTAATCATGACATGCGCTATATTAGTTCCTCAGGCCAGGT TGGTCGTTACGCTCCGTGGTATTCTGATCTGGGCGTCCGTCC GGCGTTTTACCTGGACAGTGAATATTTCGTGACGACCAGCGG CTCTGGTAGTCAGTCGAGCCCGTACATTGGTTCCGCGCCGAA CAAACAAGAAGATGACTATACCATCTCAGAACCGGCGGAAG ATGCCAACCCGGACTGGAATGTTTCGACGGAACAGAGCATT CAACTGACCCTGGGCCCGTGGTACTCGAATGATGGTAAATA TAGCAACCCGACCATTCCGGTGTATACCATCCAGAAAACGC GCTCGGATACCGAAAACATGGTGGTTGTCGTGTGCGGCGAA GGTTATACCAAATCACAGCAAGGCAAATTTATCAATGATGTT AAACGTCTGTGGCAGGACGCTATGAAATATGAACCGTACCG TAGCTATGCGGATCGCTTTAATGTGTATGCACTGTGTACGGC TTCCGAATCAACCTTCGATAACGGCGGTTCTACCTTTTTCGA TGTGATCGTTGACAAATACAACTCTCCGGTTATCAGTAACAA TCTGCATGGCAGTCAGTGGAAAAATCACATTTTTGAACGCTG CATCGGTCCGGAATTCATTGAAAAAATCCATGATGCCCACAT TAAGAAAAAATGTGACCCGAACACCATCCCGTCGGGTAGCG AATACGAACCGTATTACTATGTGCATGATTATATTGCACAGT TTGCTATGGTTGTCAATACCAAATCCGACTTCGGCGGTGCAT ATAACAATCGCGAATACGGCTTTCACTATTTCATCTCTCCGA GTGATTCCTACCGTGCCTCTAAAACCTTTGCACATGAATTCG GCCACGGTCTGCTGGGCCTGGGTGATGAATACTCGAATGGTT ATCTGCTGGATGACAAAGAACTGAAAAGCCTGAACCTGTCT AGTGTGGAAGATCCGGAAAAAATTAAATGGCGTCAGCTGCT GGGCTTTCGCAATACGTACACCTGCCGTAACGCGTATGGTTC TAAAATGCTGGTTTCCTCATACGAATGTATCATGCGCGATAC CAACTATCAATTTTGCGAAGTCTGTCGCCTGCAGGGCTTCAA |
| | ACGTATGAGCCAACTGGTTAAAGATGTCGACCTGTATGTGG CCACGCCGGAAGTTAAAGAATACACCGGTGCATATAGTAAA CCGTCCGATTTTACGGACCTGGAAACCTCGAGCTACTACAAC TACACCTACAACCGTAACGATCGCCTGCTGAGTGGCAACTC AAAATCGCGTTTCAATACGAACATGAATGGCAAGAAAATTG AACTGCGCACCGTTATTCAGAACATCAGCGATAAAAACGCC CGTCAACTGAAATTCAAAATGTGGATCAAACATTCAGATGG CTCGGTGGCAACCGACTCTAGTGGTAACCCGCTGCAGACCG TCCAAACGTTTGATATTCCGGTGTGGAACGACAAAGCCAATT TCTGGCCGCTGGGCGCACTGGATCACATCAAATCCGACTTTA ATTCAGGTCTGAAAAGCTGCTCTCTGATTTATCAGATCCCGT CTGATGCTCAACTGAAAAGTGGCGACACCGTGGCGTTCCAG GTTCTGGATGAAAACGGTAATGTGCTGGCGGATGACAACAC GGAAACCCAGCGCTACACGACCGTTTCTATCCAATACAAATT CGAAGATGGCAGTGAAATCCCGAATACGGCGGGCGGTACCT TCACC |

In some embodiments, the nucleic acids provided herein comprises nucleic acid sequences as set forth in SEQ ID NO: 3, SEQ ID NO: 5, SEQ ID NO: 7, SEQ ID NO: 9, SEQ ID NO: 11, or SEQ ID NO: 13. In some embodiments, the nucleic acids provided herein is selected from the group consisting of the following nucleotide sequences: SEQ ID NO: 3, SEQ ID NO: 5, SEQ ID NO: 7, SEQ ID NO: 9, SEQ ID NO: 11, SEQ ID NO: 13 or a nucleotide sequence having at least 90%, at least 91%, at least 92%, at least 93%, at least 94%, at least 95%, at least 96%, at least 97%, at least 98%, at least 99% sequence identity thereto.

| **SEQ ID NO** | **Nucleotide Sequence** |
|---|---|
| 3 | CATATGGCGAGCAAACCGGACATCAAAGTGGGCGACTACGT GAAAATGGGTGTGTATAATAACGCAAGCATCCTGTGGCGCT GTGTGAGCATCGACAACAATGGCCCGCTGATGCTGGCCGAT AAAATTGTTGACACGCTGGCGTATGATGCTAAAACCAACGA CAATTCGAACAGCAAATCTCATAGTCGTTCCTACAAACGCG |
| | ATGACTACGGCAGCAACTATTGGAAAGATAGTAATATGCGC TCCTGGCTGAACTCAACCGCGGCCGAGGGTAAAGTGGATTG GCTGTGCGGCAATCCGCCGAAAGACGGTTACGTCAGCGGCG TGGGTGCATATAATGAAAAAGCTGGTTTTCTGAACGCGTTCT CAAAATCGGAAATTGCAGCTATGAAAACGGTGACCCAGCGT AGCCTGGTTTCTCATCCGGAATATAATAAAGGCATTGTTGAT GGTGACGCGAACTCGGATCTGCTGTATTACACCGACATCAG CGAAGCAGTGGCTAACTACGATAGCTCTTATTTTGAAACCAC GACCGAAAAAGTTTTCCTGCTGGATGTCAAACAGGCGAACG CCGTCTGGAAAAATCTGAAAGGCTATTACGTGGCTTACAAC AATGATGGTATGGCATGGCCGTATTGGCTGCGTACCCCGGTG ACGGATTGTAATCATGACATGCGCTATATTAGTTCCTCAGGC CAGGTTGGTCGTTACGCTCCGTGGTATTCTGATCTGGGCGTC CGTCCGGCGTTTTACCTGGACAGTGAATATTTCGTGACGACC AGCGGCTCTGGTAGTCAGTCGAGCCCGTACATTGGTTCCGCG CCGAACAAACAAGAAGATGACTATACCATCTCAGAACCGGC GGAAGATGCCAACCCGGACTGGAATGTTTCGACGGAACAGA GCATTCAACTGACCCTGGGCCCGTGGTACTCGAATGATGGTA AATATAGCAACCCGACCATTCCGGTGTATACCATCCAGAAA ACGCGCTCGGATACCGAAAACATGGTGGTTGTCGTGTGCGG CGAAGGTTATACCAAATCACAGCAAGGCAAATTTATCAATG ATGTTAAACGTCTGTGGCAGGACGCTATGAAATATGAACCG TACCGTAGCTATGCGGATCGCTTTAATGTGTATGCACTGTGT ACGGCTTCCGAATCAACCTTCGATAACGGCGGTTCTACCTTT TTCGATGTGATCGTTGACAAATACAACTCTCCGGTTATCAGT AACAATCTGCATGGCAGTCAGTGGAAAAATCACATTTTTGA ACGCTGCATCGGTCCGGAATTCATTGAAAAAATCCATGATG CCCACATTAAGAAAAAATGTGACCCGAACACCATCCCGTCG GGTAGCGAATACGAACCGTATTACTATGTGCATGATTATATT GCACAGTTTGCTATGGTTGTCAATACCAAATCCGACTTCGGC GGTGCATATAACAATCGCGAATACGGCTTTCACTATTTCATC TCTCCGAGTGATTCCTACCGTGCCTCTAAAACCTTTGCACAT GAATTCGGCCACGGTCTGCTGGGCCTGGGTGATGAATACTC |
| | GAATGGTTATCTGCTGGATGACAAAGAACTGAAAAGCCTGA ACCTGTCTAGTGTGGAAGATCCGGAAAAAATTAAATGGCGT CAGCTGCTGGGCTTTCGCAATACGTACACCTGCCGTAACGCG TATGGTTCTAAAATGCTGGTTTCCTCATACGAATGTATCATG CGCGATACCAACTATCAATTTTGCGAAGTCTGTCGCCTGCAG GGCTTCAAACGTATGAGCCAACTGGTTAAAGATGTCGACCT GTATGTGGCCACGCCGGAAGTTAAAGAATACACCGGTGCAT ATAGTAAACCGTCCGATTTTACGGACCTGGAAACCTCGAGCT ACTACAACTACACCTACAACCGTAACGATCGCCTGCTGAGT GGCAACTCAAAATCGCGTTTCAATACGAACATGAATGGCAA GAAAATTGAACTGCGCACCGTTATTCAGAACATCAGCGATA AAAACGCCCGTCAACTGAAATTCAAAATGTGGATCAAACAT TCAGATGGCTCGGTGGCAACCGACTCTAGTGGTAACCCGCT GCAGACCGTCCAAACGTTTGATATTCCGGTGTGGAACGACA AAGCCAATTTCTGGCCGCTGGGCGCACTGGATCACATCAAA TCCGACTTTAATTCAGGTCTGAAAAGCTGCTCTCTGATTTAT CAGATCCCGTCTGATGCTCAACTGAAAAGTGGCGACACCGT GGCGTTCCAGGTTCTGGGCGGCGGTGGATCCGAGCCCAAAT CTTGTGACAAAACTCACACATGCCCACCGTGCCCAGCACCTG AACTCCTGGGGGGACCGTCAGTCTTCCTCTTCCCCCCAAAAC CCAAGGACACCCTCATGATCTCCCGGACCCCTGAGGTCACAT GCGTGGTGGTGGACGTGAGCCACGAAGACCCTGAGGTCAAG TTCAACTGGTACGTGGACGGCGTGGAGGTGCATAATGCCAA GACAAAGCCGCGGGAGGAGCAGTACAACAGCACGTACCGG GTGGTCAGCGTCCTCACCGTCCTGCACCAGGACTGGCTGAAT GGCAAGGAGTACAAGTGCAAGGTCTCCAACAAAGCCCTCCC AGCCCCCATCGAGAAAACCATCTCCAAAGCCAAAGGGCAGC CCCGAGAACCACAGGTGTACACCCTGCCCCCATCCCGGGAT GAGCTGACCAAGAACCAGGTCAGCCTGACCTGCCTGGTCAA AGGCTTCTATCCCAGCGACATCGCCGTGGAGTGGGAGAGCA ATGGGCAGCCGGAGAACAACTACAAGACCACGCCTCCCGTG CTGGACTCCGACGGCTCCTTCTTCCTCTACAGCAAGCTCACC GTGGACAAGAGCAGGTGGCAGCAGGGGAACGTCTTCTCATG |
| | CTCCGTGATGCATGAGGCTCTGCACAACCACTACACGCAGA AGAGCCTCTCCCTGTCTCCGGGTAAACACCATCATCATCATC ATTAAGCGGCCGC |
| 5 | AAGCTTATGTATAGAATGCAGCTGCTGTCCTGTATTGCTCTG AGCCTGGCACTGGTTACAAACAGCGGTACCGCGAGCAAACC GGACATCAAAGTGGGCGACTACGTGAAAATGGGTGTGTATA ATAACGCAAGCATCCTGTGGCGCTGTGTGAGCATCGACAAC AATGGCCCGCTGATGCTGGCCGATAAAATTGTTGACACGCT GGCGTATGATGCTAAAACCAACGACAATTCGAACAGCAAAT CTCATAGTCGTTCCTACAAACGCGATGACTACGGCAGCAACT ATTGGAAAGATAGTAATATGCGCTCCTGGCTGAACTCAACC GCGGCCGAGGGTAAAGTGGATTGGCTGTGCGGCAATCCGCC GAAAGACGGTTACGTCAGCGGCGTGGGTGCATATAATGAAA AAGCTGGTTTTCTGAACGCGTTCTCAAAATCGGAAATTGCAG CTATGAAAACGGTGACCCAGCGTAGCCTGGTTTCTCATCCGG AATATAATAAAGGCATTGTTGATGGTGACGCGAACTCGGAT CTGCTGTATTACACCGACATCAGCGAAGCAGTGGCTAACTA CGATAGCTCTTATTTTGAAACCACGACCGAAAAAGTTTTCCT GCTGGATGTCAAACAGGCGAACGCCGTCTGGAAAAATCTGA AAGGCTATTACGTGGCTTACAACAATGATGGTATGGCATGG CCGTATTGGCTGCGTACCCCGGTGACGGATTGTAATCATGAC ATGCGCTATATTAGTTCCTCAGGCCAGGTTGGTCGTTACGCT CCGTGGTATTCTGATCTGGGCGTCCGTCCGGCGTTTTACCTG GACAGTGAATATTTCGTGACGACCAGCGGCTCTGGTAGTCA GTCGAGCCCGTACATTGGTTCCGCGCCGAACAAACAAGAAG ATGACTATACCATCTCAGAACCGGCGGAAGATGCCAACCCG GACTGGAATGTTTCGACGGAACAGAGCATTCAACTGACCCT GGGCCCGTGGTACTCGAATGATGGTAAATATAGCAACCCGA CCATTCCGGTGTATACCATCCAGAAAACGCGCTCGGATACC GAAAACATGGTGGTTGTCGTGTGCGGCGAAGGTTATACCAA ATCACAGCAAGGCAAATTTATCAATGATGTTAAACGTCTGTG GCAGGACGCTATGAAATATGAACCGTACCGTAGCTATGCGG ATCGCTTTAATGTGTATGCACTGTGTACGGCTTCCGAATCAA |
| | CCTTCGATAACGGCGGTTCTACCTTTTTCGATGTGATCGTTG ACAAATACAACTCTCCGGTTATCAGTAACAATCTGCATGGCA GTCAGTGGAAAAATCACATTTTTGAACGCTGCATCGGTCCGG AATTCATTGAAAAAATCCATGATGCCCACATTAAGAAAAAA TGTGACCCGAACACCATCCCGTCGGGTAGCGAATACGAACC GTATTACTATGTGCATGATTATATTGCACAGTTTGCTATGGT TGTCAATACCAAATCCGACTTCGGCGGTGCATATAACAATCG CGAATACGGCTTTCACTATTTCATCTCTCCGAGTGATTCCTA CCGTGCCTCTAAAACCTTTGCACATGAATTCGGCCACGGTCT GCTGGGCCTGGGTGATGAATACTCGAATGGTTATCTGCTGGA TGACAAAGAACTGAAAAGCCTGAACCTGTCTAGTGTGGAAG ATCCGGAAAAAATTAAATGGCGTCAGCTGCTGGGCTTTCGC AATACGTACACCTGCCGTAACGCGTATGGTTCTAAAATGCTG GTTTCCTCATACGAATGTATCATGCGCGATACCAACTATCAA TTTTGCGAAGTCTGTCGCCTGCAGGGCTTCAAACGTATGAGC CAACTGGTTAAAGATGTCGACCTGTATGTGGCCACGCCGGA AGTTAAAGAATACACCGGTGCATATAGTAAACCGTCCGATT TTACGGACCTGGAAACCTCGAGCTACTACAACTACACCTAC AACCGTAACGATCGCCTGCTGAGTGGCAACTCAAAATCGCG TTTCAATACGAACATGAATGGCAAGAAAATTGAACTGCGCA CCGTTATTCAGAACATCAGCGATAAAAACGCCCGTCAACTG AAATTCAAAATGTGGATCAAACATTCAGATGGCTCGGTGGC AACCGACTCTAGTGGTAACCCGCTGCAGACCGTCCAAACGT TTGATATTCCGGTGTGGAACGACAAAGCCAATTTCTGGCCGC TGGGCGCACTGGATCACATCAAATCCGACTTTAATTCAGGTC TGAAAAGCTGCTCTCTGATTTATCAGATCCCGTCTGATGCTC AACTGAAAAGTGGCGACACCGTGGCGTTCCAGGTTCTGGGC GGCGGTGGATCCGAACCTAAGAGTTGCGATAAAACCCACAC TTGCCCTCCCTGTCCGGCCCCCGAACTGCTCGGCGGACCCTC AGTCTTCCTGTTCCCCCCAAAGCCAAAGGACACATTGATGAT CAGCAGGACTCCTGAAGTGACATGCGTGGTCGTAGACGTGT CACACGAGGACCCGGAGGTGAAGTTCAACTGGTACGTGGAC GGAGTGGAGGTGCATAATGCCAAAACAAAGCCCAGAGAAG |
| | AGCAGTATAACAGTACCTACAGAGTGGTGTCAGTGCTGACC GTGCTTCATCAGGATTGGCTGAACGGGAAGGAGTACAAGTG TAAGGTGAGTAATAAGGCTCTGCCTGCCCCAATTGAGAAGA CAATCTCTAAAGCCAAGGGGCAGCCCCGGGAACCCCAAGTG TATACACTCCCACCGTCCCGCGATGAACTGACAAAAAACCA GGTATCACTCACTTGTCTGGTAAAGGGCTTCTATCCATCTGA CATTGCCGTGGAGTGGGAATCAAACGGCCAACCCGAGAATA ATTATAAGACAACCCCGCCCGTGCTGGATTCCGACGGATCTT TTTTCCTGTATAGCAAATTGACTGTCGACAAAAGTCGGTGGC AGCAGGGCAATGTGTTTTCTTGCAGCGTCATGCATGAGGCGC TGCACAACCACTATACTCAGAAGTCATTGAGCTTGAGCCCTG GTAAGCACCATCATCACCATCACCATCATTAGGCGGCCGC |
| 7 | CATATGGCGAGCAAACCGGACATCAAAGTGGGCGACTACGT GAAAATGGGTGTGTATAATAACGCAAGCATCCTGTGGCGCT GTGTGAGCATCGACAACAATGGCCCGCTGATGCTGGCCGAT AAAATTGTTGACACGCTGGCGTATGATGCTAAAACCAACGA CAATTCGAACAGCAAATCTCATAGTCGTTCCTACAAACGCG ATGACTACGGCAGCAACTATTGGAAAGATAGTAATATGCGC TCCTGGCTGAACTCAACCGCGGCCGAGGGTAAAGTGGATTG GCTGTGCGGCAATCCGCCGAAAGACGGTTACGTCAGCGGCG TGGGTGCATATAATGAAAAAGCTGGTTTTCTGAACGCGTTCT CAAAATCGGAAATTGCAGCTATGAAAACGGTGACCCAGCGT AGCCTGGTTTCTCATCCGGAATATAATAAAGGCATTGTTGAT GGTGACGCGAACTCGGATCTGCTGTATTACACCGACATCAG CGAAGCAGTGGCTAACTACGATAGCTCTTATTTTGAAACCAC GACCGAAAAAGTTTTCCTGCTGGATGTCAAACAGGCGAACG CCGTCTGGAAAAATCTGAAAGGCTATTACGTGGCTTACAAC AATGATGGTATGGCATGGCCGTATTGGCTGCGTACCCCGGTG ACGGATTGTAATCATGACATGCGCTATATTAGTTCCTCAGGC CAGGTTGGTCGTTACGCTCCGTGGTATTCTGATCTGGGCGTC CGTCCGGCGTTTTACCTGGACAGTGAATATTTCGTGACGACC AGCGGCTCTGGTAGTCAGTCGAGCCCGTACATTGGTTCCGCG CCGAACAAACAAGAAGATGACTATACCATCTCAGAACCGGC |
| | GGAAGATGCCAACCCGGACTGGAATGTTTCGACGGAACAGA GCATTCAACTGACCCTGGGCCCGTGGTACTCGAATGATGGTA AATATAGCAACCCGACCATTCCGGTGTATACCATCCAGAAA ACGCGCTCGGATACCGAAAACATGGTGGTTGTCGTGTGCGG CGAAGGTTATACCAAATCACAGCAAGGCAAATTTATCAATG ATGTTAAACGTCTGTGGCAGGACGCTATGAAATATGAACCG TACCGTAGCTATGCGGATCGCTTTAATGTGTATGCACTGTGT ACGGCTTCCGAATCAACCTTCGATAACGGCGGTTCTACCTTT TTCGATGTGATCGTTGACAAATACAACTCTCCGGTTATCAGT AACAATCTGCATGGCAGTCAGTGGAAAAATCACATTTTTGA ACGCTGCATCGGTCCGGAATTCATTGAAAAAATCCATGATG CCCACATTAAGAAAAAATGTGACCCGAACACCATCCCGTCG GGTAGCGAATACGAACCGTATTACTATGTGCATGATTATATT GCACAGTTTGCTATGGTTGTCAATACCAAATCCGACTTCGGC GGTGCATATAACAATCGCGAATACGGCTTTCACTATTTCATC TCTCCGAGTGATTCCTACCGTGCCTCTAAAACCTTTGCACAT GAATTCGGCCACGGTCTGCTGGGCCTGGGTGATGAATACTC GAATGGTTATCTGCTGGATGACAAAGAACTGAAAAGCCTGA ACCTGTCTAGTGTGGAAGATCCGGAAAAAATTAAATGGCGT CAGCTGCTGGGCTTTCGCAATACGTACACCTGCCGTAACGCG TATGGTTCTAAAATGCTGGTTTCCTCATACGAATGTATCATG CGCGATACCAACTATCAATTTTGCGAAGTCTGTCGCCTGCAG GGCTTCAAACGTATGAGCCAACTGGTTAAAGATGTCGACCT GTATGTGGCCACGCCGGAAGTTAAAGAATACACCGGTGCAT ATAGTAAACCGTCCGATTTTACGGACCTGGAAACCTCGAGCT ACTACAACTACACCTACAACCGTAACGATCGCCTGCTGAGT GGCAACTCAAAATCGCGTTTCAATACGAACATGAATGGCAA GAAAATTGAACTGCGCACCGTTATTCAGAACATCAGCGATA AAAACGCCCGTCAACTGAAATTCAAAATGTGGATCAAACAT TCAGATGGCTCGGTGGCAACCGACTCTAGTGGTAACCCGCT GCAGACCGTCCAAACGTTTGATATTCCGGTGTGGAACGACA AAGCCAATTTCTGGCCGCTGGGCGCACTGGATCACATCAAA TCCGACTTTAATTCAGGTCTGAAAAGCTGCTCTCTGATTTAT |
| | CAGATCCCGTCTGATGCTCAACTGAAAAGTGGCGACACCGT GGCGTTCCAGGTTCTGGATGAAAACGGTAATGTGGGCGGCG GTGGATCCCACCATCATCACCACCATCATCATCACCACACAT GCCCACCGTGCCCAGCACCTGAACTCCTGGGGGGACCGTCA GTCTTCCTCTTCCCCCCAAAACCCAAGGACACCCTCATGATC TCCCGGACCCCTGAGGTCACATGCGTGGTGGTGGACGTGAG CCACGAAGACCCTGAGGTCAAGTTCAACTGGTACGTGGACG GCGTGGAGGTGCATAATGCCAAGACAAAGCCGCGGGAGGA GCAGTACAACAGCACGTACCGGGTGGTCAGCGTCCTCACCG TCCTGCACCAGGACTGGCTGAATGGCAAGGAGTACAAGTGC AAGGTCTCCAACAAAGCCCTCCCAGCCCCCATCGAGAAAAC CATCTCCAAAGCCAAAGGGCAGCCCCGAGAACCACAGGTGT ACACCCTGCCCCCATCCCGGGATGAGCTGACCAAGAACCAG GTCAGCCTGACCTGCCTGGTCAAAGGCTTCTATCCCAGCGAC ATCGCCGTGGAGTGGGAGAGCAATGGGCAGCCGGAGAACA ACTACAAGACCACGCCTCCCGTGCTGGACTCCGACGGCTCCT TCTTCCTCTACAGCAAGCTCACCGTGGACAAGAGCAGGTGG CAGCAGGGGAACGTCTTCTCATGCTCCGTGATGCATGAGGCT CTGCACAACCACTACACGCAGAAGAGCCTCTCCCTGTCTCCG GGTAAATAAGCGGCCGC |
| 9 | CATATGGCGAGCAAACCGGACATCAAAGTGGGCGACTACGT GAAAATGGGTGTGTATAATAACGCAAGCATCCTGTGGCGCT GTGTGAGCATCGACAACAATGGCCCGCTGATGCTGGCCGAT AAAATTGTTGACACGCTGGCGTATGATGCTAAAACCAACGA CAATTCGAACAGCAAATCTCATAGTCGTTCCTACAAACGCG ATGACTACGGCAGCAACTATTGGAAAGATAGTAATATGCGC TCCTGGCTGAACTCAACCGCGGCCGAGGGTAAAGTGGATTG GCTGTGCGGCAATCCGCCGAAAGACGGTTACGTCAGCGGCG TGGGTGCATATAATGAAAAAGCTGGTTTTCTGAACGCGTTCT CAAAATCGGAAATTGCAGCTATGAAAACGGTGACCCAGCGT AGCCTGGTTTCTCATCCGGAATATAATAAAGGCATTGTTGAT GGTGACGCGAACTCGGATCTGCTGTATTACACCGACATCAG CGAAGCAGTGGCTAACTACGATAGCTCTTATTTTGAAACCAC |
| | GACCGAAAAAGTTTTCCTGCTGGATGTCAAACAGGCGAACG CCGTCTGGAAAAATCTGAAAGGCTATTACGTGGCTTACAAC AATGATGGTATGGCATGGCCGTATTGGCTGCGTACCCCGGTG ACGGATTGTAATCATGACATGCGCTATATTAGTTCCTCAGGC CAGGTTGGTCGTTACGCTCCGTGGTATTCTGATCTGGGCGTC CGTCCGGCGTTTTACCTGGACAGTGAATATTTCGTGACGACC AGCGGCTCTGGTAGTCAGTCGAGCCCGTACATTGGTTCCGCG CCGAACAAACAAGAAGATGACTATACCATCTCAGAACCGGC GGAAGATGCCAACCCGGACTGGAATGTTTCGACGGAACAGA GCATTCAACTGACCCTGGGCCCGTGGTACTCGAATGATGGTA AATATAGCAACCCGACCATTCCGGTGTATACCATCCAGAAA ACGCGCTCGGATACCGAAAACATGGTGGTTGTCGTGTGCGG CGAAGGTTATACCAAATCACAGCAAGGCAAATTTATCAATG ATGTTAAACGTCTGTGGCAGGACGCTATGAAATATGAACCG TACCGTAGCTATGCGGATCGCTTTAATGTGTATGCACTGTGT ACGGCTTCCGAATCAACCTTCGATAACGGCGGTTCTACCTTT TTCGATGTGATCGTTGACAAATACAACTCTCCGGTTATCAGT AACAATCTGCATGGCAGTCAGTGGAAAAATCACATTTTTGA ACGCTGCATCGGTCCGGAATTCATTGAAAAAATCCATGATG CCCACATTAAGAAAAAATGTGACCCGAACACCATCCCGTCG GGTAGCGAATACGAACCGTATTACTATGTGCATGATTATATT GCACAGTTTGCTATGGTTGTCAATACCAAATCCGACTTCGGC GGTGCATATAACAATCGCGAATACGGCTTTCACTATTTCATC TCTCCGAGTGATTCCTACCGTGCCTCTAAAACCTTTGCACAT GAATTCGGCCACGGTCTGCTGGGCCTGGGTGATGAATACTC GAATGGTTATCTGCTGGATGACAAAGAACTGAAAAGCCTGA ACCTGTCTAGTGTGGAAGATCCGGAAAAAATTAAATGGCGT CAGCTGCTGGGCTTTCGCAATACGTACACCTGCCGTAACGCG TATGGTTCTAAAATGCTGGTTTCCTCATACGAATGTATCATG CGCGATACCAACTATCAATTTTGCGAAGTCTGTCGCCTGCAG GGCTTCAAACGTATGAGCCAACTGGTTAAAGATGTCGACCT GTATGTGGCCACGCCGGAAGTTAAAGAATACACCGGTGCAT ATAGTAAACCGTCCGATTTTACGGACCTGGAAACCTCGAGCT |
| | ACTACAACTACACCTACAACCGTAACGATCGCCTGCTGAGT GGCAACTCAAAATCGCGTTTCAATACGAACATGAATGGCAA GAAAATTGAACTGCGCACCGTTATTCAGAACATCAGCGATA AAAACGCCCGTCAACTGAAATTCAAAATGTGGATCAAACAT TCAGATGGCTCGGTGGCAACCGACTCTAGTGGTAACCCGCT GCAGACCGTCCAAACGTTTGATATTCCGGTGTGGAACGACA AAGCCAATTTCTGGCCGCTGGGCGCACTGGATCACATCAAA TCCGACTTTAATTCAGGTCTGAAAAGCTGCTCTCTGATTTAT CAGATCCCGTCTGATGCTCAACTGAAAAGTGGCGACACCGT GGCGTTCCAGGTTCTGGATGAAAACGGTAATGTGCTGGCGG ATGACAACACGGAAACCCAGGGCGGCGGTGGATCCCACCAT CATCACCACCATCATCATCACCACACATGCCCACCGTGCCCA GCACCTGAACTCCTGGGGGGACCGTCAGTCTTCCTCTTCCCC CCAAAACCCAAGGACACCCTCATGATCTCCCGGACCCCTGA GGTCACATGCGTGGTGGTGGACGTGAGCCACGAAGACCCTG AGGTCAAGTTCAACTGGTACGTGGACGGCGTGGAGGTGCAT AATGCCAAGACAAAGCCGCGGGAGGAGCAGTACAACAGCA CGTACCGGGTGGTCAGCGTCCTCACCGTCCTGCACCAGGACT GGCTGAATGGCAAGGAGTACAAGTGCAAGGTCTCCAACAAA GCCCTCCCAGCCCCCATCGAGAAAACCATCTCCAAAGCCAA AGGGCAGCCCCGAGAACCACAGGTGTACACCCTGCCCCCAT CCCGGGATGAGCTGACCAAGAACCAGGTCAGCCTGACCTGC CTGGTCAAAGGCTTCTATCCCAGCGACATCGCCGTGGAGTG GGAGAGCAATGGGCAGCCGGAGAACAACTACAAGACCACG CCTCCCGTGCTGGACTCCGACGGCTCCTTCTTCCTCTACAGC AAGCTCACCGTGGACAAGAGCAGGTGGCAGCAGGGGAACG TCTTCTCATGCTCCGTGATGCATGAGGCTCTGCACAACCACT ACACGCAGAAGAGCCTCTCCCTGTCTCCGGGTAAATAAGCG GCCGC CATATGGCGAGCAAACCGGACATCAAAGTGGGCGACTACGT GAAAATGGGTGTGTATAATAACGCAAGCATCCTGTGGCGCT GTGTGAGCATCGACAACAATGGCCCGCTGATGCTGGCCGAT |
| 11 | CATATGGCGAGCAAACCGGACATCAAAGTGGGCGACTACGT GAAAATGGGTGTGTATAATAACGCAAGCATCCTGTGGCGCT GTGTGAGCATCGACAACAATGGCCCGCTGATGCTGGCCGAT |
| | AAAATTGTTGACACGCTGGCGTATGATGCTAAAACCAACGA CAATTCGAACAGCAAATCTCATAGTCGTTCCTACAAACGCG ATGACTACGGCAGCAACTATTGGAAAGATAGTAATATGCGC TCCTGGCTGAACTCAACCGCGGCCGAGGGTAAAGTGGATTG GCTGTGCGGCAATCCGCCGAAAGACGGTTACGTCAGCGGCG TGGGTGCATATAATGAAAAAGCTGGTTTTCTGAACGCGTTCT CAAAATCGGAAATTGCAGCTATGAAAACGGTGACCCAGCGT AGCCTGGTTTCTCATCCGGAATATAATAAAGGCATTGTTGAT GGTGACGCGAACTCGGATCTGCTGTATTACACCGACATCAG CGAAGCAGTGGCTAACTACGATAGCTCTTATTTTGAAACCAC GACCGAAAAAGTTTTCCTGCTGGATGTCAAACAGGCGAACG CCGTCTGGAAAAATCTGAAAGGCTATTACGTGGCTTACAAC AATGATGGTATGGCATGGCCGTATTGGCTGCGTACCCCGGTG ACGGATTGTAATCATGACATGCGCTATATTAGTTCCTCAGGC CAGGTTGGTCGTTACGCTCCGTGGTATTCTGATCTGGGCGTC CGTCCGGCGTTTTACCTGGACAGTGAATATTTCGTGACGACC AGCGGCTCTGGTAGTCAGTCGAGCCCGTACATTGGTTCCGCG CCGAACAAACAAGAAGATGACTATACCATCTCAGAACCGGC GGAAGATGCCAACCCGGACTGGAATGTTTCGACGGAACAGA GCATTCAACTGACCCTGGGCCCGTGGTACTCGAATGATGGTA AATATAGCAACCCGACCATTCCGGTGTATACCATCCAGAAA ACGCGCTCGGATACCGAAAACATGGTGGTTGTCGTGTGCGG CGAAGGTTATACCAAATCACAGCAAGGCAAATTTATCAATG ATGTTAAACGTCTGTGGCAGGACGCTATGAAATATGAACCG TACCGTAGCTATGCGGATCGCTTTAATGTGTATGCACTGTGT ACGGCTTCCGAATCAACCTTCGATAACGGCGGTTCTACCTTT TTCGATGTGATCGTTGACAAATACAACTCTCCGGTTATCAGT AACAATCTGCATGGCAGTCAGTGGAAAAATCACATTTTTGA ACGCTGCATCGGTCCGGAATTCATTGAAAAAATCCATGATG |
| | CCCACATTAAGAAAAAATGTGACCCGAACACCATCCCGTCG GGTAGCGAATACGAACCGTATTACTATGTGCATGATTATATT GCACAGTTTGCTATGGTTGTCAATACCAAATCCGACTTCGGC GGTGCATATAACAATCGCGAATACGGCTTTCACTATTTCATC TCTCCGAGTGATTCCTACCGTGCCTCTAAAACCTTTGCACAT GAATTCGGCCACGGTCTGCTGGGCCTGGGTGATGAATACTC GAATGGTTATCTGCTGGATGACAAAGAACTGAAAAGCCTGA ACCTGTCTAGTGTGGAAGATCCGGAAAAAATTAAATGGCGT CAGCTGCTGGGCTTTCGCAATACGTACACCTGCCGTAACGCG TATGGTTCTAAAATGCTGGTTTCCTCATACGAATGTATCATG CGCGATACCAACTATCAATTTTGCGAAGTCTGTCGCCTGCAG GGCTTCAAACGTATGAGCCAACTGGTTAAAGATGTCGACCT GTATGTGGCCACGCCGGAAGTTAAAGAATACACCGGTGCAT ATAGTAAACCGTCCGATTTTACGGACCTGGAAACCTCGAGCT ACTACAACTACACCTACAACCGTAACGATCGCCTGCTGAGT GGCAACTCAAAATCGCGTTTCAATACGAACATGAATGGCAA GAAAATTGAACTGCGCACCGTTATTCAGAACATCAGCGATA AAAACGCCCGTCAACTGAAATTCAAAATGTGGATCAAACAT TCAGATGGCTCGGTGGCAACCGACTCTAGTGGTAACCCGCT GCAGACCGTCCAAACGTTTGATATTCCGGTGTGGAACGACA AAGCCAATTTCTGGCCGCTGGGCGCACTGGATCACATCAAA TCCGACTTTAATTCAGGTCTGAAAAGCTGCTCTCTGATTTAT CAGATCCCGTCTGATGCTCAACTGAAAAGTGGCGACACCGT GGCGTTCCAGGTTCTGGATGAAAACGGTAATGTGCTGGCGG ATGACAACACGGAAACCCAGCGCTACACGACCGTTTCTATC CAATACGGCGGCGGTGGATCCCACCATCATCACCACCATCA TCATCACCACACATGCCCACCGTGCCCAGCACCTGAACTCCT GGGGGGACCGTCAGTCTTCCTCTTCCCCCCAAAACCCAAGG ACACCCTCATGATCTCCCGGACCCCTGAGGTCACATGCGTGG |
| | TGGTGGACGTGAGCCACGAAGACCCTGAGGTCAAGTTCAAC TGGTACGTGGACGGCGTGGAGGTGCATAATGCCAAGACAAA GCCGCGGGAGGAGCAGTACAACAGCACGTACCGGGTGGTCA GCGTCCTCACCGTCCTGCACCAGGACTGGCTGAATGGCAAG GAGTACAAGTGCAAGGTCTCCAACAAAGCCCTCCCAGCCCC CATCGAGAAAACCATCTCCAAAGCCAAAGGGCAGCCCCGAG AACCACAGGTGTACACCCTGCCCCCATCCCGGGATGAGCTG ACCAAGAACCAGGTCAGCCTGACCTGCCTGGTCAAAGGCTT CTATCCCAGCGACATCGCCGTGGAGTGGGAGAGCAATGGGC AGCCGGAGAACAACTACAAGACCACGCCTCCCGTGCTGGAC TCCGACGGCTCCTTCTTCCTCTACAGCAAGCTCACCGTGGAC AAGAGCAGGTGGCAGCAGGGGAACGTCTTCTCATGCTCCGT GATGCATGAGGCTCTGCACAACCACTACACGCAGAAGAGCC TCTCCCTGTCTCCGGGTAAATAAGCGGCCGC |
| 13 | CATATGGCGAGCAAACCGGACATCAAAGTGGGCGACTACGT GAAAATGGGTGTGTATAATAACGCAAGCATCCTGTGGCGCT GTGTGAGCATCGACAACAATGGCCCGCTGATGCTGGCCGAT AAAATTGTTGACACGCTGGCGTATGATGCTAAAACCAACGA CAATTCGAACAGCAAATCTCATAGTCGTTCCTACAAACGCG ATGACTACGGCAGCAACTATTGGAAAGATAGTAATATGCGC TCCTGGCTGAACTCAACCGCGGCCGAGGGTAAAGTGGATTG GCTGTGCGGCAATCCGCCGAAAGACGGTTACGTCAGCGGCG TGGGTGCATATAATGAAAAAGCTGGTTTTCTGAACGCGTTCT CAAAATCGGAAATTGCAGCTATGAAAACGGTGACCCAGCGT AGCCTGGTTTCTCATCCGGAATATAATAAAGGCATTGTTGAT GGTGACGCGAACTCGGATCTGCTGTATTACACCGACATCAG CGAAGCAGTGGCTAACTACGATAGCTCTTATTTTGAAACCAC GACCGAAAAAGTTTTCCTGCTGGATGTCAAACAGGCGAACG CCGTCTGGAAAAATCTGAAAGGCTATTACGTGGCTTACAAC AATGATGGTATGGCATGGCCGTATTGGCTGCGTACCCCGGTG ACGGATTGTAATCATGACATGCGCTATATTAGTTCCTCAGGC |
| | CAGGTTGGTCGTTACGCTCCGTGGTATTCTGATCTGGGCGTC CGTCCGGCGTTTTACCTGGACAGTGAATATTTCGTGACGACC AGCGGCTCTGGTAGTCAGTCGAGCCCGTACATTGGTTCCGCG CCGAACAAACAAGAAGATGACTATACCATCTCAGAACCGGC GGAAGATGCCAACCCGGACTGGAATGTTTCGACGGAACAGA GCATTCAACTGACCCTGGGCCCGTGGTACTCGAATGATGGTA AATATAGCAACCCGACCATTCCGGTGTATACCATCCAGAAA ACGCGCTCGGATACCGAAAACATGGTGGTTGTCGTGTGCGG CGAAGGTTATACCAAATCACAGCAAGGCAAATTTATCAATG ATGTTAAACGTCTGTGGCAGGACGCTATGAAATATGAACCG TACCGTAGCTATGCGGATCGCTTTAATGTGTATGCACTGTGT ACGGCTTCCGAATCAACCTTCGATAACGGCGGTTCTACCTTT TTCGATGTGATCGTTGACAAATACAACTCTCCGGTTATCAGT AACAATCTGCATGGCAGTCAGTGGAAAAATCACATTTTTGA ACGCTGCATCGGTCCGGAATTCATTGAAAAAATCCATGATG CCCACATTAAGAAAAAATGTGACCCGAACACCATCCCGTCG GGTAGCGAATACGAACCGTATTACTATGTGCATGATTATATT GCACAGTTTGCTATGGTTGTCAATACCAAATCCGACTTCGGC GGTGCATATAACAATCGCGAATACGGCTTTCACTATTTCATC TCTCCGAGTGATTCCTACCGTGCCTCTAAAACCTTTGCACAT GAATTCGGCCACGGTCTGCTGGGCCTGGGTGATGAATACTC GAATGGTTATCTGCTGGATGACAAAGAACTGAAAAGCCTGA ACCTGTCTAGTGTGGAAGATCCGGAAAAAATTAAATGGCGT CAGCTGCTGGGCTTTCGCAATACGTACACCTGCCGTAACGCG TATGGTTCTAAAATGCTGGTTTCCTCATACGAATGTATCATG CGCGATACCAACTATCAATTTTGCGAAGTCTGTCGCCTGCAG GGCTTCAAACGTATGAGCCAACTGGTTAAAGATGTCGACCT GTATGTGGCCACGCCGGAAGTTAAAGAATACACCGGTGCAT ATAGTAAACCGTCCGATTTTACGGACCTGGAAACCTCGAGCT ACTACAACTACACCTACAACCGTAACGATCGCCTGCTGAGT GGCAACTCAAAATCGCGTTTCAATACGAACATGAATGGCAA GAAAATTGAACTGCGCACCGTTATTCAGAACATCAGCGATA AAAACGCCCGTCAACTGAAATTCAAAATGTGGATCAAACAT |
| | TCAGATGGCTCGGTGGCAACCGACTCTAGTGGTAACCCGCT GCAGACCGTCCAAACGTTTGATATTCCGGTGTGGAACGACA AAGCCAATTTCTGGCCGCTGGGCGCACTGGATCACATCAAA TCCGACTTTAATTCAGGTCTGAAAAGCTGCTCTCTGATTTAT CAGATCCCGTCTGATGCTCAACTGAAAAGTGGCGACACCGT GGCGTTCCAGGTTCTGGATGAAAACGGTAATGTGCTGGCGG ATGACAACACGGAAACCCAGCGCTACACGACCGTTTCTATC CAATACAAATTCGAAGATGGCAGTGAAATCCCGAATACGGC GGGCGGTACCTTCACCGGCGGCGGTGGATCCCACCATCATC ACCACCATCATCATCACCACACATGCCCACCGTGCCCAGCAC CTGAACTCCTGGGGGGACCGTCAGTCTTCCTCTTCCCCCCAA AACCCAAGGACACCCTCATGATCTCCCGGACCCCTGAGGTC ACATGCGTGGTGGTGGACGTGAGCCACGAAGACCCTGAGGT CAAGTTCAACTGGTACGTGGACGGCGTGGAGGTGCATAATG CCAAGACAAAGCCGCGGGAGGAGCAGTACAACAGCACGTA CCGGGTGGTCAGCGTCCTCACCGTCCTGCACCAGGACTGGCT GAATGGCAAGGAGTACAAGTGCAAGGTCTCCAACAAAGCCC TCCCAGCCCCCATCGAGAAAACCATCTCCAAAGCCAAAGGG CAGCCCCGAGAACCACAGGTGTACACCCTGCCCCCATCCCG GGATGAGCTGACCAAGAACCAGGTCAGCCTGACCTGCCTGG TCAAAGGCTTCTATCCCAGCGACATCGCCGTGGAGTGGGAG AGCAATGGGCAGCCGGAGAACAACTACAAGACCACGCCTCC CGTGCTGGACTCCGACGGCTCCTTCTTCCTCTACAGCAAGCT CACCGTGGACAAGAGCAGGTGGCAGCAGGGGAACGTCTTCT CATGCTCCGTGATGCATGAGGCTCTGCACAACCACTACACGC AGAAGAGCCTCTCCCTGTCTCCGGGTAAATAAGCGGCCGC |

### Vector and Cell

In another aspect, the present disclosure provides a vector comprising the nucleic acid encoding the truncated form of IgA protease described herein or comprising the nucleic acid encoding the fusion protein described herein.

The isolated polynucleotide that encodes the truncated form of IgA protease or the fusion protein described herein can be inserted into vector for further cloning (amplification of the DNA) or for expression, using recombinant techniques known in the art. Many vectors are available. The vector components generally include, but are not limited to, one or more of the followings: a signal sequence, an origin of replication, one or more marker genes, an enhancer element, a promoter (e.g., SV40, CMV, EF-1α), a transcription stop sequence.

In certain embodiments, the nucleic acid provided herein encodes the truncated form of IgA protease or the fusion protein, with at least one promoter (e.g., SV40, CMV, EF-1α) operably linked to the nucleic acid sequence, and at least one selection marker. Examples of vectors include, but are not limited to, retrovirus (including lentivirus), adenovirus, adeno-associated virus, herpesvirus (*e.g*., herpes simplex virus), poxvirus, baculovirus, papillomavirus, papovavirus (*e.g*., SV40), lambda phage, and M13 phage, plasmid pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM., pCDM8, pCDNA1.1/amp, pcDNA3.1, pRc/RSV, PCR 2.1, pEF-1, pFB, pSG5, pXT1, pCDEF3, pSVSPORT, pEF-Bos, etc.

Vectors comprising the nucleic acid sequence encoding the truncated form of IgA protease or the fusion protein described herein can be introduced to a host cell for cloning or gene expression. Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, *Enterobacteriaceae* such as *Escherichia* (*e.g*., *E. coli), Enterobacter, Erwinia, Klebsiella, Proteus, Salmonella* (*e.g*., *Salmonella typhimurium*), *Serratia* (*e.g*., *Serratia marcescans*), and *Shigella*, as well as *Bacilli* such as *B. subtilis* and *B. licheniformis*, *Pseudomonas* such as *P. aeruginosa*, and *Streptomyces.* In some embodiments, the cell is a *E.coli* cell.

In addition to prokaryotes, eukaryotic microbes such as filamentous fungi or yeast are also suitable cloning or expression hosts for the vectors encoding the truncated form of IgA protease or the fusion protein described herein. *Saccharomyces cerevisiae*, or common baker's yeast, is the most commonly used among lower eukaryotic host microorganisms. However, a number of other genera, species, and strains are commonly available and useful herein, such as *Schizosaccharomyces pombe*; *Kluyveromyces* hosts such as, *e.g. K. lactis*, *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans*, and K. *marxianus*; *yarrowia* (EP 402,226); *Pichia pastoris* (EP 183,070); *Candida*; *Trichoderma reesia* (EP 244,234); *Neurospora crassa*; *Schwanniomyces* such as *Schwanniomyces occidentalis*; and filamentous fungi such as, *e.g. Neurospora*, *Penicillium*, *Tolypocladium*, and *Aspergillus* hosts such as *A. nidulans* and *A. niger.* In some embodiments, the eukaryotic cell is a mammalian cell. In some embodiments, the mammalian cell is a human cell or a Chinese hamster ovary (CHO) cell. In some embodiments, the mammalian cell is a human embryonic kidney cell 293 (HEK293 cell).

### Pharmaceutical Composition

In another aspect, the present disclosure provides a pharmaceutical composition comprising the truncated form of IgA protease described herein, comprising the fusion protein described herein, comprising the nucleic acid described herein, comprising the vector described herein or comprising the cell described herein, and a pharmaceutically acceptable carrier.

Pharmaceutical acceptable carriers for use in the pharmaceutical compositions disclosed herein may include, for example, pharmaceutically acceptable liquid, gel, or solid carriers, aqueous vehicles, nonaqueous vehicles, antimicrobial agents, isotonic agents, buffers, antioxidants, anesthetics, suspending/dispending agents, sequestering or chelating agents, diluents, adjuvants, excipients, or non-toxic auxiliary substances, other components known in the art, or various combinations thereof.

Suitable components may include, for example, antioxidants, fillers, binders, disintegrants, buffers, preservatives, lubricants, flavorings, thickeners, coloring agents, emulsifiers or stabilizers such as sugars and cyclodextrins. Suitable antioxidants may include, for example, methionine, ascorbic acid, EDTA, sodium thiosulfate, platinum, catalase, citric acid, cysteine, thioglycerol, thioglycolic acid, thiosorbitol, butylated hydroxanisol, butylated hydroxytoluene, and/or propyl gallate. As disclosed herein, inclusion of one or more antioxidants such as methionine in a composition comprising a truncated form of IgA protease and fusion protein as provided herein decreases oxidation of the truncated form of IgA protease and fusion protein. Further provided are methods for preventing oxidation of, extending the shelf-life of, and/or improving the efficacy of a truncated form of IgA protease and fusion protein as provided herein by mixing the truncated form of IgA protease and fusion protein with one or more antioxidants such as methionine.

To further illustrate, pharmaceutical acceptable carriers may include, for example, aqueous vehicles such as sodium chloride injection, Ringer's injection, isotonic dextrose injection, sterile water injection, or dextrose and lactated Ringer's injection, nonaqueous vehicles such as fixed oils of vegetable origin, cottonseed oil, corn oil, sesame oil, or peanut oil, antimicrobial agents at bacteriostatic or fungistatic concentrations, isotonic agents such as sodium chloride or dextrose, buffers such as phosphate or citrate buffers, antioxidants such as sodium bisulfate, local anesthetics such as procaine hydrochloride, suspending and dispersing agents such as sodium carboxymethylcelluose, hydroxypropyl methylcellulose, or polyvinylpyrrolidone, emulsifying agents such as Polysorbate 80 (TWEEN-80), sequestering or chelating agents such as EDTA (ethylenediaminetetraacetic acid) or EGTA (ethylene glycol tetraacetic acid), ethyl alcohol, polyethylene glycol, propylene glycol, sodium hydroxide, hydrochloric acid, citric acid, or lactic acid. Antimicrobial agents utilized as carriers may be added to pharmaceutical compositions in multiple-dose containers that include phenols or cresols, mercurials, benzyl alcohol, chlorobutanol, methyl and propyl p-hydroxybenzoic acid esters, thimerosal, benzalkonium chloride and benzethonium chloride. Suitable excipients may include, for example, water, saline, dextrose, glycerol, or ethanol. Suitable non-toxic auxiliary substances may include, for example, wetting or emulsifying agents, pH buffering agents, stabilizers, solubility enhancers, or agents such as sodium acetate, sorbitan monolaurate, triethanolamine oleate, or cyclodextrin.

The pharmaceutical compositions can be a liquid solution, suspension, emulsion, pill, capsule, tablet, sustained release formulation, or powder. Oral formulations can include standard carriers such as pharmaceutical grades of mannitol, lactose, starch, magnesium stearate, polyvinyl pyrollidone, sodium saccharine, cellulose, magnesium carbonate, etc.

In certain embodiments, the pharmaceutical compositions are formulated into an injectable composition. The injectable pharmaceutical compositions may be prepared in any conventional form, such as for example liquid solution, suspension, emulsion, or solid forms suitable for generating liquid solution, suspension, or emulsion. Preparations for injection may include sterile and/or non-pyretic solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use, and sterile and/or non-pyretic emulsions. The solutions may be either aqueous or nonaqueous.

In certain embodiments, unit-dose parenteral preparations are packaged in an ampoule, a vial or a syringe with a needle. All preparations for parenteral administration should be sterile and not pyretic, as is known and practiced in the art.

In certain embodiments, a sterile, lyophilized powder is prepared by dissolving the truncated form of IgA protease or fusion protein as disclosed herein in a suitable solvent. The solvent may contain an excipient which improves the stability or other pharmacological components of the powder or reconstituted solution, prepared from the powder. Excipients that may be used include, but are not limited to, water, dextrose, sorbital, fructose, corn syrup, xylitol, glycerin, glucose, sucrose or other suitable agents. The solvent may contain a buffer, such as citrate, sodium or potassium phosphate or other such buffer known to those of skill in the art at, in one embodiment, about neutral pH. Subsequent sterile filtration of the solution followed by lyophilization under standard conditions known to those of skill in the art provides a desirable formulation. In one embodiment, the resulting solution will be apportioned into vials for lyophilization. Each vial can contain a single dosage or multiple dosages of the truncated form of IgA protease or fusion protein or composition thereof. Overfilling vials with a small amount above that needed for a dose or set of doses (*e.g.*, about 10%) is acceptable so as to facilitate accurate sample withdrawal and accurate dosing. The lyophilized powder can be stored under appropriate conditions, such as at about 4 °C to room temperature.

Reconstitution of a lyophilized powder with water for injection provides a formulation for use in injection administration. In one embodiment, for reconstitution the sterile and/or non-pyretic water or other liquid suitable carrier is added to lyophilized powder. The precise amount depends upon the selected therapy being given, and can be empirically determined.

### Methods of Treating or Preventing Diseases

In another aspect, the present disclosure provides a method of treating or preventing a disease associated with IgA deposition, comprising administering to a subject in need thereof the truncated form of IgA protease described herein, the fusion protein described herein, or the pharmaceutical composition described herein.

In another aspect, the present disclosure provides a method of treating or preventing a disease associated with IgA deposition, comprising administering to a subject in need thereof an IgA protease or a truncated form thereof, a fusion protein comprising the IgA protease or a truncated form thereof, or a pharmaceutical composition comprising the IgA protease or a truncated form thereof, wherein an amino acid sequence of the IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof. In some embodiments, the amino acid sequence of the IgA protease is formed after removal of the signal peptide sequence of an amino acid sequence as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76. In some embodiments, the truncated form of IgA protease has at least 70% sequence identity (e.g., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to a polypeptide as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76. In some embodiments, the truncated form of IgA protease has at least 70% sequence identity (*e.g.*, having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to polypeptide as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76, and still retains the function or activity of the IgA protease (e.g., protein hydrolytic activity, enzymatic activity of specifically cleaving IgA, etc.).

| **SEQ ID NO** | **NCBI Accession Number** | **Amino Acid Sequence** |
|---|---|---|
| 61 | WP_2488 35846.1 | MTKKITAIFLALCMAISVLPITIQAASKPDIKVGDYVK MGAYNNASILWRCVSIDNNGPLMLADKIVDTLAYD AKTNDNSNSKSHSRSYKRDDYGSNYWKDSNMRSW LNSTAAEGKVDWLCGNPPKDGYVSGVGAYNEKAG FLNAFSKSEIAAMKTVTQRSLVSHPEYNKGIVDGDA NSDLLYYTDISEAVANYDSSYFETTTEKVFLLDVKQ ANAVWKNLKGYYVAYNNDGMAWPYWLRTPVTDC NHDMRYISSSGQVGRYAPWYSDLGVRPAFYLDSEY FVTTSGSGSQSSPYIGSAPNKQEDDYTISEPAEDANP DWNVSTEQSIQLTLGPWYSNDGKYSNPTIPVYTIQK TRSDTENMVVVVCGEGYTKSQQGKFINDVKRLWQ DAMKYEPYRSYADRFNVYALCTASESTFDNGGSTFF DVIVDKYNSPVISNNLHGSQWKNHIFERCIGPEFIEKI HDAHIKKKCDPNTIPSGSEYEPYYYVHDYIAQFAMV VNTKSDFGGAYNNREYGFHYFISPSDSYRASKTFAH EFGHGLLGLGDEYSNGYLLDDKELKSLNLSSVEDPE KIKWRQLLGFRNTYTCRNAYGSKMLVSSYECIMRD TNYQFCEVCRLQGFKRMSQLVKDVDLYVATPEVKE YTGAYSKPSDFTDLETSSYYNYTYNRNDRLLSGNSK SRFNTNMNGKKIELRTVIQNISDKNARQLKFKMWIK HSDGSVATDSSGNPLQTVQTFDIPVWNDKANFWPL GALDHIKSDFNSGLKSCSLIYQIPSDAQLKSGDTVAF QVLDENGNVLADDNTETQRYTTVSIQYKFEDGSEIP NTAGGTFTVPYGTKLDLTPAKTLYDYEFIKVDGLNK |
| | | PIVSDGTVVTYYYKNKNEEHTHNLTLVAAKAATCIT AGNSAYYTCDGCDKWFADATGSVE |
| 62 | WP_0068 58468.1 | MTKKITAIFLALCMAISVLPMTIQAASKPDIKVGDYV KMGAYNNASILWRCVSIDNNGPLMLADKIVDTLAY DAKTNDNSNSKSHSRSYKRDDYGSNYWKDSNMRS WLNSTAAEGKVDWLCGNPPKDGYVSGVGAYNEKA GFLNAFSKSEIAAMKTVTQRSLVSHPEYNKGIVDGD ANSDLLYYTDISEAVANYDSSYFETTTEKVFLLDVK QANAVWKNLKGYYVAYNNDGMAWPYWLRTPVTD CNHDMRYISSSGQVGRYAPWYSDLGVRPAFYLDSE YFVTTSGSGSQSSPYIGSAPNKQEDDYTISEPAEDAN PDWNVSTEQSIQLTLGPWYSNDGKYSNPTIPVYTIQK TRSDTENMVVVVCGEGYTKSQQGKFINDVKRLWQ DAMKYEPYRSYADRFNVYALCTASESTFDNGGSTFF DVIVDKHNSPVISNNLHGSQWKNHIFERCIGPEFIEKI HDAHIKKKCDPNTIPSGSEYEPYYYVHDYIAQFAMV VNTKSDFGGAYNNREYGFHYFISPSDSYRASKTFAH EFGHGLLGLGDEYSDGYLLDDKELKSLNLSSVEDPE KIKWRQLLGFRNTYTCRNAYGSKMLVSSYECIMRD TNYQFCEVCRLQGFKRMSQLVKDVDLYVATPEVKE YTGAYSKPSDFTDLETSSYYNYTYNRNDRLLSGNSK SRFNTNMNGKKIELRTVIQNISDKNARQLKFKMWIK HSDGSVATDSSGNPLQTVQTFDIPVWNDKANFWPL GALDHIKSDFNSGLKSCSLIYQIPSDAQLKSGDTVAF QVLDENGNVLADDNTETQRYTTVSIQYKFEDGSEIP NTAGGTFTVPYGTKLDLTPAKTLYDYEFIKVDGLNK PIVSDGTVVTYYYKNKNEEHTHNLTLVAAKAATCT TAGNSAYYTCDGCDKWFADATGSVEITDKTSVKIPA PGHTAGTEWKSDDTNHWHECSRCHDKKDEAAHDY GSDNVCDTCGYYKTVPHTHNLTLVAAKAATCTESG KEAYYKCEGCGKFYEDVLGTKEITDLASWGNIAKIA HTTKQTVTKASSIKLKATSLTYNGKVRTPKVIVKDR |
| | | TGKTLVKNTDYTVSYAKGRKYVGKYAVKITFKGKY SGTKTLYFTIKPKATSISSLKAGSKKFTVKWKKQAT QTTGYQVQYSASSKFSKAKTVTVGKNTTVSKKISKL SGKKKYYVRVRTYKTVKINGKSIRIYSGWSKAKTVT TKK |
| 63 | WP_0053 63310.1 | MTKKITAIFLALCMAISVLPMTIQAASKPDIKVGDYV KMGAYNNASILWRCVSIDNNGPLMLADKIVDTLAY DAKTNDNSNSKSHSRSYKRDDYGSNYWKDSNMRS WLNSTAAEGKVDWLCGNPPKDGYVSGVGAYNEKA GFLNAFSKSEIAAMKTVTQRSLVSHPEYNKGIVDGD ANSDLLYYTDISEAVANYDSSYFETTTEKVFLLDVK QANAVWKNLKGYYVAYNNDGMAWPYWLRTPVTD CNHDMRYISSSGQVGRYAPWYSDLGVRPAFYLDSE YFVTTSGSGSQSSPYIGSAPNKQEDDYTISEPAEDAN PDWNVSTEQSIQLTLGPWYSNDGKYSNPTIPVYTIQK TRSDTENMVVVVCGEGYTKSQQGKFINDVKRLWQ DAMKYEPYRSYADRFNVYALCTASESTFDNGGSTFF DVIVDKYNSPVISNNLHGSQWKNHIFERCIGPEFIEKI HDAHIKKKCDPNTIPSGSEYEPYYYVHDYIAQFAMV VNTKSDFGGAYNNREYGFHYFISPSDSYRASKTFAH EFGHGLLGLGDEYSNGYLLDDKELKSLNLSSVEDPE KIKWRQFLGFRNTYTCRNAYGSKMLVSSYECIMRD TNYQFCEVCRLQGFKRMSQLVKDVDLYVATPEVKE YTGAYSKPSDFTDLETSSYYNYTYNRNDRLLSGNSK SRFNTNMNGKKIELRTVIQNISDKNARQLKFKMWIK HSDGSVATDSSGNPLQTVQTFDIPVWNDKANFWPL GALDHIKSDFNSGLKSCSLIYQIPSDAQLKSGDTVAF QVLDENGNVLADDNTETQRYTTVSIQYKFEDGSEIP NTAGGTFTVPYGTKLDLTPAKTLYDYEFIKVDGLNK PIVSDGTVVTYYYKNKNEEHTHNLTLVAAKAATCT TAGNSAYYTCDGCDKWFADATGSIEITDKTSVKIPA PGHTAGTEWKSDDTNHWHECSRCHDKKDEAAHSA |
| | | SEWIIDTAATETAEGAKHKECTVCKKVLETATIPATG SSHTHSYGVYVGMTYTAGNLIYQITSIDTATLGQSK VIGVVAAKKNKITKITIPDRADCKGYRLNVTTIGNNA FAGCKALKKLTIGNKVTVIGKNAFKKCSKLKTVVIG KAVKTISSKAFIGDNKIKKITFKGKKLKTVNKNAFSK KAKKNIKSKKTKLKGNKKAIKLFKKKLKIK |
| 64 | WP_0700 97494.1 | MTKKITAIFLALCMAISVLPMTIQAASKPDIKVGDYV KMGAYNNASILWRCVSIDNNGPLMLADKIVDTLAY DAKTNDNSNSKSHSRSYKRDDYGSNYWKDSNMRS WLNSTAAEGKVDWLCGNPPKDGYVSGVGAYNEKA GFLNAFSKSEIAAMKTVTQRSLVSHPEYNKGIVDGD ANSDLLYYTDISEAVANYDSSYFETTTEKVFLLDVK QANAVWKNLKGYYVAYNNDGMAWPYWLRTPVTD CNHDMRYISSSGQVGRYAPWYSDLGVRPAFYLDSE YFVTTSGSGSQSSPYIGSAPNKQEDDYTISEPAEDAN PDWNVSTEQSIQLTLGPWYSNDGKYSNPTIPVYTIQK TRSDTENMVVVVCGEGYTKSQQGKFINDVKRLWQ DAMKYEPYRSYADRFNVYALCTASESTFDNGGSTFF DVIVDKYNSPVISNNLHGSQWKNHIFERCIGPEFIEKI HDAHIKKKCDPNTIPSGSEYEPYYYVHDYIAQFAMV VNTKSDFGGAYNNREYGFHYFISPSDSYRASKTFAH EFGHGLLGLGDEYSNGYLLDDKELKSLNLSSVEDPE KIKWRQLLGFRNTYTCRNAYGSKMLVSSYECIMRD TNYQFCEVCRLQGFKRMSQLVKDVDLYVATPEVKE YTGAYSKPSDFTDLETSSYYNYTYNRNDRLLSGNSK SRFNTNMNGKKIELRTVIQNISDKNARQLKFKMWIK HSDGSVATDSSGNPLQTVQTFDIPVWNDKANFWPL GALDHIKSDFNSGLKSCSLIYQIPSDAQLKSGDTVAF QVLDENGNVLADDNTETQRYTTVSIQYKFEDGSEIP NTAGGTFTVPYGTKLDLTPAKTLYDYEFIKVDGLNK PIVSDGTVVTYYYKNKNEEHTHNLTLVAAKAATCT EGGKEAYYKCEGCGKFYEDVLGTKEITDLASWGNI |
| | | AKIAHTTKQTVTKATPTANGKIVNYCSVCKKTLSTT VIPKASSIKLKATSLTYNGKVRTPKVIVKDRTGKTLV KNTDYTVSYAKGRKYVGKYAVKITFKGKYSGTKTL YFTIKPKATSISSLKAGSKKFTVKWKKQATQTTGYQ VQYSASSKFSKAKTVTVGKNTTVSKKISKLSGKKKY YVRVRTYKTVKINGKSIRIYSGWSKAKTVTTKK |
| 65 | WP_1603 40763.1 | MTKKITAIFLALYMAISVLPMTIQAASKPDIKVGDYV KMGVYNNASILWRCVSIDNNGPLMLADKIVDTLAY DAKTNDNSNSKSHSRSYKRDDYGSNYWKDSNMRS WLNSTAAEGKVDWLCGNPPKDGYVSGVGAYNEKA GFLNAFSKSEIAAMKTVTQRSLVSHPEYNKGIVDGD ANSDLLYYTDISEAVANYDSSYFETTTEKVFLLDVK QANAVWKNLKGYYVAYNNDGMAWPYWLRTPVTD CNHDMRYISSSGQVGRYAPWYSDLGVRPAFYLDSE YFVTTSGSGSQSSPYIGSAPNKQEDDYTISEPAEDAN PDWNVSTEQSIQLTLGPWYSNDGKYSNPTIPVYTIQK TRSDTENMVVVVCGEGYTKSQQGKFINDVKRLWQ DAMKYEPYRSYADRFNVYALCTASESTFDNGGSTFF DVIVDKYNSPVISNNLHGSQWKNHIFERCIGPEFIEKI HDAHIKKKCDPNTIPSGSEYEPYYYVHDYIAQFAMV VNTKSDFGGAYNNREYGFHYFISPSDSYRASKTFAH EFGHGLLGLGDEYSNGYLLDDKELKSLNLSSVEDPE KIKWRQLLGFRNTYTCRNAYGSKMLVSSYECIMRD TNYQFCEVCRLQGFKRMSQLVKDVDLYVATPEVKE YTGAYSKPSDFTDLETSSYYNYTYNRNDRLLSGNSK SRFNTNMNGKKIELRTVIQNISDKNARQLKFKMWIK HSDGSVATDSSGNPLQTVQTFDIPVWNDKANFWPL GALDHIKSDFNSGLKSCSLIYQIPSDAQLKSGDTVAF QVLDENGNVLADDNTETQRYTTVSIQYKFEDGSEIP NTAGGTFTVPYGTKLDLTPAKTLYDYEFIKVDGLNK PIVSDGTVVTYYYKNKNEEHTHNLTLVAAKAATCT TAGNSAYYTCDGCDKWFADATGSVEITDKTSVKIPA |
| | | PGHTAGTEWKSDDTNHWHECSRCHDKKDEAAHDY GSDNVCDTCGYYKTVPHTHNLTLVAAKAATCTTAG NSAYYTCDGCDKWFADATGSVEITDKTSVKIPAPGH TAGTEWKSDDTNHWHECTVAGCGVIIESTKSAHTA GEWIVDTPATATTAGTKHKECTVCHRVLETQPIPST GTELKIIAGDNQIYNKASGSDVTITCNGDFAKFTGIK VDGSVVDSSNYTAVSGSTVLTLKASYLGTLTDGSHT ITFVYTDGEANANLTVRTAGSGHIHDYGTEWKSNA DNHWHECNCGDKKDEAAHSFKWVVDKEATATKK GSKHEECKICGYKRSAVEIPATGTSTAPTDTTKPNDT TKPGNTNGSEKSPQTGDNSNIFLWFALLFVSAAGVT GITAYNKKKKEHAE |
| 66 | MCJ7966 723.1 | MTKKITAIFLALCMAISVLPMTIQAASKPDIKVGDYV KMGAYNNASILWRCVSIDNNGPLMLADKIVDTLAY DAKTNDNSNSKSHSRSYKRDDYGSNYWKDSNMRS WLNSTAAEGKVDWLCGNPPKDGYVSGVGAYNEKA GFLNAFSKSEIAAMKTVTQRSLVSHPEYNKGIVDGD ANSDLLYYTDISEAVANYDSSYFETTTEKVFLLDVK QANAVWKNLKGYYVAYNNDGMAWPYWLRTPVTD CNHDMRYISSSGQVGRYAPWYSDLGVRPAFYLDSE YFVTTSGSGSQSSPYIGSAPNKQEDDYTISEPAEDAN PDWNVSTEQSIQLTLGPWYSNDGKYSNPTIPVYTIQK TRSDTENMVVVVCGEGYTKSQQGKFINDVKRLWQ DAMKYEPYRSYADRFNVYALCTASESTFDNGGSTFF DVIVDKYNSPVISNNLHGSQWKNHIFERCIGPEFIEKI HDAHIKKKCDPNTIPSGSEYEPYYYVHDYIAQFAMV VNTKSDFGGAYNNREYGFHYFISPSDSYRASKTFAH EFGHGLLGLGDEYSNGYLLDDKELKSLNLSSVEDPE KIKWRQLLGFRNTYTCRNAYGSKMLVSSYECIMRD TNYQFCEVCRLQGFKRMSQLVKDVDLYVATPEVKE YTGAYSKPSDFTDLETSSYYNYTYNRNDRLLSGNSK SRFNTNMNGKKIELRTVIQNISDKNARQLKFKMWIK |
| | | HSDGSVATDSSGNPLQTVQTFDIPVWNDKANFWPL GALDHIKSDFNSGLKSCSLIYQIPSDAQLKSGDTVAF QVLDENGNVLADDNTETQRYTTVSIQYKFEDGSEIP NTAGGTFTVPYGTKLDLTPAKTLYDYEFIKVDGLNK PIVSDGTVVTYYYKNKNEEHTHNLTLVAAKAATCT TAGNSAYYTCDGCDKWFADATGLVEITDKTSVKIPA LGHTAGTEWKSDDTNHWHECSRCHDKKDEAAHDY GSDNVCDTCGYYKTVPHTHNLTLVAAKAATCTTAG NSAYYTCDGCDKWFADATGLVEITDKTSVKIPALGH TAGTEWKSDDTNHWHECSRCHDKNDEAAHSTSEWI IDTAATETAEGAKHKECTVCKKVLETATIPATGSSHT NSYGVYVGMTYTAGNLIYQITSIDTATVGQSKVIGV VAAKKNKIKKVTIPDRADCKGYRLNVTTIGNNAFAG CKALEKLTIGNKVTVIGKNAFKNCSKLETVVIGKAV KTISSKAFIGDNKIKKITFKGDKLKTVKKNAFSKKAK KNIKSKKTKLKGNKKAIKLFKKKLKIK |
| 67 | HAC1090 2.1 | MKKYFEKTSIALIIAMMFILAIFGGEAMKTHTIDDITK YKMVVNAQGVKTENGTRTTTQVELGNYISLGKYNG KEILWRCVGEDENGALMLADNIIDTLPYDAKTNDN NRSKSHSRNYKRDTYGSNYWKDSNMRSWLNSTAV AGEVKWLCGNPPREDSVNGNAYDQKAGFLNDFSK AEIAAMKNVTQRSIVSHPEYNLGFHDGEGRSDLELN FDIENVASNFDSAYGENSTEKVFLLDVKQVNTVWK NFGNYYIGRNEQGMAWPYWLRTPVTDCNHDMRYV HSNGSVGREWPNTDYIGVRPAFYLDSDYYATTSGD GSASNPYVGSAPDKIEDDYTVAEPEEDPNQEWDISL DQQLRLTLGPYYSSDGKYATPTIPVYTIQKTRSDTEN MVILICGEGYTKSQQQKFIDDVKKVWEGAMQYEPY RSYADRFNVYALCTASESNFNSGGSTFFDVVIDKKS GPMISVNKSAWKNHIFERCIGPTFLEQIHDAHIPNKT DPDTFIWDDDKMYPPFYYVHKYINQFAVLVNTTQD FGGSHRNYKRGIHYLITPADSPRAQKTFTHELGHGLL |
| | | ELGDEYMTTAAESTDYTSLNVAYTHDPEKVKWKQ MLGFRKTFTCNTSPSYTAYNSSWECLMRDTTYQFCE VCKLQGSKRMSQLIDGKSLYVADPEVKKYTVQYSK PSDFADTTYNGYYYFENYRNNVLLSGVDKNKFNTS MAGEKIQLRTIVQNLSDTTQRYVTMKLWIKHAGGS VATTTGGQRLEATQTFTIPVWSEKSKFWPKGALSYE GSNMNSGLENCELIYQIPLDADLKHGDYVAFEVTDE SGNILANDNTETQTYANINIEYKFEDGTPMPNANKA TIPVAVGSQLNWTAPSTMFGHTFVRAEGHDQMVNG SGQTVTYYYTKQSNVHIHDWGEVKYTWTSDNTCK AERVCKHDSTHIESETVTATGTTITAATCKEKGKMK YTATFVNTAFSMQEKEVDIDFAEHTYGAWIEEVPAT CIAGGMKAHYKCTVCGKYFDENKNETTEEALKTPV SPYYGHSFGFWVEEQYATCQAPGRKGYKHCSICNK DYDASNTEITDFVIPINPDGHELGDLVAEVPATCKDT GVKEHRDCRLCGKHCDPITRKEIADLTIPTTNNHTYG ELIPEVSPTTTEFGVKEHKDCTVCGRHFDKDGNEITE LRIAKIGTHNVIVNGESKFYAHGESVTVTANEPAEG KVFKGWQDASGKIVSTDKSYTFTVNGETTLTAVYE DKSSGGGEITPPAKKDGLSGGQIAGIVTGSAAVAGL GGFAVLWFVVKKKTFADLGALLKKGFTAIGNFFKT LGEKIKALFTKKK |
| 68 | WP_0552 60806.1 | MKKQLTALVLCICMVLSVLPFSSTQAAAEETSSVGT SNISIGDYIRLGNYNGQPILWRCVDVDEMGPLMLSD QVLATMAYDAKTSENSATRSHSRNLKRGTYGSNQW RDSNMRSWLNSKAEAGKVEWLCGNPPKSGFVGENP YDQAAGFLNGFKEDEIAAIKTVTQRSIVSHPEYNAG MIEGQGADLPYDTNIEAAANGFDQAYYENVTDKVF LMDVKQINKVYQNNSQLGGSYHIAYKGGVRWPYW LRTPVTDCNHDMRYVETDGRIDRNAPYLGFYGVRP AFYLDTQYYQVTGGDGTADSPYRGAAVNKPEENFT VSGDGPTPGQEWDVSLDKSIQLYLGPNYSKSKKYES |
| | | ATIPIQVIQKTRSDNENMVVVICGEGYTKGEQQKFV AAAKRLWEGAMQYEPYKTYKDRFNVYALCTASDK TYSAIDGYDSTFFDVWGKNISVNGSQWKNHIFERCI GPAFIEKVHDAHIPQQADPNVDWDFEKYKYVHDYIS QFVLLVNSANDFGGAFNDLDYGFHYIVSPAYSQRAV ETLTHELGHGLLWLGDEYNSGSFMGEASEKTSLNRT GISDPEQVKWRQLLGFRKTYSVPHTDYDTDKIYNSS RECMMRQTWNGFCEVCKLQGNKRMRQLVTEGPDL YVAEPEVTKRTDAYTKLSDFSDATGWGYTKFDADK KTRLLTGADKITFQPTEMKGQKIELRTIVQNLSDTKL SQVTLQVWVNHADGTIATADGQPVAASETFKIPLW TEKGNFRPKGTLEYHGSDFNSGLKNCSLLYTIPSNAD LRTGDTVGYAVRDEQGTVLAYEGTLPNKGQDILPAP EPAKSYTVTFCYNDGRENTTKTTGINGKLGDLPAPA REGYVFDGWYTTGGEKVDLTRVYSSNTVLYARWSE YIAPSPNVKKAPVILLAASPDTVTEGEQVMLSVSETS GFGVDLSGVTYTADPSLPISGTGEAQTIRLDQAGTYT FTAHYSGDNEKYLAADSNRVTVTVTKKADVSGGTT SGGGSSSGGGSTAGGGSSAGGGSSAGGGSSSGGGAA GGATAGGGAANGNASATTTPDIKDSDGTTVAIVNG KKGMITAEVQLSEKAIANAEKSGEAVKLPVEVKAG KNIKAASTVTINLPEGAGKTKVKIPVKNMTAGTVAV LVNADGTEKIVKKSVAAKDGVQLIVDEDTTVKLVD KAKNFKDTKKHWAKDSIDFVSARGLMNGKSSTAFA PEAKITRARLWTILARWEDVDLTGGKKWYSKARA WAKNQGISDGSRPNAAITRAEAITMLWRAQGKPAA EQETAFKDVSSDEYYAQAVAWAKEKGIAQANSKGR FNPDAACTRAEIAAFLYRMSLSE |
| 69 | CDE2481 1.1 | MKKNFGKASIALIIAMMFILAFFGGDVMKAHTIDGS ASNLYVGSALDKVEDDYTVAEPEKAPNQELDISIEQ SLNLTLGEWYSSDGKYANPTIPVYTIQKTRSDTENM VILICGEGYTKNQQQKFVNDVKKVWEGAMQHEPYR |
| | | SYADRFNVYALCTVSESSFNSGGSTFFDVVIDKNSGP MIAISKSICKNHIFERCIGPAFLEQIHDVHIPKKVDPNS SYWVGNNSPLSEYEPFYYVHEYINQFAILVNTTQDF GGSHRNYERGIHYLVTPADSDRAQKTFTHELGHGLL ELGDEYMSSTTQQTDLTSLNVAHTHDPNNVKWKQL LGFRKTYTCNALGYGNAYNSSYECLMRDTAYQFCE VCKLQGSKRMSQLIDGKSLYVAVPEVKKYTGQYSK PSDFIDTTYNGYYYFENYRKGVLLSGTDKNKFNTSM AGETIQLRTIVQNLSDTKQRYVTMKLWIKHADGSVA TTTGGQRLEATQTFTIPVWSEKSKFWPKGALSYEGS HMNSGLENCELIYQIPSDAVLNNGDTVAFEVIDENG NILANDNTETQAYANINIEYKFEDGTPMPNVNQAMI PVAVGSQLNWTAPSTMFGHKFVRAEGHDQMVNGS GQTVTYYYKKQSNVHIHDWGEVIYTWISDNICKAER VCKHDSAHIESETITATGTVIKASTCTEKGKVKYTAR FTNTAFGVQYREVDIDLVEHKFGEWIDEIPATTENFG TKGHKDCTVCKKHFDKDGNEITDLRIAKISTYTVTV KDGADETNTHYKSGDTVTIKVTIPTGKHFVKWSAVT GISLSASQLTQEEITFTMPDNDVTLIAELEDILYRVTV IGGTTTLNEAKYQENVTVTANTPEVGKEFDKWIVSG ITLSNTNLTRSTLTFTMPENDVTFTATYKDIVYRVMV EEGKATPEMAIYQTEVTVMANEPAIDMYFDKWEVM GLDTTGMDLTKTQIKFQMPAGNVTFKATYLPHIKYG ILVVDGTKDKSPVMAGEIVTITANPAKPGKVFDKWT CETVGVTIEFASATSKQTTFVMPAQDIKIKAHFKDIE VAPSVEIKVEGGTGAGTYKPGDSVTITANEPAEGKV FKCWKDEKGEIVSTDRSYAFIVNGETTLTAVYEDKA SGGAIAGIVIGSILGVGIIGFVIFWFAVKKKDVF |
| 70 | HCS2457 7.1 | MRWNIMKKYFGKANIALIIAMMFILAFFGGEAMKT HTIDGSASNLYVGSALDKVEDDYTVAEPEKAPNQEL DISIEQSLNLTLGEWYSSDGKYANPTIPVYTIQKTRSD TENMVILICGEGYTKNQQQKFVNDVKKVWEGAMQ |
| | | HEPYRSYADRFNVYALCTVSESSFNSGGSTFFDVVID KNSGPMIAISKSICKNHIFERCIGPAFLEQIHDVHIPKK VDPNSSYWVGNNSPLSEYEPFYYVHEYINQFAILVN TNQDFGGSHRNYERGIHYLVTPADSDRAQKTFTHEL GHGLLELGDEYMSSTTQQTDLTSLNVAHTHDPNNV KWKQLLGFRKTYTCNALGYGNAYNSSYECLMRDT AYQFCEVCKLQGSKRMSQLIDGKSLYVAVPEVKKY TGQYSKPSDFIDTTYNGYYYFENYRKGVLLSGTDKN KFNTSMAGETIQLRTIVQNLSDTKQRYVTMKLWIKH ADGSVATTTGGQRLEATQAFTIPVWSEKSKFWPKG ALSYEGSHMNSGLENCELIYQIPSDAVLNNGDTVAF EVIDENGNILANDNTETQAYANINIEYKFEDGTLMPN VNQAMIPVAVGSRLNWTAPSTMFGHKFVRAEGHDQ MVNGSGQTVTYYYKKQSNVHIHDWGEAIYTWTSD NICKAERVCKHDSAHIESETITATGTVIKAATCTEKG KVKYTARFTNTAFGVQYREVDIDLVEHKFGEWIDEI PATTENFGTKGHKDCTVCKKHFDKDGNEITDLRIAK ISTYTVTVKDGADETNTHYKSGDTVTIKVTIPTGKHF VKWSAVTGISLSASQLTQEEITFTMPDNDVTLIAELE DILYRVTVIGGTTTLNEAKYQENVTVTANTSEVGKE FDKWIVSGITLSNTNLTRSTLTFTMSENDVTFTATYK DIVYRVMVEEGKATPEMAIYQTEVTVMANEPAIDM YFDKWEVMGLDTTGMDLTKTQIKFQMPAGNVTFK ATYLPHIKYGILVVDGTKDKSPVMAGEIVTITANPAK PGKVFDKWTCETVGVTIEFASATSKQTTFVMPAQDI KIKAHFKDIEVAPSVEIKVEGGTGAGTYKPGDSVTIT ANEPAEGKVFKCWKDEKGEIVSTDRSYAFTVNGETT LTAVYEDKASGGAIAGIVIGSILGVGIIGFVIFWFAVK KKDVF |
| 71 | MBD9025 975.1 | MILLQIYYTKEGVKMKNKQINRTLSLLLSVVMVLSL CPLIAKAEGTKPNIKIGDYIKLGTYENEPILWRCVDID DNGPLMLMDKVLGSMPYDAKTSENSATRSHSRNSF |
| | | RSSYGSNHWRDSNMRSWLNSDADAGKVDWLCGNP PKSDYVGYGSEYDKKAGFLNGFSKAEIAAIKTVTQR SIVSHPEYSAGYIAGPGADLPYNTDIASVAYGFEKAY YENIIDKVFLPDVKQLNTIYNNSNILGNYYLAKNKD GIRWSYWLRTPITDCNHDMRYVETDGNIYRVAPYF GHIGVRPAFYLDTDYYIVSEGNGEVNSPYVGDAADK PGDDISISGPDEEGGDGDWDIDTDQSIQLNLGPWYSS DGEYANSTIPVQVIQKTRSDLENMVIVICGEGYTKD QQQKFINDVKRIWAGVLKHEPYRSMADRFNVYALC TASKTSGFASENTFFDITMSTTSRSPMISLYKSVLKN QILTRCIGPAFIEKIHDAHIKEKTNPNEITIGDEYAPYY YVNEYISQFVVLVNSGQYGGASMNNLDVGLHYVTA TVDNIQSEYTLAHELGHGLLHLGDEYNAYGGAYTM PEQQDKQSLNIAGLRESPITIKWKDMLGFRKTYTCR DSNTSNSSNMVNSSWQCMMRTQNQELCDVCQLQG FKVMSQLIKDTDDIYIAIPEVKLYTGNYKNPFEDYSA YTEAEYYGYLAYASDRAQRLLSGTSKNKFTKDMKG QEVELRTIAQNLSGIEEQEITLQLWVEHEDGTRAVTE NGEEILKEQTFTVPVWDEKENFYIKGMRNYSGTEFD SGLMNCSLIYKIPENADLKDGDTIKFSVIDKMGKTLA DDNTETQNYANVTISYQLEDSNAVPNTQTAVIPVPIG TKMDIEPPGELYGYKFVKAEGLGKIVGDDGLNIICY YEDPSGKLPVEYKVEYDWGTDFPTDTTLPTDNTKY DSIENAKESVKNQKYDENSTSTVKKNDKDGTWTFS GWTATVEGTTVKFTGAWTFTATPIITYTVTYDWGT DFPTGEMLPVDSKTYKSEEDAKAAMDGKYTSLSTST AEKDGKSGTWKFSGWIATLIGTTVKFNGMWTFTPD APVVDADTPTNIKLVSDEYKIGDKATALDGKATVSD NGVLSYQWYKSDKADNFNGTAIDGQNGETFVPDTS KEGTYYYYVIATNTKADATGKKTASVTSSMAIIHVK ESVKYTVVYDWGSDYPTDVTVPKDDTKYENIEKAK EAVKNQKYDENSTSTAEKNSKSGKWSFSGWTTAVE GTTVKFTGVWTFTENAIPVVTRKPSSGGSGGSGSST |
| | | YNIKVSPEITNGSLSVNPSRASNGKKVSVIVKPNNGY VLNSVIVKDSNDKEIAVTKQSDGTYTFIMPSSNVTVS AKFDTELAKDVVTEIEKSIEFKDVKKGDWYFDAVQ WAVKNNITEGSGKDTFSPDVICTRTQMVTFLWRVA GSPEPKITKCDFRDVDNSAYYYKAVLWAVEKGVTV GTSDTTFSPNENVTRGQTVAFLYREAGSPFETGEDVF NDVNSNDYYFKAVSWATKNGITVGTGNGKFEPDM DCNRAQIVAMLYRTQR |
| 72 | CDE1602 7.1 | MKKHLKKTSIALTIVMMFIPAIFGGKAITIHTNADNT NYKTAVNAQGVKTEKETKATTQVELGNYISLGKYN GNEVIWRCVSIDEKGALMLADNIIDTLPYDAKTNDN NHSKSHSRNNNRDNHGSNYWKDSNMRSWLNSTAV AGEVTWLCGNPPRAGYLNENAYDQKAGFLNDFSKA EIALMKNVTQRSLVSHPEYNHGFHDGDGHSDLEFNE NIENVSSNFNSAYGENSTEKVFLLDVQQVNKVWENF DNYYIGRKEGVAWPYWLRTPLSSCNHLMRYVGSNG LVGKDYPTNAIGVRPAFYLDSDYYVTTSGNGSASNP YVGSAPDKIEGDYIIAEPEEDPNQEWDVSLDQQLRL ALGPYYSSDGKYSTPTIPVYTIQKTRSDTENMVILICG EGYTKSQQQKFINDAKKVWEGAIQYEPYRSYADRF NVYALCTASESSFNNSGSTFFDVVIDKKVGPMISVN KSSWKNHIFERCIGPAFIEQIHDAHIPNKTDPDTFIWD DDKMYSPFYYVHKYINQFALLVNTSQDFGGSHRNY KRGIHYFITPADNNRAVKSFAHELGHGLLELGDEYM TVAAESTDYTSLNVAYNHNPEQVKWKKLLGFRKTF TCNTYPFYTAYNSSWECLMRDTNYQFCEVCKLQGY KRMSQLIDGKNLYVADPEVKKYTDRYTNPSDFAET NYNGYINFTNYRDEILLSGWNKNKFNTGMVGEKIQL RTIVQNLSDTTERQVTMKLWIKHADGSIATTTNSQR LEATQTFTIPVWSEKSKFWPKGALEYNGSNLNSGLE NCELIYQIPSDAVLNNGDTVAFEVTDENGNVLAHDN TETQPYANVNIEYKFEDGSPMPNANKAVIPLAVGSYI |
| | | NWTAAPSLYGYALSRVEGLNQIVSGSDQTVTYYYT KKIGTHIHDWGDWVSNGDGTHTRTCTKDSSHTETE NCSGGTATCTTKAVCSVCGFTYGEKLGHNWGEVKY TWMSDNICKAERVCKHDSTHIESEMVTATGTVITKA TCKEKGKMKYTATFVNTAFTVQEKEIDTDFAPHTFG AWKDEIPATTEEFGTKGHKDCMDCGRHFDKDGKEI TELRIAKIGTYNVVINGESKFYADGESVTVKAEDKE GKIFKGWQDESGEIVSTEKSYTFTVTGDRSLTAVYE NVLATKKGLSDRQIAGIIIGSVVAAGLGGFAIFWFVI KKKGLRL |
| 73 | WP_1182 65594.1 | MNIIKHKYGKRTVSLLLAVILVLCPLPVRAADNKPTI EIGDYIQMGTYGGVPIVWRCVAKDSNGPLMLSDRV LCDYMPYDAKTNKNAETGSHRRNSWRDNFGSNHW RDSNIRSWLNSNAEAGKVKWLCGNPPTEDSVYPKT AAYDQKEGFLRSFRSDELGAIRTVKQRSIVSHPEYTA GYIDAAGVDLLYNTTIDTVADGYDSAHYEYIWDRV FLLDVQQLKTVNDNLNGYHIAKNRSGVAWNYWLR TPITTCNHDMRFVTPQGNILRDAPYKGYYGVRPAFY LNTENYTVSSGTGQSAQDPYVVSAPDATDDSIGISG AVREDVNGDWNVNTDEYLQLEMSTLYTEDPAYAN VTVPVYTIQKPRSDKENMVIVYCAEGYTKSQQKQFV EDVKKLWGEVLQIEPYRSMADRFNVYALCTASVDG YGGTSTFFAATAKGGISTNKGNWRNHVLERIIGPAFI EKIHDAHIPNETHPNENTMDHNYRQYDYVYENINQF VVLANSGEYFGGSHDNKQYGIHYIVASARNAYSAFT QRHELGHGLFHLGDEYNYSTVPVDEWNYTTSLNMT ATKDPTKVKWKQLLGFRNTYTCPHLDYYPYTYNSS RDCLMRETFQNDFCDVCKLQGIKVMSQLITNPPALY VAVPEVKKYIGGYRNPTKDPSAFEAANSSAYASYQN DRNSRLLSGGSKNSFDYSSMKGQQVELRTIIQNLSNT QAKTVTLRLWVEHSNGEKAVTTDGEQVFTTQEFDIP VWKEKSKFWTKGALDYEGSDFNSGLVNCSLVYTIP |
| | | ENAILQSGDTIGFEIVDHATGEVLADDDTEQQRYVN VTIQYQLEDGTDVPNTMPTTFTVPVGKKVDWQPPQ ELHGYTFVKAEGMENAVPNSGMTIRYIYKRSEERPE PPVTKNYTVQYNWGSVFPTGATLPLNSSSYSSVQQA KAAVDKKYTSTTRIQAQKDGKNGTWAFSGWDAGN LNGTTVVFRGSWSFTADTAPITPPSGTASYKITATAG IGGTISPGGTTTVSAAGQLTYTIKANEGYYIADVKVD GSEVTATTSYTFSDVNTDHTIEVTFKQESQTPDVPDV IAPSITTQPGNATVKVGETASFTIAASGTDLTYQWQI DRNDGKGWVNIDGATATSYTTSTVNISCNGFKYKC VVSNSAGNVESNSATLTVQDAGGSDNPDTPNNTYQI IDGANSSWTHDSDGNITIRGNGDFSKFTGVKVDGNLI DKSNYTAKEGSTIITLKASYLNTLSAGNHTVEILWTD GSASTTFTTKANISDNSNNNQNDNNNSNSSDDKPSS GTDKKDVTAPKTGDNTPSVWLFILSILSGTGLIITVK KRRENLNS |
| 74 | WP_2431 21302.1 | MNVIKHRYGKRTVSLLLAVILVLCPLRVRAADNKPT IGIGDYIKSAQDPYVVSAPDAPDYSIGISGAVRDDVN GDWNVNTDEYLQLKMSTYYTEDTAYANVTVPVYTI QKPRSDKENMVIVYCAEGYTKSQQKQFIEDVKKLW GEVLQIEPYRSMADRFNVYALCTASVDSFGGTSTFF NATKKGISNSKGAWRNHILERIIGPAFIEKIHDAHIPN KTHPNENPGDHDYRQYDYVVYENINQFVLLANSGEY FGGSHDNKEHGIHYIIASARSQYSAFTQRHELGHGLF HLGDEYNYSTVPVAEANYTTSLNMTATKDPTKVKW KQLLGFRNTYTCPHDDRYPYTYNSSRDCLMRETFQ NDFCDVCKLQGIKVMSQLITNPPALYVAVPEVKKYI GDYRNPTEKPSAFEAANSSAYASYQYDRNSRLLSGG SKNSFDYGSMKGQQVELRTIIQNLSDTQAKTVTLRL WVEHSNGEKAVTTEGQQVFATKDFAIPAWSEKSKF WPKGALDYKGSDFDSGLVNCSLVYTIPENATLQYG DTIGFDIVDRATGEVLAHDDTEKQPYADVTIQYQLE |
| | | DGTDVPNTMPTTFTVPVGKKVDWQPPQELNGYTFV QAEGMEETVPSNGMTIRYIYKRTEERPEPPVTKNYT VKYDWGSVFPTGVTLPQNSNSYSSEQQAKAAVDKK YTTSTRIKAQKDGKNGTWAFSGWDSGSLNGTTVVF RGSWSFTADTAPITPPSGGGGGGAATTASYKITAT AGIGGTISPGGTTTVSAAGQLTYTIKANEGYYIADVK VDGKSVGAVSSFAFEKITASHTIEASFAKKDSATVKD PIKRLPIHLPIKMKVKKM |
| 75 | UKI49074 .1 | MKKTYLFKSYKRKSQKEVEHNEKNILKKTIIALIIAM MFILAIFFGGEAMKTHTIDDVAKYKMVVNAQGVKM GNGTRTQTQVDLGNYISLDQQLKLTLGPYYSSDGKY STPTIPVYTIQKTRSDTENMIILICGEGYTKSQQQKFIE DVKRVWDGAIQHEPYRSYADRFNVYALCTASESSF NSGGSTFFDVVIDKKSGPRISGNKSAWKNHIFERCIG PTFLEQIHDAHIPNKTDPDTFIWDDDKMYPPFYYVH KYINQFAVLVNTEQDFGGSHRNYKSGIHYLITPADSP RAQKTFTHELGHGLLELGDEYMTSATESTDYTSLNV AYTHDPEKVKWEKMLGFRKTFTCHTNSSYTAYNSS WECLMRDTTYQFCEVCKLQGSKRMSQLIDGKSLYV ADPEVKKYTGQYSQPSDFADTTYNGYANFSYYRSG VLLSGWDKNKFNTDMAGEKIQLRTIVQNLSDTTQR YVTMKLWIKHADGSVATTTGGQRLEATQTFTIPVW SEKSKFWPKGALSYEGSNMNSGLENCELIYQIPLDA VLNKGDTVAFEVTDENGNVLANDNTETQTYANINIE YKFEDGTPMPNVNKATIPIAVGSKLNWTAPSTMFGH TFVRAEGHDQMVNGSDQTVTYYYAKQSNVHIHDW EEWVSNGNGTHTRTCRTDNSHSETANCVGGTATCT HKPVCEVCHGEYGQAKSHDWGKATYTWTDTVCKA ERVCKHDSAHTESETRTATGTVIKAATCKEKGKMK YTATFENTAFTKQEKKVDINFAGHTFGKWQDEIPAT TEAFGTKGHKDCSVCGRHFDKDGNEITELRIAKIVT HNVIVNGESKFYAHGESVTVTANEPAEGKVFKGWQ |
| | | DASGKIVSTKKSYTFTVNGETNLTAVYEDKTSGGEI VPPAKKDGLSGGQVAGVVIGSAAVAGIGGFAIFWFT VKKKTFADLIAAIKSLFTKKKTK |
| 76 | WP_0056 04305.1 | MNIIKHKYGKRTVSLLLAVILVLCPLQVRAADNKPTI EIGDYIQMGTYGGVPIVWRCVAKDSNGPLMLSDRV LCDYMPYDAKTNKNAETGSHRRNSWRDNFGSNHW RDSNIRSWLNSNAEAGKVKWLCGNPPTEDSVYPKT AAYDQKEGFLRSFRSDELGAIRTVKQRSIVSHPEYTA GYIDVAGVDLPYNTTIDTVADGYDSAHYEYIWDRV FLLDVQQLKTVNDNLNGYHIAKNRSGVAWNYWLR TPITTCNHDMRFVTPQGNILRDAPYKGYYGVRPAFY LNTENYTVSSGTGQSAQDPYVVSAPDAPDDSIGISGA VREDVNGDWNVNTDEYLQLEMSTLYTEDPAYANV TVPVYTIQKPRSDKENMVIVYCAEGYTKSQQKQFVE DVKKLWGEVLQIEPYRSMADRFNVYALCTASVDGY GGTSTFFAATAKGGISTNKGNWRNHVLERIIGPAFIE KIHDAHIPNETHPNENTMDHNYRQYDYVYENINQFV VLANSGEYFGGSHDNKQYGIHYIVASARNAYSAFTQ RHELGHGLFHLGDEYNYSTVPVDEWNYTTSLNMTA TKDPTKVKWKQLLGFRNTYTCPHLDYYPYTYNSSR DCLMRETFQNDFCDVCKLQGIKVMSQLITNPPALYV AVPEVKKYIGGYRNPTKDPSAFEAANSSAYASYQND RNSRLLSGGSKNSFDYSSMKGQQVELRTIIQNLSNTQ AKTVTLRLWVEHSNGEKAVTTDGEQVFTTQEFDIPV WKEKSKFWTKGALDYEGSDFNSGLVNCSLVYTIPE NAILQSGDTIGFEIVDHATGEVLADDDTEQQRYVNV TIQYQLEDGTDVPNTMPTTFTVPVGKKVDWQPPQEL HGYTFVKAEGMENAVPNSGMTIRYIYKRSEERPEPP VTKNYTVQYNWGSVFPTGATLPLNSSSYSSVQQAK AAVDKKYTSTTRIQAQKDGKNGTWAFSGWDAGNL NGTTVVFRGSWSFTADTAPITPPSGTASYKITATAGI GGTISPGGTTTVSAAGQLTYTIKANEGYYIADVKVD |
| | | GSEVTATTSYTFSDVNTDHTIEVTFKQESQTPDNTYQ IIDGANSSWTPDSDGNITIRGNGDFSKFTGVKVDGNL IDKSNYTAKEGSTIITLKASYLNTLSAGTHTVEILWT DGSASTTFTIKANTSDDKPSSGTDKKDDAPKTGDNT PSVWLFILSILSGTGLIITVKKRRENLNS |

In another aspect, the present disclosure provides use of the truncated form of IgA protease described herein, the fusion protein described herein or the pharmaceutical composition described herein in the manufacture of a medicament for treating or preventing a disease associated with IgA deposition.

In another aspect, the present disclosure provides use of an IgA protease or a truncated form thereof, a fusion protein comprising the IgA protease or a truncated form thereof, or a pharmaceutical composition comprising the IgA protease or a truncated form thereof in the manufacture of a medicament for treating or preventing a disease associated with IgA deposition, wherein an amino acid sequence of the IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof. In some embodiments, the amino acid sequence of the IgA protease is formed after removal of the signal peptide sequence of an amino acid sequence as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76. In some embodiments, the truncated form of IgA protease has at least 70% sequence identity (e.g., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to a polypeptide as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76. In some embodiments, the truncated form of IgA protease has at least 70% sequence identity (e.g., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to polypeptide as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76, and still retains the function or activity of the IgA protease (*e.g.,* protein hydrolytic activity, enzymatic activity of specifically cleaving IgA, etc.).

In another aspect, the present disclosure provides a truncated form of IgA protease, fusion protein or pharmaceutical composition described herein for use in treating or preventing a disease associated with IgA deposition.

In another aspect, the present disclosure provides an IgA protease or a truncated form thereof, a fusion protein comprising the IgA protease or a truncated form thereof, or a pharmaceutical composition comprising the IgA protease or a truncated form thereof for use in treating or preventing a disease associated with IgA deposition, wherein the amino acid sequence of IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof. In some embodiments, the amino acid sequence of the IgA protease is formed after removal of the signal peptide sequence of an amino acid sequence as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76. In some embodiments, the truncated form of IgA protease has at least 70% sequence identity (*e.g.*, having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to a polypeptide as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76. In some embodiments, the truncated form of IgA protease has at least 70% sequence identity (e.g., having at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity) to a polypeptide as set forth in SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75 or SEQ ID NO: 76, and still retains the function or activity of the IgA protease (*e.g.*, protein hydrolytic activity, enzymatic activity of specifically cleaving IgA, etc.).

In some embodiments, the disease associated with IgA deposition described herein comprises IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis. In some embodiments, the disease associated with IgA deposition described herein is IgA1 nephropathy. In some embodiments, the disease associated with IgA deposition described herein is IgA vasculitis. In some embodiments, the disease associated with IgA deposition described herein is Kawasaki disease.

### EXAMPLES

The biological materials involved in all examples, such as *E. coli* strains, various cloning and expression plasmids, culture media, tool enzymes, buffers, and various culture methods, protein extraction and purification methods, and other molecular biology manipulations, are familiar to those skilled in the art and can be found in "Molecular Cloning, Sambrook et al. (Laboratory Manual, Cold Spring Harbor, 1989)" and "A Concise Guide to Molecular Biology (F. Osborne *et al.,* translated by Yan Ziying et al., Beijing, Science Press, 1998)".

### Example 1: Study on the shortest active site of AK183 IgA protease

To construct the PET30a-Fc-AK183 plasmid, the inventors removed the N-terminal signal peptide (i.e., amino acids from position 1 to position 30 of SEQ ID NO: 1) and the C-terminal transmembrane region plus the intracellular region (*i.e.,* amino acids from position 1205 to position 1234 of SEQ ID NO: 1) of the wild-type IgA protease from *Clostridium ramosum* strain AK183 (its amino acid sequence is as set forth in SEQ ID NO: 1); the Fc sequence of human IgG1 (HR-CH2-CH3, the amino acid sequence of which is as set forth in SEQ ID NO: 24) was then added to the N-terminus of the amino acid sequence of the IgA protease with the signal peptide, transmembrane region and intracellular region removed (*i.e*., the truncated form of IgA protease consisting of amino acids from position 31 to position1204 of SEQ ID NO: 1).

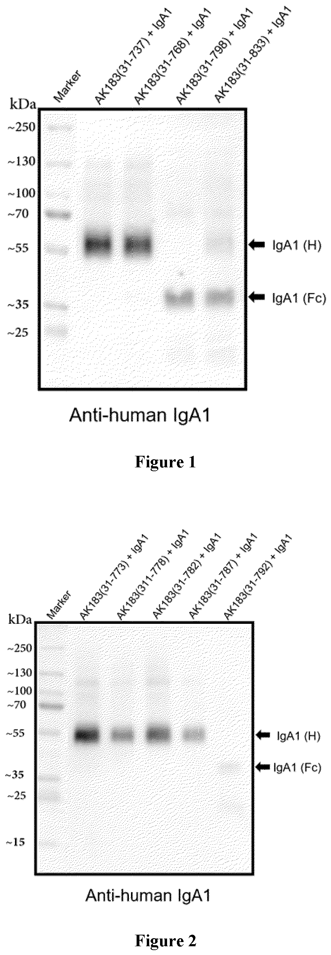

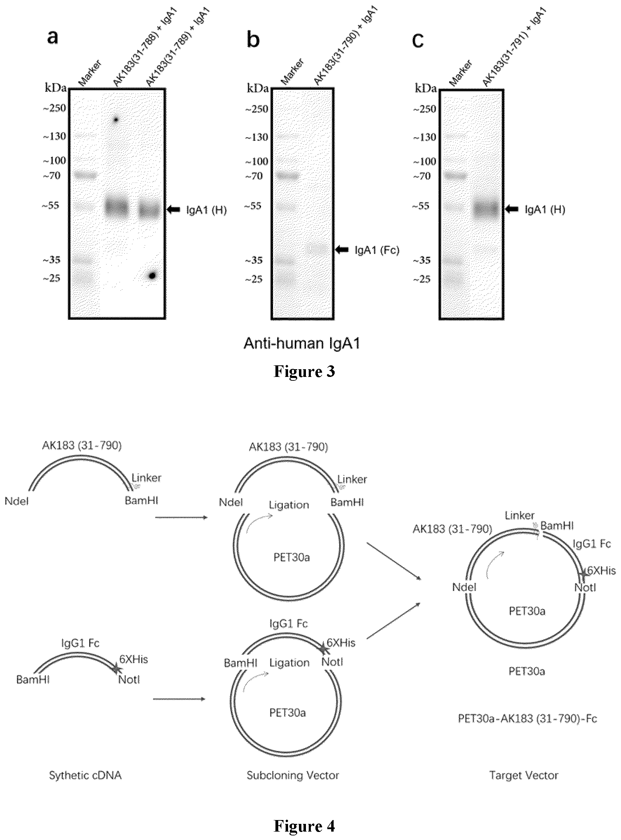

The inventors then used the PET30a-Fc-AK183 plasmid as a template for stop mutations and constructed a series of truncated forms of Fc-AK183 to investigate the shortest active site at the C-terminus of the AK183 IgA protease. Based on the results of the previous study, the inventors concluded that there was a self-cleaving site between amino acids from position 730 to position 840 of the AK183 IgA protease. Therefore, the inventors performed the first round of stop mutations at four amino acid sites respectively, *i.e.,* position 738, position 769, position 799 and position 834 of the AK183 IgA protease, and the results are shown in Figure 1. The results showed that the AK183(31-737) and AK183(31-768) IgA protease truncated fragments obtained after the stop mutation of amino acids at position 738 and position 769 respectively have no *in vitro* self-cleaving activity, while the AK183(31-798) and AK183(31-833) IgA protease truncated fragments obtained after the stop mutation of amino acids at position 799 or position 834 were active. Thus, the first round of stop mutations concluded that the shortest active C-terminal site of AK183 IgA protease was located between amino acids from position 768 to position 798; a second round of stop mutations was then performed at five amino acid sites respectively, *i.e.*, position 774, position 779, position 783, position 788 or position 793 of the AK183 IgA protease, and the results are shown in Figure 2. The truncated fragments of AK183(31-773), AK183(31-778), AK183(31-782), AK183(31-787) IgA protease obtained by stop mutations of amino acids at position 774, position 779, position 783 or position 788 have no *in vitro* self-cleaving activity, while the AK183(31-792) truncated fragment obtained by stop mutation of amino acid at position 793 was still active. Therefore, the second round of stop mutations concluded that the shortest active C-terminal site of AK183 IgA protease was located between amino acids from position 787 to position 792; then, the inventors performed a third round of stop mutations at four amino acid sites respectively, *i.e.*, position 789, position 790, position 791 or position 792 of the AK183 IgA protease, and the results are shown in Figure 3. The AK183(31-788) and AK183(31-789) IgA protease truncated fragments obtained from the amino acid stop mutations at position 789 and position 790 respectively have no *in vitro* self-cleaving activity, while the AK183(31-790) and AK183(31-791) IgA protease truncated fragments obtained from the amino acid stop mutations at position 791 or position 792 respectively were still active (wherein position 791 was incomplete active possibly due to protease conformation problems and only exhibited slight enzymatic cleavage). Therefore, the third round of stop mutations concluded that the shortest C-terminal active fragment of AK183 IgA protease is AK183(31-790).

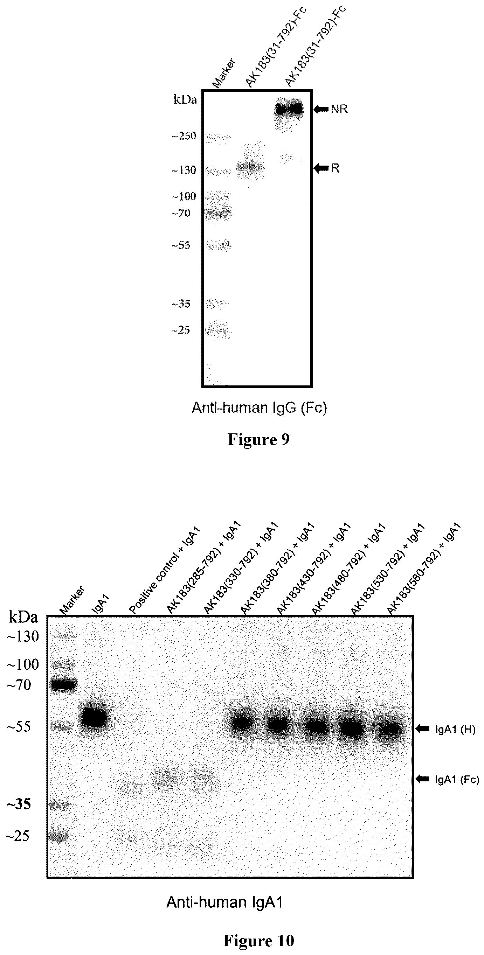

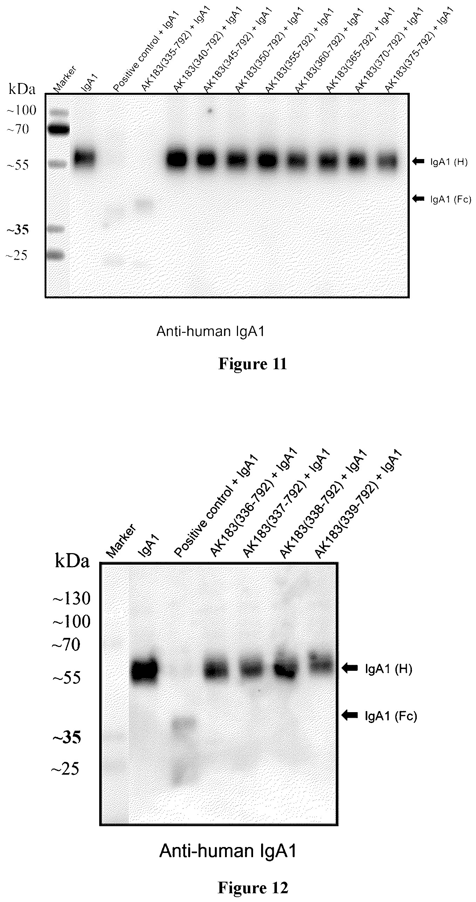

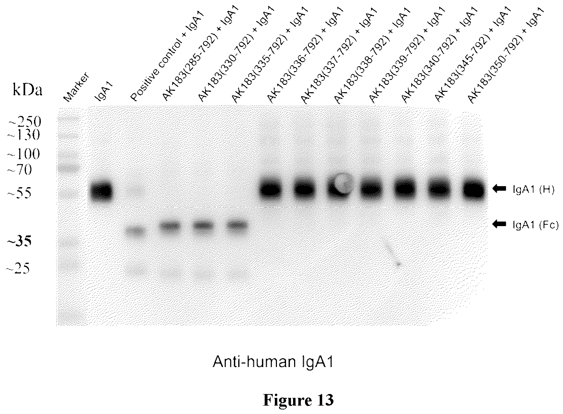

Similarly, the inventors performed three rounds of truncation mutations to investigate the shortest active site at the N-terminus of the AK183 IgA protease. First, the inventors performed a first round of truncation mutations to remove a domain of unknown function (DUF) from the N-terminus of AK183 (31-792), with the C-terminal amino acid site fixed at position 792. For example, AK183(285-792) IgA protease truncated fragment was obtained by removing the N-terminal DUF corresponding to amino acids from position 31 to position 284 of SEQ ID NO: 1. A similar approach was taken to obtain AK 183 (33 0-792), AK183 (3 80-792), AK183(430-792), AK183(480-792), AK183(530-792), AK183(580-792) IgA protease truncated fragments, respectively. The results of the in vitro enzymatic cleavage activity assay of the resulting IgA protease truncated fragments against IgA1 are shown in Figure 10. As shown in Figure 10, AK183(285-792), AK183(330-792) IgA protease truncated fragments still had *in vitro* enzymatic activity, while AK183(380-792), AK183(430-792), AK183(480-792), AK183(530-792), AK183(580-792) IgA protease truncated fragments had no *in vitro* enzymatic activity. Thus, the first round of truncation mutations concluded that the shortest active N-terminal site of the AK183 IgA protease was located between amino acids from position 330 to position 380. A second round of truncation mutations was then carried out, with a truncated form being constructed every five amino acids between amino acids from position 330 to position 380, resulting in AK183(335-792), AK183(340-792), AK183(345-792), AK183(350-792), AK183(355-792) AK183(360-792), AK183(365-792), AK183(370-792), AK183(375-792) IgA protease truncated fragments. The results of the *in vitro* enzymatic cleavage activity assay of the obtained IgA protease truncated fragments against IgA1 are shown in Figure 11. As shown in Figure 11, AK183(335-792) IgA protease truncated fragment still had *in vitro* enzymatic cleavage activity, while AK183(340-792), AK183(345-792), AK183(350-792), AK183(355-792), AK183(360-792), AK183(365-792), AK183(370-792), AK183(375-792) IgA protease truncated fragments had no *in vitro* enzymatic cleavage activity. Thus, the second round of truncation mutations concluded that the shortest active N-terminal site of the AK183 IgA protease was located between amino acids from position 335 to position 340. Then, the inventors performed a third round of truncation mutations to construct truncated forms by every amino acid between amino acids from position 335 to position 340 to obtain AK183(336-792), AK183(337-792), AK183(338-792), and AK183(339-792) IgA protease truncated fragments, respectively. The results of the *in vitro* enzymatic cleavage activity assay of the obtained IgA protease truncated fragments against IgA1 are shown in Figure 12. As shown in Figure 12, AK183(336-792), AK183(337-792), AK183(338-792) and AK183(339-792) IgA protease truncated fragments had no *in vitro* enzymatic cleavage activity. Therefore, the third round of truncation mutations concluded that the shortest N-terminal active site of AK183 IgA protease is located at amino acid position 335. Finally, the inventors validated the results of the three rounds again by re-expressing AK183(285-792), AK183(330-792), AK183(335-792), AK183(336-792), AK183(337-792), AK183(338-792), AK183(339-792), AK183(340-792), AK183(345-792), AK183(350-792) IgA protease truncated fragments at the same time. The results of the *in vitro* enzymatic cleavage activity assay of the obtained IgA protease truncated fragments against IgA1 are shown in Figure 13. As shown in Figure 13, AK183(285-792), AK183(330-792), AK183(335-792) IgA protease truncated fragments still had *in vitro* enzymatic cleavage activity, while AK183(336-792), AK183(337-792), AK183(338-792), AK183(339-792), AK183(340-792), AK183(345-792), AK183(350-792) IgA protease truncated fragments had no *in vitro* enzymatic cleavage activity, which was consistent with the conclusion of the previous three rounds of truncation mutations, *i.e.*, that the shortest active N-terminal site of AK183 IgA protease is located at amino acid position 335.

In summary, the shortest active fragment of AK183 IgA protease is AK183(335-790).

### Example 2: Preparation of fusion protein comprising the truncated form of AK183 IgA protease or the full length of AK183 IgA protease

### 2.1 Plasmid Construction

After identifying the shortest C-terminal active fragment of AK183 IgA protease (AK183(31-790)), in order to construct the PET30a-AK183(31-790)-Fc plasmid, the inventors placed the Fc domain at the C-terminus of amino acid position 790 of AK183 IgA protease, with GGGGS ligated in the middle and a 6XHis tag at the C-terminus of Fc for protein purification, and the construction flow is shown in Figure 4. Then, the inventors used the PET30a-AK183(31-790)-Fc plasmid as a template and constructed the PET30a-AK183(31-792)-Fc plasmid by adding the 791st and 792nd amino acids after the truncated form of AK183(31-790) by PCR.

Meanwhile the inventors commissioned Beijing Liuhe BGI Science and Technology Co. Ltd. to construct four alternative subclones, PET30a-AK183(31-798)-Fc, PET30a-AK183(31-807)-Fc, PET30a-AK183(31-816)-Fc and PET30a-AK183(31-833)-Fc. The hinge region of Fc (CH2-CH3) of the alternative subclones was removed and amino acid sequence of Fc (CH2-CH3) of the alternative subclones is set forth in SEQ ID NO: 6 (compared to SEQ ID NO: 2, the first 9 amino acids (EPKSCDKTH) of SEQ ID NO: 2 is absent in SEQ ID NO: 6) and 10 His were added between the truncated form of IgA protease and Fc (located between the linker GGGGS and Fc). Four alternative subclones were used as alternatives for later protease yield and purity screening.

To study whether the way in which the truncated form of the AK183 IgA protease is linked to the Fc region affects its enzymatic cleavage activity against IgA, the inventors further constructed two alternative subclones, PET30a-AK183(285-816)-Fc and PET30a-Fc-AK183(285-816), wherein the amino acid sequence of Fc is as set forth in SEQ ID NO: 25.

To compare the enzymatic cleavage activity against IgA of the fusion protein formed by the truncated fragment of AK183 IgA protease with Fc and the fusion protein formed by the full length of AK183 IgA protease with Fc, the inventors further constructed the alternative subclone PET30a-Fc-AK183 (31-1203), wherein the amino acid sequence of Fc is as set forth in SEQ ID NO: 24.

To study the effects of IgG1 Fc, IgG4 Fc and albumin on IgA enzymatic cleavage activity of the fusion protein comprising the truncated form of AK183 IgA protease, the inventors further constructed two alternative subclones, PET30a-AK183(31-816)-IgG4 Fc, PET30a-AK183(31-816)-albumin, wherein the amino acid sequence of IgG4 Fc is as set forth in in SEQ ID NO: 77 and the amino acid sequence of albumin is as set forth in SEQ ID NO: 60.

To study the effect of different linkers on the IgA enzymatic cleavage activity of the fusion protein comprising the truncated form of AK183 IgA protease, the inventors further constructed six alternative subclones PET30a-AK183(285-816)-linker-Fc. Among the fusion proteins expressed by these six alternative subclones, except for the different linkers, the amino acid sequences of AK183(285-816) and Fc were identical, wherein the amino acid sequence of AK183 (285-816) is as set forth in SEQ ID NO: 46 and the amino acid sequence of Fc is as set forth in SEQ ID NO: 25, while the amino acid sequences of the linkers were HHHHHHHHHH (SEQ ID NO: 59, also known as "10xHis"), EEKKKEKEKEEQEERETK (SEQ ID NO: 58, also known as "IgD linker"), GGGGS (SEQ ID NO: 22, also known as "1xlinker"), GGGGSGGGGS (SEQ ID NO: 78, also known as a "2xlinker"), GGGGSGGGGSGGGGS (SEQ ID NO: 79, also known as a "3xlinker") and GGGGSGGGGSGGGGSGGGGS (SEQ ID NO: 80, also known as "4xlinker"), respectively.

### 2.2 Preparation Method of Fusion Proteins

The expression vector was transfected into *E. Coli* (BL21-DE3) competent cells and selected for resistance by LB agar dishes containing 50ug/ml of kanamycin, and then the monoclonal colonies were picked into LB medium containing the corresponding antibiotics and shaken until the exponential growth period (OD600: 0.6-0.8). After the exponential growth period was achieved, 0.1-0.5 mM of isopropyl-β-D-thiogalactoside (IPTG) was added to induce expression at 16°C for 24h. After completion of expression, the *E. coli* cells was sonicated and centrifuged at high speed according to conventional methods. The supernatant was retained and then purified by affinity chromatography and molecular sieve purification to obtain the recombinant fusion protein.

The amino acid sequence of the AK183(31-792)-Fc fusion protein expressed by the PET30a-AK183(31-792)-Fc plasmid is as set forth in SEQ ID NO: 2 and its encoding nucleic acid sequence is as set forth in SEQ ID NO: 3.

The amino acid sequence of the AK183(31-798)-Fc fusion protein expressed by the PET30a-AK183(31-798)-Fc plasmid is as set forth in SEQ ID NO: 6 and its encoding nucleic acid sequence is as set forth in SEQ ID NO: 7.

The amino acid sequence of the AK183(31-807)-Fc fusion protein expressed by the PET30a-AK183(31-807)-Fc plasmid is as set forth in SEQ ID NO: 8 and its encoding nucleic acid sequence is as set forth n in SEQ ID NO: 9.

The amino acid sequence of the AK183(31-816)-Fc fusion protein expressed by the PET30a-AK183(31-816)-Fc plasmid is as set forth in SEQ ID NO: 10 and its encoding nucleic acid sequence is as set forth in SEQ ID NO: 11.

The amino acid sequence of the AK183(31-833)-Fc fusion protein expressed by the PET30a-AK183(31-833)-Fc plasmid is as set forth in SEQ ID NO: 12 and its encoding nucleic acid sequence is as set forth in SEQ ID NO: 13.

The amino acid sequence of the AK183(285-816)-Fc fusion protein expressed by the PET30a-AK183(285-816)-Fc plasmid is as set forth in SEQ ID NO: 81.

The amino acid sequence of the Fc-AK183(285-816) fusion protein expressed by the PET30a-Fc-AK183(285-816) plasmid is as set forth in SEQ ID NO: 82.

The amino acid sequence of the Fc-AK183(31-1203) fusion protein expressed by the PET30a-Fc-AK183(31-1203) plasmid is as set forth in SEQ ID NO: 83.

The amino acid sequence of the AK183(31-816)-IgG4 Fc fusion protein expressed by the PET30a-AK183(31-816)-IgG4 Fc plasmid is as set forth in SEQ ID NO: 84.

The amino acid sequence of the AK183(31-816)-albumin fusion protein expressed by the PET30a-AK183(31-816)-albumin plasmid is as set forth in SEQ ID NO: 85.

### 2.3 In Vitro Activity Assay Method

The obtained fusion protein comprising the truncated form of AK183 IgA protease was mixed *in vitro* with the substrate IgA1 purified from the plasma of patients with IgA nephropathy and reacted at 37°C for 2~12h, followed by Western blot to verify its enzymatic activity against the substrate IgA1.

### 2.4 In Vivo Activity Assay Method

The obtained fusion protein comprising the truncated form of AK183 IgA protease was injected into humanized IgA1 alpha chain knock-in (α1KI-Tg) C57BL/6 mice via tail vein and blood samples were collected before injection, 5 min, 2h, 4h and 24h after injection, followed by Western blot validation.

### 2.5 Results

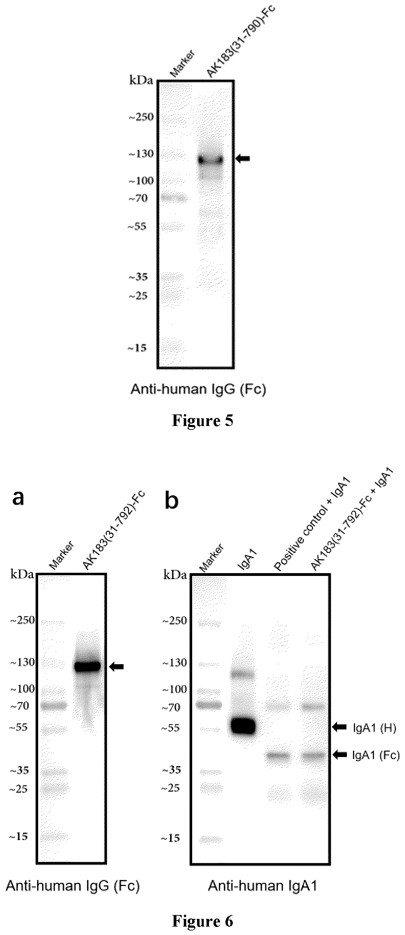

The assay showed that the PET30a-AK183(31-790)-Fc plasmid successfully expressed the AK183(31-790)-Fc fusion protein (as shown in Figure 5). Also, the AK183(31-792)-Fc fusion protein had the expected full-length protein expression (as shown in Figure 6a) and also had *in vitro* enzymatic activity against IgA1 (as shown in Figure 6b).

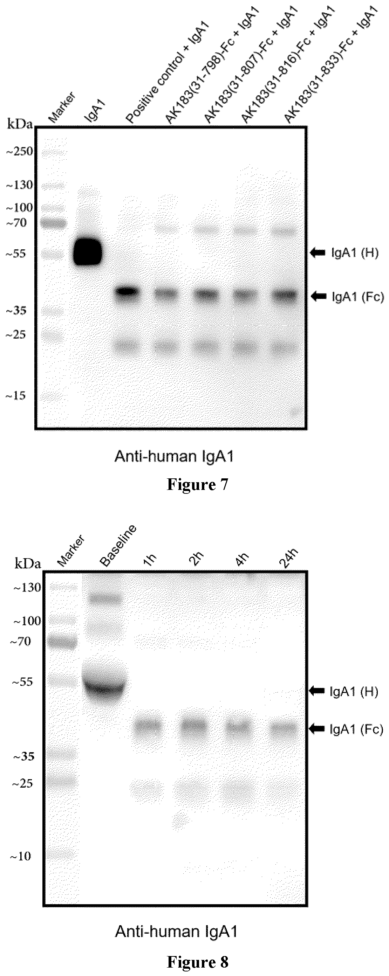

The four alternative subclones PET30a-AK183(31-798)-Fc, PET30a-AK183(31-807)-Fc, PET30a-AK183(31-816)-Fc and PET30a-AK183(31-833)-Fc all expressed the fusion protein and all of them had *in vitro* enzymatic cleavage activity against IgA1 (as shown in Figure 7).

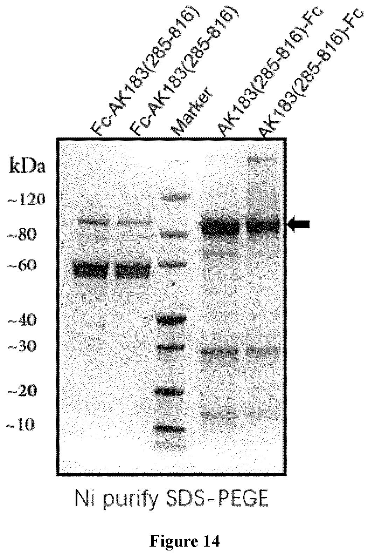

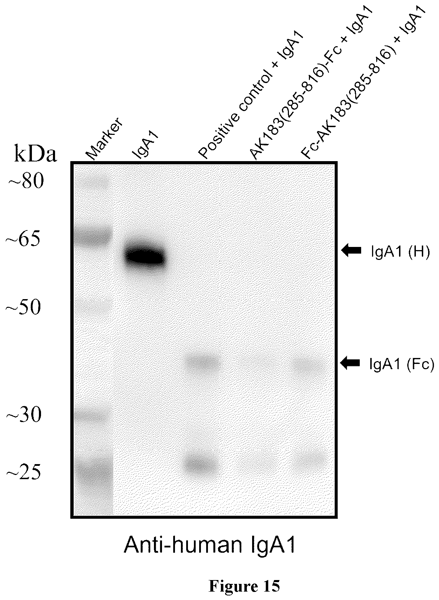

In addition, subclones PET30a-AK183(285-816)-Fc, PET30a-Fc-AK183(285-816) both expressed the fusion proteins (as shown in Figure 14) and both of them had *in vitro* enzymatic cleavage activity (as shown in Figure 15).

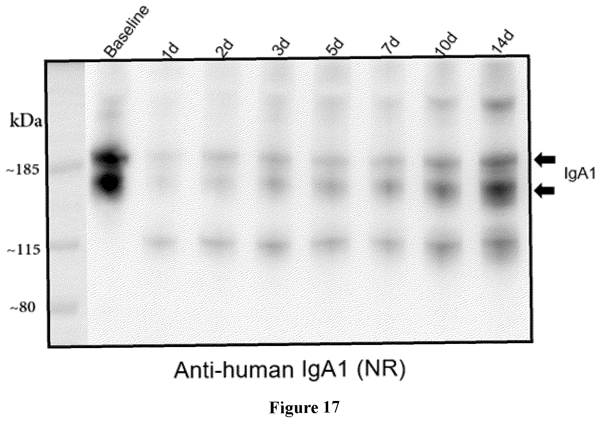

The inventors also verified the *in vivo* activity of the AK183(31-807)-Fc fusion protein expressed by subclone PET30a-AK183(31-807)-Fc and the Fc-AK183(285-816) fusion protein expressed by subclone PET30a-Fc-AK183(285-816). The results are as shown in Figure 8 (AK183(31-807)-Fc, under reducing conditions) and Figure 17 (Fc-AK183(285-816), under non-reducing conditions). As shown in Figure 8, after humanized IgA1 mice (α1KI-Tg) C57BL/6 receiving a single tail vein injection of AK183(31-807)-Fc fusion protein, all intact IgA1 heavy chain (H) in the blood disappeared and persisted until at least 24 h. As shown in Figure 17, after humanized IgA1 mice (α1KI-Tg) C57BL/6 receiving a single tail vein injection of Fc-AK183 (285-816) fusion protein, intact IgA1 heavy chain (H) in the blood disappeared, and persisted until at least 2 weeks.

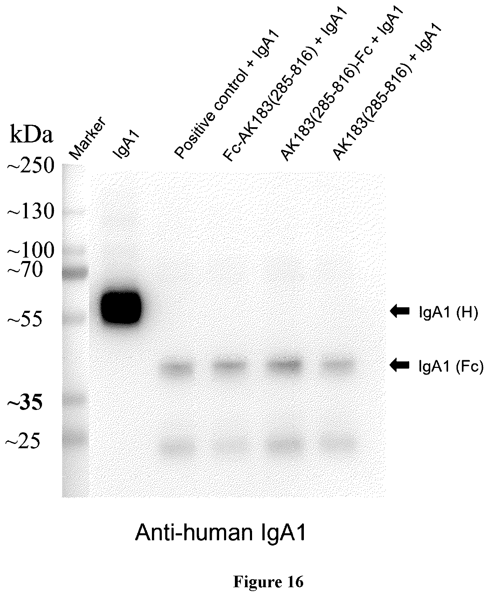

The inventors also compared the enzymatic cleavage activity of Fc-AK183(285-816) fusion protein, AK183(285-816)-Fc fusion protein, and truncated form of AK183(285-816) IgA protease against IgA1, and the results are shown in Figure 16. As shown in Figure 16, all three proteins had enzymatic cleavage activity against IgA1.

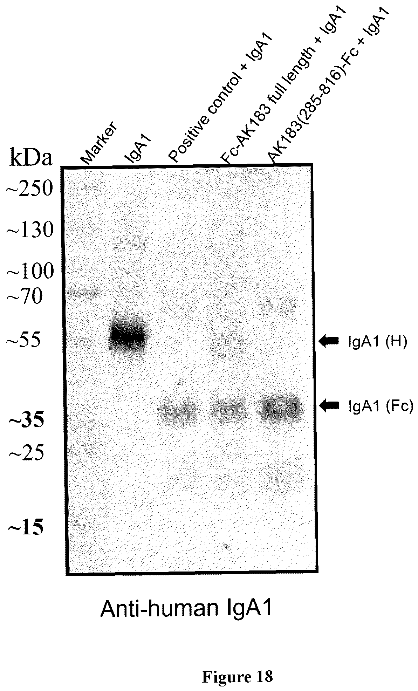

The inventors also compared the enzymatic cleavage activity of AK183(285-816)-Fc fusion protein and Fc-AK183(31-1203) fusion protein on IgA1, and the results are shown in Figure 18. As shown in Figure 18, AK183(285-816)-Fc fusion protein and Fc-AK183(31-1203) fusion protein both have enzymatic cleavage activity against IgA1.

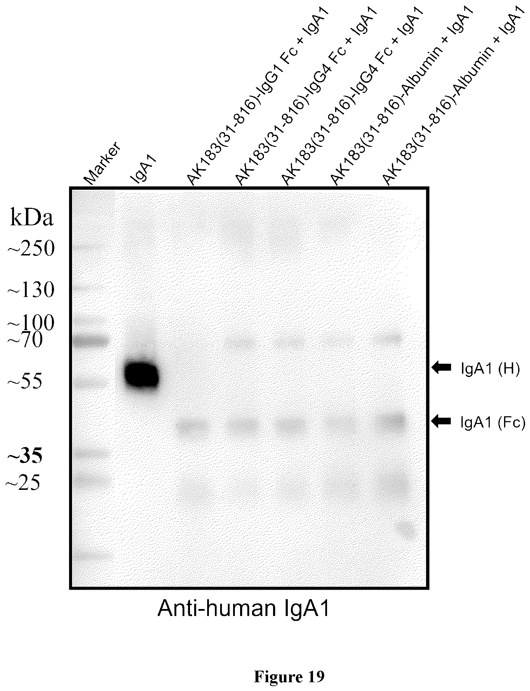

The inventors also compared the enzymatic cleavage activity of AK183(31-816)-IgG1 Fc fusion protein, AK183(31-816)-IgG4 Fc fusion protein and AK183(31-816)-albumin fusion protein against IgA1, and the results are shown in Figure 19. As shown in Figure 19, all three fusion proteins had enzymatic cleavage activity against IgA1.

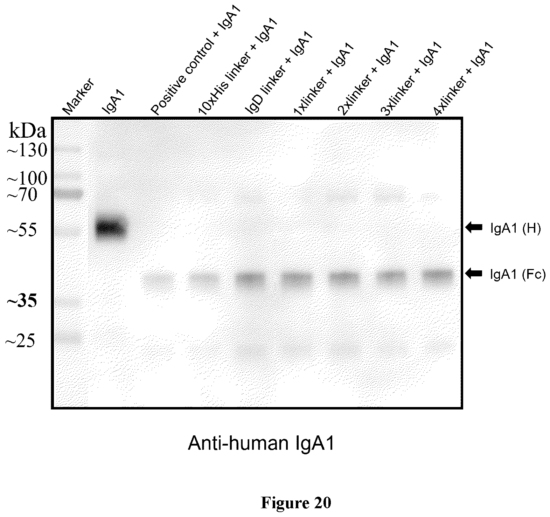

The inventors also compared the enzymatic cleavage activity of AK183(285-816)-Fc fusion proteins with different linkers (10xHis, IgD linker, 1xlinker, 2xlinker, 3xlinker or 4xlinker) against IgA1, and the results are shown in Figure 20. As shown in Figure 20, all six fusion proteins had enzymatic cleavage activity against IgA1.

### 2.6 Eukaryotic Expression System

The aforementioned experiments were performed in *E*. *Coli* (BL21-DE3) competent cells (i.e., prokaryotic expression system). Next, the inventors cloned the AK183(31-792)-Fc fusion cDNA sequence into the pcDNA3.1/hygro(+) expression vector with the N-terminus of the fusion protein being added with ATGTACAGGATGCAACTCCTGTCTTGCATTGCACTAAGTCTTGCACTTGTC ACGAATTCG (SEQ ID NO: 41) that encodes and expresses a human IL-2 signal peptide. The pcDNA3.1/hygro(+)-IL2-AK183(31-792)-Fc plasmid was thereby constructed and used to transfect eukaryotic expression system HEK293 cells. Codon optimization was performed for Fc sequence against the eukaryotic expression system. The amino acid sequence of the IL2-AK183(31-792)-Fc fusion protein expressed by pcDNA3.1/hygro(+)-IL2-AK183(31-792)-Fc is as set forth in SEQ ID NO: 4 and its encoding nucleic acid sequence is as set forth in SEQ ID NO: 5.

The results of AK183(31-792)-Fc fusion protein expression in HEK293 cells are shown in Figure 9. The results indicate that the AK183(31-792)-Fc fusion protein had the expected full-length expression and that there was dimeric form (dimer) of fusion protein expressed in the eukaryotic system.

### Example 3: Preparation and Activity Assay of AK183 IgA Protease Mutants

The inventors conducted site-directed mutagenesis to the truncated form of AK183(31-1203) IgA protease at position 844, position 862, position 931 and position 933, position 978, position 1002 and position 1004 (aforementioned positions were numbered relative to SEQ ID NO: 1), respectively, in particular, proline (P) at these positions were mutated to glycine (G). The site-directed mutagenesis resulted in five mutants of truncated forms of AK183(31-1173) IgA protease with amino acid sequences as set forth in SEQ ID NO: 53 (also known as "PA-GA Mut"), SEQ ID NO: 54 (also known as "PI-GI Mut"), SEQ ID NO: 55 (also known as "PAP-GAG Mut"), SEQ ID NO: 56 (also known as "PAT-GAT Mut") and SEQ ID NO: 57 ((also known as "PIP-GIG Mut"), respectively.

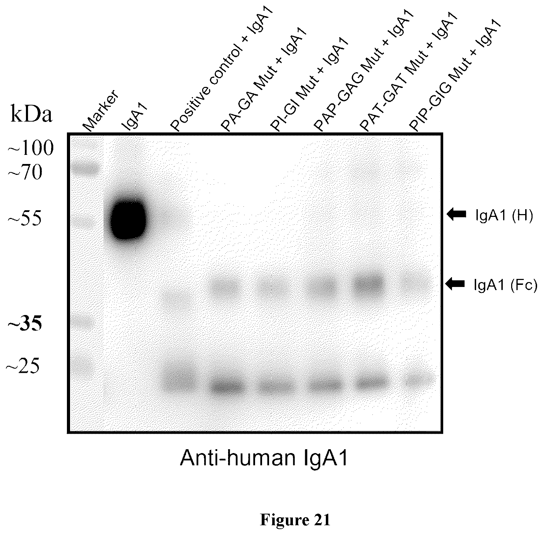

The inventors tested the enzymatic cleavage activity of each of these five mutants against IgA1 and the results are shown in Figure 21. As shown in Figure 21, all five mutants had enzymatic cleavage activity against IgA1.

In addition, based on the amino acid sequence of the AK183(31-816)-Fc fusion protein (*i.e.,* SEQ ID NO: 10) prepared in Example 2, the inventors conducted site-directed mutagenesis at position 7 of its Fc region (this position was numbered relative to SEQ ID NO: 25), in particular, alanine (A) at this position was mutated to valine (V), glycine (G), serine (S), and leucine (L) to obtain four mutants of the AK183(31-816)-Fc fusion protein, referred to as A-V Mut, A-G Mut, A-S Mut and A-L Mut, respectively.

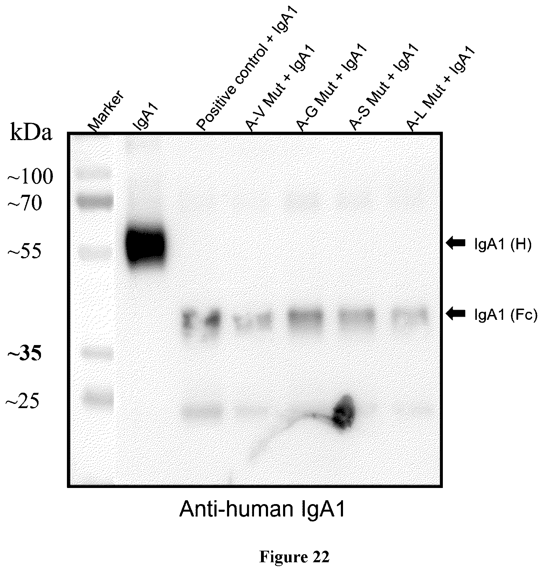

The inventors tested the enzymatic cleavage activity of each of these four mutants against IgA1 and the results are shown in Figure 22. As shown in Figure 22, all four mutants had enzymatic cleavage activity against IgA1.

### Example 4: Exploring Other IgA Proteases

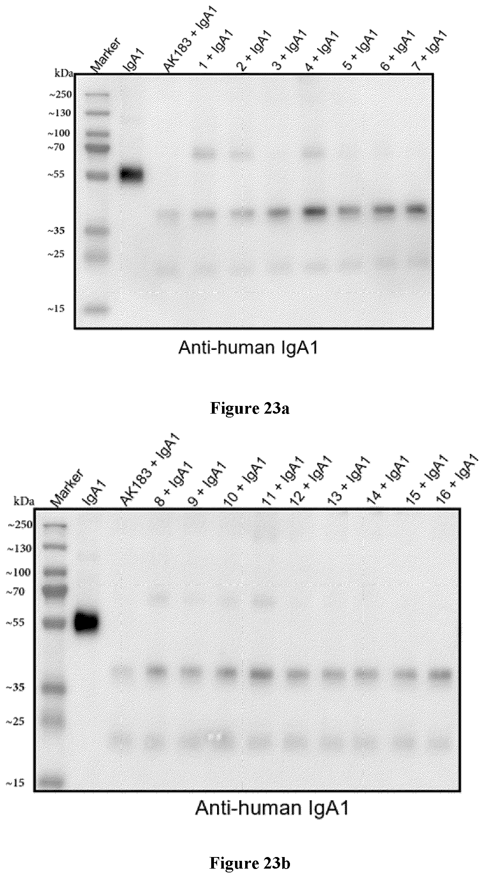

The inventors screened several amino acid sequences from the metagenomic database with some homology to the wild-type IgA enzyme of AK183 and synthesized sixteen (16) AK183 homologous enzymes. Their amino acid sequences are as set forth in SEQ ID NO: 61 ~ SEQ ID NO: 76, respectively. The inventors tested the enzymatic cleavage activity of each of these AK183 homologous enzymes against IgA1 according to the method of *in vitro* activity assay described in Example 2.3. The results are shown in Figure 23a and Figure 23b. In Figure 23a, "1 + IgA1" indicates that the peptide as set forth in SEQ ID NO: 61 was mixed *in vitro* with the substrate IgA1, "2 + IgA1" indicates that the peptide as set forth in SEQ ID NO: 62 was mixed *in vitro* with the substrate IgA1, and so on, "16 + IgA1" in Figure 23b indicates that the peptide as set forth in SEQ ID NO: 76 was mixed *in vitro* with the substrate IgA1.

As shown in Figures 23a and 23b, the polypeptides as set forth in SEQ ID NO: 61 ~ 76 all had enzymatic cleavage activity against IgA1.

Although the present disclosure presents and describes the invention in a particular manner by reference to particular examples, it should be understood by those skilled in the art that the disclosure above may be subject to various variations in form and detail without departing from the main concept and scope of protection disclosed in the present disclosure.

## Claims

1. An isolated truncated form of IgA protease comprising a non-natural truncated fragment of a wild-type IgA protease obtained from or derived from *Clostridium ramosum* or having at least 70% sequence identity to the non-natural truncated fragment.

2. The truncated form of IgA protease of claim 1, wherein the non-natural truncated fragment has an amino acid substitution, deletion, insertion or modification compared to the wild-type IgA protease of *Clostridium ramosum,* such that the truncated form of IgA protease loses or reduces its self-cleaving function.

3. The truncated form of IgA protease of claim 2, wherein the amino acid substitution, deletion, insertion or modification occurs at a natural self-cleaving site of the wild-type IgA protease of *Clostridium ramosum,* within 5 sites upstream and/or within 5 sites downstream of the natural self-cleaving site.

4. The truncated form of IgA protease of any one of claims 1 to 3, wherein the non-natural truncated fragment is a N-terminal or C-terminal truncated fragment of a wild-type IgA protease obtained from or derived from *Clostridium ramosum.*

5. The truncated form of IgA protease of any one of the preceding claims, wherein the *Clostridium ramosum* is *Clostridium ramosum* strain AK183.

6. The truncated form of IgA protease of claim 4 or 5, wherein the N-terminal truncated fragment comprises a polypeptide fragment of at least 760 continuous amino acids starting from position 31 of the N-terminus of a wild-type IgA protease obtained from or derived from *Clostridium ramosum,* or having at least 70% sequence identity to the polypeptide fragment.

7. The truncated form of IgA protease of any one of the preceding claims, wherein the non-natural truncated fragment comprises a polypeptide fragment of at least 456 continuous amino acids starting from position 335 of the N-terminus of a wild-type IgA protease obtained from or derived from *Clostridium ramosum,* or having at least 90% or at least 95% sequence identity to the polypeptide fragment.

8. The truncated form of IgA protease of any one of the preceding claims, wherein an amino acid sequence of the wild-type IgA protease of *Clostridium ramosum* is as set forth in SEQ ID NO: 1.

9. The truncated form of IgA protease of any one of claims 3 to 8, wherein the natural self-cleaving site is between position 730 and position 840 (e.g., between position 792 and position 797) of the amino acid sequence as set forth in SEQ ID NO: 1.

10. The truncated form of IgA protease of claim 9, wherein the natural self-cleaving site is at position 790, position 791, position 792, position 793, position 794, position 795, position 796, position 797, position 798, position 799 or position 800 of the amino acid sequence as set forth in SEQ ID NO: 1.

11. The truncated form of IgA protease of any one of the preceding claims, comprising a polypeptide fragment of at least 760 (*e.g.*, at least 761, at least 762, at least 763, at least 764, at least 765, at least 766, at least 767, at least 768, at least 769, at least 770, at least 771, at least 772, at least 773, at least 774, at least 775, at least 776, at least 777, at least 778, at least 779, at least 780, at least 781, at least 782, at least 783, at least 784, at least 785, at least 786, at least 787, at least 788, at least 789, at least 790, at least 791, at least 792, at least 793, at least 794, at least 795, at least 796, at least 797, at least 798, at least 799, at least 800, at least 801, at least 802, at least 803, at least 804, at least 805, at least 806, at least 807, at least 808, at least 809, at least 810, at least 900, at least 950, at least 1000, at least 1100, at least 1150 or at least 1200) continuous amino acids starting from position 31 of the amino acid sequence as set forth in SEQ ID NO: 1.

12. The truncated form of IgA protease of any one of the preceding claims, comprising a polypeptide fragment selected from the group consisting of amino acids from position 31 to position 790 of the amino acid sequence as set forth in SEQ ID NO: 1, amino acids from position 31 to position 792 of the amino acid sequence as set forth in SEQ ID NO: 1, amino acids from position 31 to position 798 of the amino acid sequence as set forth in SEQ ID NO: 1, amino acids from position 31 to position 807 of the amino acid sequence as set forth in SEQ ID NO: 1, amino acids from position 31 to position 816 of the amino acid sequence as set forth in SEQ ID NO: 1, amino acids from position 31 to position 833 of the amino acid sequence as set forth in SEQ ID NO: 1, and a polypeptide fragment having at least 70% sequence identity thereto.

13. The truncated form of IgA protease of any one of the preceding claims, comprising a polypeptide fragment of at least 456 (e.g., at least 457, at least 458, at least 459, at least 460, at least 461, at least 462, at least 463, at least 464, at least 465, at least 466, at least 467, at least 468, at least 469, at least 470, at least 471, at least 472, at least 473, at least 474, at least 475, at least 476, at least 477, at least 478, at least 479, at least 480, at least 481, at least 482, at least 483, at least 484, at least 485, at least 486, at least 487, at least 488, at least 489, at least 490, at least 491, at least 492, at least 493, at least 494, at least 495, at least 496, at least 497, at least 498, at least 499, at least 500, at least 550, at least 600, at least 650, at least 700, at least 750, at least 800, at least 850 or at least 900) continuous amino acids starting from position 335 of the amino acid sequence as set forth in SEQ ID NO: 1.

14. The truncated form of IgA protease of claim 13, comprising a polypeptide fragment selected from the group consisting of amino acids from position 335 to position 790 of the amino acid sequence as set forth in SEQ ID NO: 1, amino acids from position 335 to position 791 of the amino acid sequence as set forth in SEQ ID NO: 1, amino acids from position 335 to position 792 of the amino acid sequence as set forth in SEQ ID NO: 1, amino acids from position 285 to position 790 of the amino acid sequence as set forth in SEQ ID NO: 1, amino acids from position 285 to position 791 of the amino acid sequence as set forth in SEQ ID NO: 1, amino acids from position 285 to position 792 of the amino acid sequence as set forth in SEQ ID NO: 1, amino acids from position 330 to position 790 of the amino acid sequence as set forth in SEQ ID NO: 1, amino acids from position 330 to position 791 of the amino acid sequence as set forth in SEQ ID NO: 1, amino acids from position 330 to position 792 of the amino acid sequence as set forth in SEQ ID NO: 1, amino acids from position 285 to position 816 of the amino acid sequence as set forth in SEQ ID NO: 1, and a polypeptide sequence having at least 90% or at least 95% sequence identity thereto.

15. The truncated form of IgA protease of any one of the preceding claims, having an amino acid conservative substitution at one or more sites compared to the amino acid sequence of the polypeptide fragment.

16. The truncated form of IgA protease of any one of the preceding claims, wherein an amino acid mutation occurs at one or more sites of the polypeptide fragment, wherein the one or more sites correspond to position 844, position 862, position 931, position 933, position 978, position 1002, and/or position 1004 of SEQ ID NO: 1.

17. The truncated form of IgA protease of claim 16, wherein one or more sites of the polypeptide fragment are mutated to glycine, wherein the one or more sites correspond to position 844, position 862, position 931, position 933, position 978, position 1002, and/or position 1004 of SEQ ID NO: 1.

18. The truncated form of IgA protease of claim 16 or 17, wherein the polypeptide fragment has an amino acid mutation at position 844, an amino acid mutation at position 862, amino acid mutations at positions 931 and 933, an amino acid mutation at position 978, or amino acid mutations at positions 1002 and 1004 corresponding to SEQ ID NO: 1.

19. The truncated form of IgA protease of any one of claims 16 to 18, wherein the amino acid sequence of the polypeptide fragment is as set forth in SEQ ID NO: 53 (also referred to as "PA-GA Mut"), SEQ ID NO: 54 (also referred to as "PI-GI Mut"), SEQ ID NO: 55 (also referred to as "PAP-GAG Mut"), SEQ ID NO: 56 (also referred to as "PAT-GAT Mut") or SEQ ID NO: 57 (also referred to as "PIP-GIG Mut").

20. The truncated form of IgA protease of any one of the preceding claims, having an enzymatic activity of specifically cleaving human IgA.

21. The truncated form of IgA protease of claim 20, having an enzymatic activity of specifically cleaving human IgA heavy chain.

22. The truncated form of IgA protease of claim 21, having an enzymatic activity of specifically cleaving the intersection of human IgA heavy chain CH1 and hinge region.

23. The truncated form of IgA protease of any one of claims 20 to 22, having an enzymatic activity of specifically cleaving human IgA1.

24. A fusion protein comprising a first polypeptide and a second polypeptide, wherein the first polypeptide comprises a full-length wild-type IgA protease obtained from or derived from *Clostridium ramosum,* a polypeptide formed by removing a signal peptide of a wild-type IgA protease obtained from or derived from *Clostridium ramosum,* or the truncated form of IgA protease of any one of the preceding claims; the second polypeptide comprising an amino acid sequence for extending half-life of the first polypeptide in a subject.

25. The fusion protein of claim 24, wherein the first polypeptide comprises a sequence as set forth in SEQ ID NO: 1 or SEQ ID NO: 42.

26. The fusion protein of claim 24, wherein the second polypeptide is located at N-terminus or C-terminus of the first polypeptide.

27. The fusion protein of any one of claims 24 to 26, wherein the first polypeptide and the second polypeptide are linked via a linker.

28. The fusion protein of any one of claims 24 to 26, wherein the first polypeptide and the second polypeptide are directly linked to each other.

29. The fusion protein of claim 27, wherein the linker is selected from the group consisting of a cleavable linker, a non-cleavable linker, a peptide linker, a flexible linker, a rigid linker, a helical linker and a non-helical linker.

30. The fusion protein of claim 29, wherein the linker comprises a peptide linker.

31. The fusion protein of claim 30, wherein the peptide linker comprises a linker comprising a glycine and a serine.

32. The fusion protein of claim 31, wherein the linker comprising a glycine and a serine comprises one, two, three, four or more repeats of a sequence as set forth in SEQ ID NO: 21 (GGGS), SEQ ID NO: 22 (GGGGS), SEQ ID NO: 86 (GGGGGS) or SEQ ID NO: 87 (GGGGGGGS).

33. The fusion protein of claim 30, wherein the linker comprises an amino acid sequence as set forth in SEQ ID NO: 23 (GGCGGCGGTGGATCC), SEQ ID NO: 58 (EEKKKEKEKEEQEERETK) or SEQ ID NO: 59 (HHHHHHHHHH).

34. The fusion protein of any one of claims 24 to 33, wherein the second polypeptide is selected from an Fc domain and albumin.

35. The fusion protein of claim 34, wherein the Fc domain comprises a hinge region.

36. The fusion protein of claim 35, wherein the Fc domain is derived from human IgG Fc domain.

37. The fusion protein of claim 36, wherein the Fc domain is derived from human IgG1 Fc domain, human IgG2 Fc domain, human IgG3 Fc domain or human IgG4 Fc domain.

38. The fusion protein of any one of claim 34 to 37, wherein the Fc domain comprises an amino acid sequence having at least 70%, at least 75%, at least 80%, at least 85%, at least 90% or at least 95% sequence identity to SEQ ID NO: 24, SEQ ID NO: 25, SEQ ID NO: 32 or SEQ ID NO: 77.

39. The fusion protein of claim 38, wherein the Fc domain comprises an amino acid sequence as set forth in SEQ ID NO: 24, SEQ ID NO: 25, SEQ ID NO: 32 or SEQ ID NO: 77.

40. The fusion protein of claim 39, wherein the Fc domain has an amino acid mutation at a site corresponding to position 7 of SEQ ID NO: 25.

41. The fusion protein of claim 40, wherein amino acid (*e.g.*, alanine) at a site of the Fc domain is mutated to valine, glycine, serine or leucine, wherein the site corresponds to position 7 of SEQ ID NO: 25.

42. The fusion protein of any one of claims 34 to 41, wherein the Fc domain comprises one or more mutations that extend half-life of the fusion protein.

43. The fusion protein of any one of claims 34 to 42, wherein the Fc domain is linked to C-terminus or N-terminus of the first polypeptide.

44. The fusion protein of claim 34, wherein the albumin comprises one or more domains of human serum albumin.

45. The fusion protein of claim 44, wherein the albumin comprises a D3 domain of human serum albumin.

46. The fusion protein of any one of claims 24 to 45, further comprising a label.

47. The fusion protein of claim 46, wherein the label is selected from the group consisting of a fluorescent label, a luminescent label, a purification label and a chromogenic label.

48. The fusion protein of claim 46 or 47, wherein the label is selected from the group consisting of a c-Myc tag, an HA tag, a VSV-G tag, a FLAG tag, a V5 tag and a HIS tag.

49. The fusion protein of claim 48, wherein the label is a HIS tag comprising 6, 7, 8, 9 or 10 histidine.

50. The fusion protein of any one of claims 46 to 49, wherein the second polypeptide is located at C-terminus of the first polypeptide and the label is located at C-terminus of the second polypeptide.

51. A fusion protein of any one of claims 46 to 50, wherein the fusion protein has a half-life of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days in blood circulation of a subject.

52. An isolated nucleic acid comprising a nucleotide sequence encoding the truncated form of IgA protease of any one of claims 1 to 23 or comprising a nucleotide sequence encoding the fusion protein of any one of claims 24 to 51.

53. The nucleic acid of claim 52, comprising a nucleotide sequence selected from the group consisting of SEQ ID NO: 3, SEQ ID NO: 5, SEQ ID NO: 7, SEQ ID NO: 9, SEQ ID NO: 11, SEQ ID NO: 13, SEQ ID NO: 33, SEQ ID NO: 34, SEQ ID NO: 35, SEQ ID NO: 36, SEQ ID NO: 37, SEQ ID NO: 38 and a nucleotide sequence having at least 70% sequence identity thereto.

54. A vector comprising the nucleic acid of claim 52 or 53.

55. A cell comprising the nucleic acid of claim 52 or 53 or the vector of claim 54.

56. The cell of claim 55, wherein the cell is a prokaryotic cell or a eukaryotic cell.

57. The cell of claim 56, wherein the prokaryotic cell is an *E. coli* cell.

58. The cell of claim 56, wherein the eukaryotic cell is a mammalian cell.

59. The cell of claim 58, wherein the mammalian cell is a human cell or a Chinese hamster ovary (CHO) cell.

60. The cell of claim 58, wherein the mammalian cell is a human embryonic kidney cell 293 (HEK293 cell).

61. A pharmaceutical composition comprising the truncated form of IgA protease of any one of claims 1 to 23, comprising the fusion protein of any one of claims 24 to 51, comprising the nucleic acid of claim 52 or 53, comprising the vector of claim 54 or comprising the cell of any one of claims 55 to 60, and a pharmaceutically acceptable carrier.

62. A method of producing a fusion protein comprising a step of culturing the cell of any one of claims 55 to 60.

63. A method of treating or preventing a disease associated with IgA deposition, comprising administering to a subject in need thereof the truncated form of IgA protease of any one of claims 1 to 23, the fusion protein of any one of claims 24 to 51 or the pharmaceutical composition of claim 61.

64. A method of treating or preventing a disease associated with IgA deposition, comprising administering to a subject in need thereof an IgA protease or a truncated form thereof, a fusion protein comprising the IgA protease or a truncated form thereof, or a pharmaceutical composition comprising the IgA protease or a truncated form thereof, wherein an amino acid sequence of the IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof.

65. Use of the truncated form of IgA protease of any one of claims 1 to 23, the fusion protein of any one of claims 24 to 51 or the pharmaceutical composition of claim 61 in the manufacture of a medicament for treating or preventing a disease associated with IgA deposition.

66. Use of an IgA protease or a truncated form thereof, a fusion protein comprising the IgA protease or a truncated form thereof, or a pharmaceutical composition comprising the IgA protease or a truncated form thereof in the manufacture of a medicament for treating or preventing a disease associated with IgA deposition, wherein an amino acid sequence of the IgA protease is selected from the group consisting of SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76 or a combination thereof.

67. The method of claim 63 or 64 or the use of claim 65 or 66, wherein the disease associated with IgA deposition is selected from the group consisting of IgA nephropathy, dermatitis herpetiformis, Henoch-Schönlein purpura (also known as IgA vasculitis), Kawasaki disease, purpura nephritis, IgA vasculitis renal impairment, IgA rheumatoid factor-positive rheumatoid arthritis, IgA-mediated anti-GBM disease or IgA-mediated ANCA-associated vasculitis.

68. The method of claim 63 or 64 or the use of claim 65 or 66, wherein the disease associated with IgA deposition is IgA nephropathy, IgA vasculitis or Kawasaki disease.
